(19) Europäisches Patentamt
European Patent Office
Office européen des brevets

(11) **EP 3 382 007 A1**

(12) **EUROPEAN PATENT APPLICATION**

(43) Date of publication:
03.10.2018 Bulletin 2018/40

(51) Int Cl.:
*C12N 5/00* *(2006.01)*  *C12N 5/073* *(2010.01)*

(21) Application number: 18159408.6

(22) Date of filing: 12.12.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 14.12.2012 US 201213715532

(62) Document number(s) of the earlier application(s) in
accordance with Art. 76 EPC:
13812432.6 / 2 931 875

(71) Applicant: **DePuy Synthes Products, Inc.**
**Raynham, MA 02767 (US)**

(72) Inventors:
• **BHATIA, Ravinder**
**Blue Bell, PA 19422 (US)**

• **HONG, L.S. Klaudyne**
**New York, NY 10023 (US)**
• **OZTURK, Sadettin S.**
**Paoli, PA 19301 (US)**
• **KAMARAJU, Venkat H.**
**North Wales, PA 19454 (US)**

(74) Representative: **Carpmaels & Ransford LLP**
**One Southampton Row**
**London WC1B 5HA (GB)**

Remarks:
•This application was filed on 01-03-2018 as a
divisional application to the application mentioned
under INID code 62.
•Claims filed after the date of filing of the application
/ after the date of receipt of the divisional application
(Rule 68(4) EPC).

(54) **NUTRIENT ENRICHED MEDIA FOR HUTC GROWTH**

(57) This invention provides for methods of growing anchorage-dependent cells (*e.g.* hUTC) in culture medium comprising amino acids, vitamins, salts nucleosides, insulin, transferrin, ethanolamine and sodium selenium, wherein the culture medium is supplemented with serum. The method further comprises addition of a serum-free nutrient solution comprising amino acids, vitamins, salts nucleosides, insulin, transferrin, ethanolamine and sodium selenium. The invention also provides for culture media and serum-free nutrient solutions for growing anchorage-dependent cells.

EP 3 382 007 A1

**Description**

**FIELD OF THE INVENTION**

This application relates to nutrient enriched culture media for the growth of anchorage-dependent cells, such as *e.g.* umbilical cord tissue-derived cells.

**BACKGROUND OF THE INVENTION**

For an allogeneic cell therapy product, cells or tissues are obtained from a donor, which are further manipulated before administering to patients. Generally, a manufacturing process for non-homologous cell therapy product includes the following steps: thawing a cell bank vial and expanding cells to inoculate a production vessel; producing cells in a production vessel; removing undesirable impurities used during production of cells such as serum and trypsin; concentrating cells; formulating cells into the final formulation buffer; and freezing cells. Such manufacturing processes can be quite complex and expensive. For example, the cells for cell therapy applications are generally cultivated and expanded in growth medium supplemented with serum. The cost of production of cell therapy can be very high due to the high cost of serum, medium, and other consumables used in the process. Multiple factors can limit cells from growing to high cell density, including nutrient limitation, cell byproduct accumulation in culture, physical environment, etc. Accordingly, it is desirable to increase volumetric cells produced from a production vessel by growing cells to high cell density.

Previously, it has been shown that anchorage-dependent cells, such as *e.g.* human umbilical cord tissue derived-cells (hUTCs), can be grown to high cell density by exchanging cell growth medium containing 15% Fetal Bovine Serum (FBS) on day 3 of the run. However, such a medium exchange is less desirable due to high serum cost, high serum usage for production, and additional operational manipulations. Therefore, a process with medium exchange is not commercially feasible.

What is needed is a new method for growing cells without the need of exchanging cell growth medium that is commercially feasible.

**SUMMARY OF THE INVENTION**

One embodiment of the invention is a culture medium for growing anchorage-dependent cells comprising: (1) the amino acids L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Aspargine; L-Aspartic Acid; L-Glutamic Acid; L-Proline; and L-Taurine; (2) the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin); (3) the salts calcium chloride, potassium chloride, magnesium sulfate, sodium chloride, and one or more sodium phosphate salts; (4) the nucleosides thymidine, adenosine, cytidine, uridine and guanosine; (5) insulin; (6) transferrin; (7) lipoic acid/thioctic acid; (8) ethanolamine; (9) sodium selenite; and (10) one or more energy sources.

In one embodiment, the culture medium comprises:

at least about 0.05 g/L of L-Arginine; at least about 0.02 g/L of L-Cystine; at least about 0.2 g/L of L-Glutamine; at least about 0.01 g/L Glycine; at least about 0.02 g/L of L-Histidine; at least about 0.09 g/L of L-Isoleucine; at least about 0.09 g/L of L-Leucine; at least about 0.09 g/L of L-Lysine; at least about 0.02 g/L of L-Methionine; at least about 0.05 g/L of L-Phenylalanine; at least about 0.03 g/L of L-serine; at least about 0.08 g/L of L-Threonine; at least about 0.009 g/L of L-tryptophan; at least about 0.08 g/L of L-tyrosine; at least about 0.08 g/L of L-Valine; at least about 0.005 g/L L-Cysteine; at least about 0.0004 g/L of L-Alanine; at least about 0.01 g/L of L-Aspargine; at least about 0.006 g/L of L-Aspartic Acid; at least 0.03 g/L of L-Glutamic Acid; at least about 0.005 g/L L-Proline; and at least about 0.0003 g/L of L-Taurine;
from about 5 x 10$^{-6}$ g/L to about 0.015 g/L of each of the vitamins;
at least about 0.05 g/L of Calcium Chloride, Anhydrous, at least about 0.1 g/L of potassium chloride; at least about 0.2 g/L of magnesium sulfate, at least about 0.08 g/L of sodium phosphate, monobasic, $H_2O$ and at least about 0.0005 g/L of sodium phosphate, dibasic heptahydrate ($Na_2HPO_4.7H_2O$);
at least about 0.0001 g/L of thymidine and least about 0.005 g/L of each of adenosine, cytidine, uridine, and guanosine; and
at least 0.003 g/L of insulin, at least 0.05 g/L of transferrin, at least about 5 x 10$^{-6}$ g/L of lipoic acid/thioctic acid, at least 0.05 g/L of ethanolamine and at least about 0.00004 g/L of sodium selenite.

Another embodiment of the invention is a serum-free nutrient solution for growing anchorage dependent cells, which comprises:

the amino acids L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Aspargine, L-Aspartic Acid, L-Glutamic Acid, L-Proline and L-Taurine;
the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);
the salts sodium phosphate, monobasic and sodium phosphate, dibasic heptahydrate;
the nucleosides adenosine, cytidine, uridine and guanosine;
insulin; transferrin; lipoic acid/thioctic acid; ethanolamine; and sodium selenite.

The invention also provides kits for growing anchorage-dependent cells. The kits comprise the culture medium and/or serum-free nutrient solution. The culture medium, serum-free solution, and kits may be used in a method of growing anchorage-dependent cells (such as *e.g.* umbilical cord tissue-derived cells).

Accordingly, another embodiment of the invention is a method of culturing isolated umbilical cord tissue-derived cells comprising:

growing umbilical cord tissue-derived cells seeded on microcarriers in a culture medium comprising amino acids, vitamins, salts, nucleosides, lipoic/thioctic acid, ethanolamine, insulin, transferrin, sodium selenium, which is supplemented with serum, for a sufficient period of time to allow for the cells to achieve a desired initial population density;
adding a serum-free nutrient solution, which comprises amino acids, vitamins, salts, insulin, transferrin, ethanolamine, lipoic acid/thioctic acid, sodium selenium, after the cells have achieved the desired initial population density; and
growing the cells for a sufficient period of time to allow for the cells to achieve a desired final population density.

The method may further comprise additional steps. In one embodiment, the method further comprises seeding the cells on the microcarriers. In another embodiment, the method further comprises isolating the cells after culturing. In one embodiment, the desired initial population density is achieved after 3 to 4 days. The method does not require medium exchange. The method may be carried out in a roller bottle culture system and the microcarriers may have an amine treated surface.

The umbilical cord tissue-derived cells used in the method are isolated from human umbilical cord tissue substantially free of blood, are capable of self-renewal and expansion in culture, have the potential to differentiate, express CD13, CD90, HLA-ABC, and do not express CD34, CD117, and HLA-DR. In one embodiment, the cells do not express hTERT or telomerase. The characteristics of the cells before and after culture are substantially the same. In one embodiment, the characteristics of the cells before and after culturing are the same.

In one embodiment, the culture medium used in the method comprises:

the amino acids L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Aspargine; L-Aspartic Acid; L-Glutamic Acid; L-Proline; and L-Taurine;
the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);
the salts calcium chloride, potassium chloride, magnesium sulfate, sodium chloride, and one or more sodium phosphate salts;
the nucleosides thymidine, adenosine, cytidine, uridine and guanosine;
insulin; transferrin; lipoic acid/thioctic acid; ethanolamine; sodium selenite; and one or more energy sources (such as *e.g.* D-glucose and sodium pyruvate).

In another embodiment, the culture medium used in the method comprises:

at least about 0.05 g/L of L-Arginine; at least about 0.02 g/L of L-Cystine; at least about 0.2 g/L of L-Glutamine; at least about 0.01 g/L Glycine; at least about 0.02 g/L of L-Histidine; at least about 0.09 g/L of L-Isoleucine; at least about 0.09 g/L of L-Leucine; at least about 0.09 g/L of L-Lysine; at least about 0.02 g/L of L-Methionine; at least about 0.05 g/L of L-Phenylalanine; at least about 0.03 g/L of L-serine; at least about 0.08 g/L of L-Threonine; at least about 0.009 g/L of L-tryptophan; at least about 0.08 g/L of L-tyrosine; at least about 0.08 g/L of L-Valine; at least about 0.005 g/L L-Cysteine; at least about 0.0004 g/L of L-Alanine; at least about 0.01 g/L of L-Aspargine; at least about 0.006 g/L of L-Aspartic Acid; at least 0.03 g/L of L-Glutamic Acid; at least about 0.005 g/L L-Proline; and at least about 0.0003 g/L of L-Taurine;
from about 5 x $10^{-6}$ g/L to about 0.015 g/L of each of the vitamins;
at least about 0.05 g/L of Calcium Chloride, Anhydrous, at least about 0.1 g/L of potassium chloride; at least about 0.2 g/L of magnesium sulfate, at least about 0.08 g/L of sodium phosphate, monobasic, $H_2O$ and at least about 0.0005 g/L of sodium phosphate, dibasic heptahydrate ($Na_2HPO_4.7H_2O$);

at least about 0.0001 g/L of thymidine and least about 0.005 g/L of each of adenosine, cytidine, uridine, and guanosine; and

at least 0.003 g/L of insulin, at least 0.05 g/L of transferrin, at least about $5 \times 10^{-6}$ g/L of lipoic acid/thioctic acid, at least 0.05 g/L of ethanolamine and at least about 0.00004 g/L of sodium selenite.

The culture medium used in the method is supplemented with 2 to 20% of FBS. In one embodiment, the culture medium is supplemented with about 7.5%, about 10%, or about 15% of FBS.

In one embodiment, the serum-free nutrient solution used in the method comprises:

the amino acids L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Aspargine, L-Aspartic Acid, L-Glutamic Acid, L-Proline and L-Taurine;

the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);

the salts sodium phosphate, monobasic and sodium phosphate, dibasic heptahydrate;

the trace minerals Copper(II)sulfate pentahydrate ($CuSO_4.5H_2O$), Zinc sulfate, heptahydrate, ($ZnSO_4.7H_2O$);

the nucleosides adenosine, cytidine, uridine and guanosine;

insulin; transferrin; ethanolamine; lipoic acid/thioctic acid; and sodium selenite.

The culture medium and serum-free nutrient solution may also contain further components. For example, the culture medium and/or serum-free nutrient solution may further comprise putrescine, a stabilizer, and/or a foaming agent.

Other features and advantages of the invention will be apparent from the detailed description that follows.

## DETAILED DESCRIPTION

In the following detailed description of the illustrative embodiments, reference is made to the accompanying drawings that form a part hereof. These embodiments are described in sufficient detail to enable those skilled in the art to practice the invention, and it is understood that other embodiments may be utilized and that logical structural, mechanical, electrical, and chemical changes may be made without departing from the spirit or scope of the invention. To avoid detail not necessary to enable those skilled in the art to practice the embodiments described herein, the description may omit certain information known to those skilled in the art. The following detailed description is, therefore, not to be taken in a limiting sense.

In one aspect, the invention provides for a process for growing anchorage-dependent cells, such as *e.g.* human umbilical cord tissue-derived cells ("hUTC"), attached to microcarriers in a serum-containing medium to high cell density without medium exchange by feeding culture with serum-free nutrient solution in the middle of the run. The process allows for growth of cells to high cell density without affecting the biological function of the cells. Since the invention increases the yield from the production bioreactor, the invention provides economic and commercial benefits.

In another aspect, the invention provides for culture media and nutrient solutions that are suitable for growing anchorage-dependent cells in spinner flasks, preferably without the need for medium exchange.

In one embodiment, the invention discloses the composition of an enriched medium and a concentrated serum-free nutrient solution to grow hUTC to high density in spinner flasks without medium exchange. This method reduces serum usage and increases volumetric productivity to reduce cost of manufacturing. In particular, the method allows for enhanced doubling with media exchange.

In another embodiment, the invention provides a serum-free nutrient solution comprising: amino acids (to replenish consumed amino acids in culture); vitamins; salts (to maintain osmotic balance in culture); nucleosides; insulin; transferrin; and optionally but preferably ethanolamine; and sodium selenium. This invention allows scalability of the process to large-scale bioreactors. In yet another embodiment, the invention provides a culture medium comprising amino acids, vitamins, salts nucleosides, insulin, transferrin, and optionally but preferably ethanolamine and sodium selenium. Prior to use, this culture medium may be supplemented with serum.

Another aspect of the invention is a method of growing hUTCs attached to microcarriers to high cell density in suspension culture in spinner flasks by enriching the serum containing growth medium with nutrients and feeding the culture with serum-free nutrients is also disclosed. This method eliminates medium exchange to grow hUTCs to high density.

Various terms are used throughout the specification and claims. Such terms are to be given their ordinary meaning in the art unless otherwise indicated. Other specifically defined terms are to be construed in a manner consistent with the definition provided herein.

In one embodiment, the cells used in the present invention are generally referred to as postpartum cells or postpartum-derived cells (PPDCs). These cells are more specifically "umbilicus-derived cells" or "umbilical cord-derived cells" (UDC), or "umbilical cord tissue-derived cells" (UTC). In addition, the cells may be described as being stem or progenitor cells, the latter term being used in the broad sense. The term "derived" is used to indicate that the cells have been obtained from their biological source and grown or otherwise manipulated *in vitro* (e.g., cultured in a growth medium to expand the population and/or to produce a cell line). The *in vitro* manipulations of umbilical stem cells and the unique features of the umbilicus-derived cells of the present invention are described in detail below.

Stem cells are undifferentiated cells defined by the ability of a single cell both to self-renew and to differentiate to produce progeny cells, including self-renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation, and to contribute substantially to most, if not all, tissues following injection into blastocysts.

Stem cells are classified according to their developmental potential as: (1) totipotent; (2) pluripotent; (3) multipotent; (4) oligopotent; and (5) unipotent. Totipotent cells are able to give rise to all embryonic and extraembryonic cell types. Pluripotent cells are able to give rise to all embryonic cell types. Multipotent cells include those able to give rise to a subset of cell lineages, but all within a particular tissue, organ, or physiological system. For example, hematopoietic stem cells (HSC) can produce progeny that include HSC (self-renewal), blood cell-restricted oligopotent progenitors, and all cell types and elements (*e.g.*, platelets) that are normal components of the blood. Cells that are oligopotent can give rise to a more restricted subset of cell lineages than multipotent stem cells. Cells, which are unipotent, are able to give rise to a single cell lineage (*e.g.*, spermatogenic stem cells).

Stem cells are also categorized based on the source from which they are obtained. An adult stem cell is generally a multipotent undifferentiated cell found in tissue comprising multiple differentiated cell types. The adult stem cell can renew itself. Under normal circumstances, it can also differentiate to yield the specialized cell types of the tissue from which it originated, and possibly other tissue types. An embryonic stem cell is a pluripotent cell from the inner cell mass of a blastocyst-stage embryo. A fetal stem cell is one that originates from fetal tissues or membranes. A postpartum stem cell is a multipotent or pluripotent cell that originates substantially from extraembryonic tissue available after birth, namely, the umbilical cord. These cells have been found to possess features characteristic of pluripotent stem cells, including rapid proliferation and the potential for differentiation into many cell lineages. Postpartum stem cells may be blood-derived (*e.g.*, as are those obtained from umbilical cord blood) or non-blood-derived (*e.g.*, as obtained from the non-blood tissues of the umbilical cord and placenta).

Various terms are used to describe cells in culture. "Cell culture" refers generally to cells taken from a living organism and grown under controlled conditions ("in culture" or "cultured"). A "primary cell culture" is a culture of cells, tissues, or organs taken directly from an organism(s) before the first subculture. Cells are "expanded" in culture when they are placed in a growth medium under conditions that facilitate cell growth and/or division, resulting in a larger population of the cells. When cells are expanded in culture, the rate of cell proliferation is sometimes measured by the amount of time needed for the cells to double in number. This is referred to as "doubling time."

The term "cell line" generally refers to a population of cells formed by one or more subcultivations of a primary cell culture. Each round of subculturing is referred to as a passage. When cells are subcultured, they are referred to as having been "passaged." A specific population of cells, or a cell line, is sometimes referred to or characterized by the number of times it has been passaged. For example, a cultured cell population that has been passaged ten times may be referred to as a P10 culture. The primary culture, *i.e.,* the first culture following the isolation of cells from tissue, is designated P0. Following the first subculture, the cells are described as a secondary culture (P1 or passage 1). After the second subculture, the cells become a tertiary culture (P2 or passage 2), and so on. It will be understood by those of skill in the art that there may be many population doublings during the period of passaging; therefore, the number of population doublings of a culture is greater than the passage number. The expansion of cells (*i.e.,* the number of population doublings) during the period between passaging depends on many factors, including, but not limited to, the seeding density, substrate, medium, growth conditions, and time between passaging.

"Differentiation" is the process by which an unspecialized ("uncommitted") or less specialized cell acquires the features of a specialized cell, such as *e.g.* a nerve cell or a muscle cell. A "differentiated" cell is one that has taken on a more specialized ("committed") position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. "De-differentiation" refers to the process by which a cell reverts to a less specialized (or committed) position within the lineage of a cell. As used herein, the "lineage" of a cell defines the heredity of the cell, *i.e.,* which cells it came from and what cells it can give rise to. The lineage of a cell places the cell within a hereditary scheme of development and differentiation.

In a broad sense, a "progenitor cell" is a cell that has the capacity to create progeny that are more differentiated than itself, and yet retains the capacity to replenish the pool of progenitors. By that definition, stem cells themselves are also progenitor cells, as are the more immediate precursors to terminally differentiated cells. When referring to the cells of the present invention, as described in more detail below, this broad definition of progenitor cell may be used. In a narrower sense, a progenitor cell is often defined as a cell that is intermediate in the differentiation pathway, *i.e.*, it arises from a stem cell and is intermediate in the production of a mature cell type or subset of cell types. This type of progenitor cell is generally not able to self-renew. Accordingly, if this type of cell is referred to herein, it will be referred to as a "non-renewing progenitor cell" or as an "intermediate progenitor or precursor cell."

Generally, a "trophic factor" is defined as a substance that promotes survival, growth, proliferation, and/or maturation of a cell, or stimulates increased activity of a cell.

The term "standard growth conditions," as used herein refers to culturing of cells at 37°C, in a standard atmosphere comprising 5% $CO_2$ and relative humidity maintained at about 100%. While the foregoing conditions are useful for culturing, it is to be understood that such conditions are capable of being varied by the skilled artisan who will appreciate the options available in the art for culturing cells.

The term "isolate" as used herein generally refers to a cell, which has been separated from its natural environment. This term includes gross physical separation from its natural environment, *e.g.*, removal from the donor animal. In preferred embodiments, an isolated cell is not present in a tissue, *i.e.*, the cell is separated or dissociated from the neighboring cells with which it is normally in contact. Preferably, cells are administered as a cell suspension. As used herein, the phrase "cell suspension" includes cells which are in contact with a medium and which have been dissociated, *e.g.*, by subjecting a piece of tissue to gentle trituration.

One embodiment of the invention is a method of culturing isolated anchorage-dependent cells utilizing the culture medium and serum-free nutrient solution of the invention. The method for culturing isolated anchorage-dependent cells (such as *e.g.* umbilical cord tissue-derived cells) provides culturing the cells on at least one carrier particle, *e.g.,* a microcarrier. The microcarrier can be comprised of natural or synthetically-derived materials. Examples include collagen-based microcarriers, dextran-based microcarriers, or cellulose-based microcarriers, as well as glass, ceramics, polymers (such as polystyrene), or metals. The microcarrier can be protein-free or protein-coated, *e.g.* with collagen. In a further aspect the microcarrier can be comprised of, or coated with, compounds that enhance binding of the cell to the microcarrier and enhance release of the cell from the microcarrier including, but not limited to, sodium hyaluronate, poly(monostearoylglyceride co-succinic acid), poly-D,L-lactide-co-glycolide, fibronectin, laminin, elastin, lysine, n-isopropyl acrylamide, vitronectin, and collagen. Examples further include microcarriers that possess a microcurrent, such as microcarriers with a particulate galvanic couple of zinc and copper that produces low levels of biologically relevant electricity; or microcarriers that are paramagnetic, such as paramagnetic calcium-alginate microcarriers. The methods may be carried out in a roller bottle system.

It is to be understood that this invention is not limited to particular methods, reagents, compounds, compositions or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a cell" includes a combination of two or more cells, and the like.

"Microcarriers" refers to particles, beads, or pellets useful for attachment and growth of anchorage-dependent cells in culture. The microcarriers have the following properties: (a) they are small enough to allow them to be used in suspension cultures (with a stirring rate that does not cause significant shear damage to the microcarriers or the cells); (b) they are solid, or have a solid core with a porous coating on the surface; and (c) their surfaces (exterior and interior surface in case of porous carriers) may be positively or negatively charged. In one aspect, the microcarriers have an overall particle diameter between about 150 and 350 $\mu$m, and have a positive charge density of between about 0.8 and 2.0 meq/g. Exemplary useful microcarriers include Cytodex® 1, Cytodex® 2, or Cytodex® 3 (GE Healthcare Life Sciences).

In another aspect, the microcarrier is a solid carrier. Solid carriers are particularly suitable for adhesion cells, *e.g.*, anchorage-dependent cells. The carrier particle can also be a porous microcarrier. Examples further include microcarriers that possess a microcurrent, such as microcarriers with a particulate galvanic couple of zinc and copper that produces low levels of biologically relevant electricity; or microcarriers that are paramagnetic, such as paramagnetic calcium-alginate microcarriers.

"Porous microcarriers" refers to particles useful for attachment and growth of anchorage- dependent cells in culture. The porous microcarriers have the following properties: (a) they are small enough to allow them to be used in suspension cultures (with a stirring rate that does not cause significant shear damage to the microcarriers or the cells); (b) they have pores and interior spaces of sufficient size to allow cells to migrate into the interior spaces of the particle; and (c) their surfaces (exterior and interior) may be positively or negatively charged. In one series of embodiments, the carriers (a) have an overall particle diameter between about 150 and 350 $\mu$m; (b) have pores having an average pore opening diameter of between about 15 and about 40 $\mu$m; and (c) have a positive charge density of between about 0.8 and 2.0 meq/g. In some embodiments, DEAE (N, N,-diethylaminoethyl) groups provide the positive charge. Useful porous microcarriers include, without limitation, Cytopore 1® and Cytopore 2® (GE Healthcare Life Sciences, Piscataway N.J.).

"Anchorage-dependent cells" are cells, including mammalian cells, which need to attach to a surface, *e.g.,* a tissue culture flask surface or a microcarrier particle surface, to replicate in tissue culture.

As used herein, the term "about" when referring to a measurable value such as an amount, a temporal duration, and the like, is meant to encompass variations of ± 20% or ± 10%, more preferably ± 5%, even more preferably ± 1%, and still more preferably ± 0.1% from the specified value, as such variations are appropriate to perform the disclosed methods.

## I. Culture Media and Serum-Free Nutrient Feed Solution

This invention provides for enriched media and concentrated nutrient feed solutions, which are serum-free. The media and the serum-free nutrient solution can be used to grow cells to high density. In a preferred embodiment, the media and the serum-free nutrient solution can be used to grow anchorage-dependent cells, such as *e.g.* hUTC, to high density in spinner flasks at lower serum consumption.

In one embodiment, the culture medium and serum-free nutrient solution are used for the growth of umbilical cord tissue-derived cells. In another embodiment, culture medium and serum-free nutrient solution are suitable for the growth of progenitor cells including but not limited to mesenchymal stem cells, bone marrow derived stem cells, cells derived from placental tissue, adherent cells derived from non-marrow tissues, such as *e.g.* adipose tissue, muscle tissue, blood vessel including internal mammary artery derived cells, cells derived from the dental pulp of teeth, cells derived from amniotic fluid and fibroblasts including neonatal foreskin fibroblasts.

## A. Culture Media

One aspect of the invention is a culture medium comprising amino acids; vitamins; salts; nucleosides; insulin; transferrin; ethanolamine; sodium selenite; D-glucose and sodium pyruvate.

In one embodiment, the culture medium comprises the common L-amino acids. In another embodiment, the culture medium comprises the following amino acids: L-Arginine; L-Cystine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; and optionally L-Cysteine; L-Alanine; L-Aspargine; L-Aspartic Acid; L-Glutamic Acid; L-Proline; and L-Taurine. In an alternate embodiment, the culture medium comprises from about 0.0006 g/L to about 0.1 g/L of each of the amino acid.

In yet another embodiment, the culture medium comprises at least about 0.1 g/L of L-Arginine; at least about 0.05 g/L of L-Cystine; at least about 0.3 g/L of L-Glutamine; at least about 0.02 g/L Glycine; at least about 0.03 g/L of L-Histidine; at least about 0.08 g/L of L-Isoleucine; at least about 0.08 g/L of L-Leucine; at least about 0.1 g/L of L-Lysine; at least about 0.1 g/L of L-Methionine; at least about 0.05 g/L of L-Phenylalanine; at least about 0.03 g/L of L-serine; at least about 0.08 g/L of L-Threonine; at least about 0.01 g/L of L-tryptophan; at least about 0.09 g/L of L-tyrosine; at least about 0.07 g/L of L-Valine; and optionally at least about 0.007 g/L L-Cysteine; at least about 0.0005 g/L of L-Alanine; at least about 0.02 g/L of L-Aspargine; at least about 0.006 g/L of L-Aspartic Acid; at least 0.03 g/L of L-Glutamic Acid; at least about 0.01 g/L L-Proline; and at least about 0.0006 g/L of L-Taurine.

In an alternate embodiment, the culture medium comprises about 0.09705 g/L of L-Arginine; about 0.06779 g/L of L-Cystine; about 0.009224 g/L of L-Cysteine; about 0.584 g/L of L-Glutamine; about 0.031125 g/L of Glycine; about 0.048288 g/L of L-Histidine; about 0.163713 g/L of L-Isoleucine; about 0.163713 g/L of L-Leucine; about 0.16807 g/L of L-Lysine; about 0.036748 g/L of L-Methionine; about 0.073695 g/L of L-Phenylalanine; about 0.05145 g/L of L-serine; about 0.108609 g/L of L-Threonine; about 0.018457 g/L of L-tryptophan; about 0.121813 g/L of L-tyrosine; about 0.111105 g/L of L-Valine; about 0.000668 g/L of L-Alanine; about 0.031978 g/L of L-Aspargine; about 0.0080 g/L of L-Aspartic Acid about 0.054728 g/L of L-Glutamic Acid; about 0.02403 g/L of L-Proline; and about 0.000844 g/L of L-Taurine.

The culture medium further comprises one or more vitamins. In one embodiment, culture medium comprises at least the following vitamins: D-calcium pantothenate; choline chloride; folic acid; I-inositol; niacinamide; pyridoxal; riboflavin; thiamine; and optionally d-Biotin; pyridoxine; and Vitamin $B_{12}$ (cyanocobalamin). The media may further comprise Vitamin C and Vitamin A. In another embodiment, the culture medium comprises from about $5 \times 10^{-6}$ g/L to about 0.015 g/L of each of the vitamins. In one embodiment, the culture medium comprises about 0.004338 g/L of D-Calcium Pantothenate; about 0.006094 g/L of Choline Chloride; about 0.004302 g/L of Folic Acid; about 0.009568 g/L of I-Inositol; about 0.004302 g/L of Niacinamide; about 0.004153 g/L of Pyridoxal; about 0.000431 g/L of Riboflavin; about 0.004304 g/L of thiamine; about 3.75E-05 g/L of d-Biotin; about 1.85E-05 g/L of Pyridoxin and about 0.000102 g/L of Vitamin $B_{12}$ (cyanocobalamin).

The culture medium further comprises one or more inorganic salts. In one embodiment, the culture medium comprises calcium chloride, potassium chloride, magnesium sulfate, sodium chloride, and one or more sodium phosphate salt. In another embodiment, the culture medium comprises Calcium Chloride, Anhydrous, potassium chloride, magnesium sulfate, anhydrous, sodium phosphate, monobasic, $H_2O$, and optionally sodium phosphate, dibasic heptahydrate ($Na_2HPO_4 \cdot 7H_2O$). Depending on the salt used, the culture medium may comprise from at least about 0.005 g/L to about least about 7 g/L of the salts. In one embodiment, the culture medium comprises at least about 0.01 g/L of Calcium Chloride, Anhydrous, at least about 0.1 g/L of potassium chloride; at least about 0.2 g/L of magnesium sulfate, at least about 0.08 g/L of sodium phosphate, monobasic, $H_2O$ and optionally at least about 0.0005 g/L of sodium phosphate, dibasic heptahydrate ($Na_2HPO_4.7H_2O$). In another embodiment, the culture medium comprises about 0.2 g/L of Calcium Chloride, Anhydrous, about 0.4 g/L of potassium chloride; about 0.9767 g/L of magnesium sulfate, at least about 0.13 g/L of sodium phosphate, monobasic, $H_2O$, about 6.4 g/L of sodium chloride and optionally at least about 0.002 g/L of sodium phosphate, dibasic heptahydrate ($Na_2HPO_4.7H_2O$).

The culture medium further comprises nucleosides. In one embodiment, the culture medium comprises the nucleic acid derivatives (nucleosides) thymidine, adenosine, cytidine, uridine, and guanosine. In an alternate embodiment, the medium comprises at least about 0.0001 g/L to at least about 0.02 g/L of the each of the nucleosides. In one embodiment, the medium comprises at least about 0.0001 g/L of thymidine and least about 0.005 g/L of each of adenosine, cytidine, uridine, and guanosine. In another embodiment, the medium comprises about 0.00045 g/L of thymidine and about 0.015 g/L of each of adenosine, cytidine, uridine, and guanosine.

In one embodiment, the culture medium further comprises insulin, transferrin, ethanolamine, and sodium selenate. In another embodiment, the culture medium comprises at least about 0.003 g/L of insulin. In another embodiment, the culture medium comprises about 0.01 g/L of insulin. In an alternate embodiment, the culture medium comprises at least about 0.05 g/L of transferrin. In another embodiment, the culture medium comprises about 0.055 g/L of transferrin. In yet another embodiment, the culture medium comprises at least about 0.01 g/L of ethanolamine. In an alternate embodiment, the culture medium comprises about 0.02 g/L of ethanolamine. In one embodiment, the culture medium comprises at least about 0.00004 g/L of sodium selenite. In another embodiment, the culture medium comprises about 0.000067g/L of sodium selenite. In yet another embodiment, the culture medium does not further comprise insulin, transferrin, and ethanolamine.

One embodiment of the invention is a culture medium comprising:
*the amino acids:* L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Aspargine; L-Aspartic Acid; L-Glutamic Acid; L-Proline; and L-Taurine; *the vitamins:* D-calcium pantothenate; choline chloride; folic acid; I-inositol; niacinamide; pyridoxal; riboflavin; thiamine; d-Biotin; pyridoxine; and Vitamin $B_{12}$ (cyanocobalamin); *the salts:* calcium chloride, potassium chloride, magnesium sulfate, sodium chloride, and one or more sodium phosphate salts; *the nucleosides (nucleic acid derivatives):* thymidine, adenosine, cytidine, uridine and guanosine; insulin; transferrin; ethanolamine; sodium selenite; and one or more energy sources. In one embodiment, this culture medium comprises from about 0.0006 g/L to about 0.1 g/L of each of the amino acids. In another embodiment, the culture medium comprises at least about 0.05 g/L of L-Arginine; at least about 0.02 g/L of L-Cystine; at least about 0.2 g/L of L-Glutamine; at least about 0.01 g/L Glycine; at least about 0.02 g/L of L-Histidine; at least about 0.09 g/L of L-Isoleucine; at least about 0.09 g/L of L-Leucine; at least about 0.09 g/L of L-Lysine; at least about 0.02 g/L of L-Methionine; at least about 0.05 g/L of L-Phenylalanine; at least about 0.03 g/L of L-serine; at least about 0.08 g/L of L-Threonine; at least about 0.009 g/L of L-tryptophan; at least about 0.08 g/L of L-tyrosine; at least about 0.08 g/L of L-Valine; at least about 0.005 g/L L-Cysteine; at least about 0.0004 g/L of L-Alanine; at least about 0.01 g/L of L-Aspargine; at least about 0.006 g/L of L-Aspartic Acid; at least 0.03 g/L of L-Glutamic Acid; at least about 0.005 g/L L-Proline; and at least about 0.0003 g/L of L-Taurine. In an alternate embodiment, the culture medium comprises from about 5 x10$^{-6}$ g/L to about 0.015 g/L of each of the vitamins. In one embodiment, the culture medium comprises at least about 0.005 g/L of Calcium Chloride, Anhydrous, at least about 0.1 g/L of potassium chloride; at least about 02 g/L of magnesium sulfate, at least about 0.1 g/L of sodium phosphate, monobasic, $H_2O$ and at least about 0.0005 g/L of sodium phosphate, dibasic heptahydrate ($Na_2HPO_4.7H_2O$). In another embodiment, the medium comprises at least about 0.0001 g/L to at least about 0.02 g/L of the each of the nucleosides. In one embodiment, the medium comprises at least about 0.0001 g/L of thymidine and least about 0.005 g/L of each of adenosine, cytidine, uridine, and guanosine. In one embodiment, the culture medium further comprises at least 0.005 g/L of insulin, and at least 0.03 g/L of transferrin, at least 0.01 g/L of ethanolamine and at least about 0.00004 g/L of sodium selenite. The one or more energy sources may be D-glucose and sodium pyruvate.

In another embodiment, the culture medium comprises: (1) the amino acids L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Aspargine; L-Aspartic Acid; L-Glutamic Acid; L-Proline; and L-Taurine; (2) the vitamins D-calcium pantothenate; choline chloride; folic acid; I-inositol; niacinamide; pyridoxal; riboflavin; thiamine; d-Biotin; pyridoxine; and Vitamin $B_{12}$(cyanocobalamin); (3) the salts calcium chloride, potassium chloride, magnesium sulfate, sodium chloride, and one or more sodium phosphate salts; (4) the nucleosides thymidine, adenosine, cytidine, uridine and guanosine; (5) insulin; (6) transferrin; (7) lipoic acid/thioctic acid; (8) ethanolamine; (9) sodium selenite; and (10) one or more energy sources.

One embodiment of the invention is a culture medium comprising: *the amino acids:* L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Aspargine; L-Aspartic Acid; L-Glutamic Acid; L-Proline;

and L-Taurine; *the vitamins:* D-calcium pantothenate; choline chloride; folic acid; I-inositol; niacinamide; pyridoxal; riboflavin; thiamine; d-Biotin; pyridoxine; and Vitamin $B_{12}$ (cyanocobalamin); *the salts:* calcium chloride, potassium chloride, magnesium sulfate, sodium chloride, and one or more sodium phosphate salts; *the nucleosides (nucleic acid derivatives):* thymidine, adenosine, cytidine, uridine and guanosine; lipoic/thoictic acid; the trace minerals ferric nitrate, copper sulfate, zinc sulfate; and one or more energy sources. In one embodiment, this culture medium comprises from about 0.0006 g/L to about 0.1 g/L of each of the amino acid. In another embodiment, the culture medium comprises at least about 0.05 g/L of L-Arginine; at least about 0.02 g/L of L-Cystine; at least about 0.2 g/L of L-Glutamine; at least about 0.01 g/L Glycine; at least about 0.02 g/L of L-Histidine; at least about 0.09 g/L of L-Isoleucine; at least about 0.09 g/L of L-Leucine; at least about 0.09 g/L of L-Lysine; at least about 0.02 g/L of L-Methionine; at least about 0.05 g/L of L-Phenylalanine; at least about 0.03 g/L of L-serine; at least about 0.08 g/L of L-Threonine; at least about 0.009 g/L of L-tryptophan; at least about 0.08 g/L of L-tyrosine; at least about 0.08 g/L of L-Valine; at least about 0.005 g/L L-Cysteine; at least about 0.0004 g/L of L-Alanine; at least about 0.01 g/L of L-Aspargine; at least about 0.006 g/L of L-Aspartic Acid; at least 0.03 g/L of L-Glutamic Acid; at least about 0.005 g/L L-Proline; and at least about 0.0003 g/L of L-Taurine. In an alternate embodiment, the culture medium comprises from about $5 \times 10^{-6}$ g/L to about 0.015 g/L of each of the vitamins. In one embodiment, the culture medium comprises at least about 0.01 g/L of Calcium Chloride, Anhydrous, at least about 0.1g/L of potassium chloride; at least about 0.2 g/L of magnesium sulfate (anhydrous), at least about 0.1 g/L of sodium phosphate, monobasic, $H_2O$ and at least about 0.005 g/L of sodium phosphate, dibasic heptahydrate ($Na_2HPO_4.7H_2O$). In another embodiment, the medium comprises at least about 0.0001 g/L to at least about 0.02 g/L of the each of the nucleosides. In one embodiment, the medium comprises at least about 0.0001 g/L of thymidine and least about 0.005 g/L of each of adenosine, cytidine, uridine, and guanosine. The one or more energy sources may be D-glucose and sodium pyruvate.

In addition to the trace mineral sodium selenite, in certain embodiments, the culture medium further comprises one or more additional trace minerals. In one embodiment, the medium further comprises ferric nitrate, copper sulfate, and zinc sulfate. In one embodiment, the medium further comprises ferric nitrate ($9H_2O$), copper(II)sulfate pentahydrate ($CuSO_4.5H_2O$) and Zinc sulfate, heptahydrate, ($ZnSO_4.7H_2O$). The trace minerals may be present in an amount ranging from about $5 \times 10^{-8}$ g/L to about $3 \times 10^{-4}$ g/L of each of the trace minerals. In an alternate embodiment, the medium further comprises about 0.001 g/L of ferric nitrate ($9H_2O$), about 9.33 E-08 g/L of copper(II)sulfate pentahydrate ($CuSO_4.5H_2O$) and 3.24 E-05 g/L of Zinc sulfate, heptahydrate, ($ZnSO_4.7H_2O$).

Furthermore, the medium may further comprise lipoic acid/thioctic acid, which may comprise at least about $5 \times 10^{-6}$ g/L of the solution. In one embodiment, the medium further comprises about 0.000015 g/L of lipoic acid/thioctic acid.

Optionally, the medium may further comprise putrescine, albumin, a stabilizer, and/or foaming agent. In one embodiment, the albumin is bovine serum albumin. In one embodiment, bovine serum albumin is provided in the form of the commercially available AlbuMAX® I (Gibco™ Cell Culture, Invitrogen Corporation, Carlsbad, CA), which is lipid-rich bovine serum albumin. In another embodiment, the medium further comprises the commercially available stabilizer/anti-foaming agent Pluronic® F68 (Invitrogen Corporation, Carlsbad, CA).

Exemplary suitable embodiments of the culture medium are shown in the table below:

|  | Embodiment A Amount (g/L) | Embodiment B Amount (g/L) | Embodiment C Amount (g/L) |
|---|---|---|---|
| **Inorganic Salts** | | | |
| Calcium Chloride, Anhydrous | 0.2 | At least about 0.05 | 0.05 - 0.4 |
| potassium chloride, USP | 0.4 | At least about 0.1 | 0.1 - 0.8 |
| Mg sulfate, anhydrous | 0.9767 | At least about 0.2 | 0.2 - 1.8 |
| sodium chloride, USP | 6.4 | At least about 5 | 5 - 8 |
| sodium phosphate, monobasic, $H_2O$, USP | 0.133175 | At least about 0.08 | 0.08 - 0.2 |
| sodium phosphate, dibasic heptahydrate ($Na_2HPO_4.7H_2O$) | 0.00201 | At least about 0.0005 | 0.0005 - 0.004 |
| **Trace Minerals** | | | |
| Ferric Nitrate (9 $H_2O$) | 0.0001 | At least about 5 E-05 | 0.00002 - 0.0002 |
| Copper(II)sulfate pentahydrate ($CuSO_4.5H_2O$) | 9.33E-08 | At least about 5E-08 | 5E-08 - 2E-07 |

(continued)

| Trace Minerals | | | |
|---|---|---|---|
| Zinc sulfate, heptahydrate, ($ZnSO_4.7H_2O$) | 3.24E-05 | At least about 1E-05 | 1E - 05 -5E-05 |
| Sodium Selenate ($Na_2SeO_3$) | 0.000067 | At least about 4E-05 | 3E-05 - 1.2E-04 |
| **Amino Acids** | | | |
| L-Arginine | 0.09705 | At least about 0.05 | 0.05 - 0.20 |
| L-Cystine | 0.06779 | At least about 0.02 | 0.02 - 0.12 |
| L-Cvsteine | 0.009224 | At least about 0.005 | 0.005 - 0.020 |
| L-Glutamine | 0.584 | At least about 0.2 | 0.2 - 1.0 |
| Glycine | 0.031125 | At least about 0.01 | 0.01 - 0.06 |
| L- Histidine | 0.048288 | At least about 0.02 | 0.01 - 0.09 |
| L- Isoleucine | 0.163713 | At least about 0.09 | 0.05 - 0.4 |
| L-Leucine | 0.163713 | At least about 0.09 | 0.05 - 0.4 |
| L-Lysine | 0.16807 | At least about 0.09 | 0.05 - 0.4 |
| L- Methionine | 0.036748 | At least about 0.02 | 0.01 - 0.07 |
| L-Phenylalanine | 0.073695 | At least about 0.05 | 0.02 - 0.14 |
| L-serine | 0.05145 | At least about 0.03 | 0.02 - 0.1 |
| L- Threonine | 0.108609 | At least about 0.08 | 0.04 - 0.2 |
| L-tryptophan | 0.018457 | At least about 0.009 | 0.005 - 0.04 |
| L-tyrosine | 0.121813 | At least about 0.08 | 0.04 - 0.25 |
| L-Valine | 0.111105 | At least about 0.08 | 0.04 - 0.25 |
| L-Alanine | 0.000668 | At least about 0.0004 | 0.0002 - 0.0013 |
| L-Aspargine | 0.031978 | At least about 0.01 | 0.01 - 0.07 |
| L-Aspartic Acid | 0.00803 | At least about 0.006 | 0.002 - 0.017 |
| L-Glutamic Acid | 0.054728 | At least about 0.03 | 0.02 - 0.1 |
| L- Proline | 0.02403 | At least about 0.005 | 0.005 - 0.05 |
| L-Taurine | 0.000844 | At least about 0.0006 | 0.0003 - 0.002 |
| **Vitamins** | | | |
| D-Calcium Pantothenate | 0.004338 | At least about 0.001 | 0.001 - 0.009 |
| Choline Chloride | 0.006094 | At least about 0.002 | 0.002 - 0.012 |
| Folic Acid | 0.004302 | At least about 0.001 | 0.001 - 0.009 |
| l-Inositol | 0.009568 | At least about 0.005 | 0.005 - 0.020 |
| Niacinamide | 0.004302 | At least about 0.001 | 0.001 - 0.009 |
| Pyridoxal | 0.004153 | At least about 0.001 | 0.001 - 0.009 |
| Riboflavin | 0.000431 | At least about 0.0001 | 0.0001 - 0.009 |
| thiamine | 0.004304 | At least about 0.001 | 0.001 - 0.009 |
| d-Biotin | 3.75E-05 | At least about 1E-05 | 1E-05 - 7E-05 |
| Pyridoxin | 1.85E-05 | At least about 8E-06 | 5E-06 - 4E-05 |
| Vitamin $B_{12}$ (cvanocobalamin) | 0.000102 | At least about 0.00002 | 0.00002 - 0.0002 |

(continued)

| Lipids | | | |
|---|---|---|---|
| Lipoic Acid/Thioctic Acid | 0.000015 | At least about 5E-06 | 5E-06 - 3E-05 |
| Ethanolamine | 0.02 | At least about 0.005 | 0.005 - 0.04 |
| **Proteins** | | | |
| Insulin | 0.01 | At least about 0.003 | 0.003 - 0.02 |
| Transferrin | 0.055 | At least about 0.03 | 0.03 - 0.12 |
| **Energy Substrates** | | | |
| D-Glucose | 1 | At least about 0.5 | 0.5 - 3 |
| Sodium Pyruvate | 0.11 | At least about 0.03 | 0.03 - 0.2 |
| **Nucleic Acid Derivatives** | | | |
| Thymidine | 0.00045 | At least about 0.0001 | 0.0001 - 0.0009 |
| Adenosine | 0.015 | At least about 0.005 | 0.005 - 0.03 |
| Cytidine | 0.015 | At least about 0.005 | 0.005 - 0.03 |
| Uridine | 0.015 | At least about 0.05 | 0.005 - 0.03 |
| Guanosine | 0.015 | At least about 0.005 | 0.005 - 0.03 |

These media may further comprise bovine serum albumin (BSA), putrescine, and Pluronic® F68. Specifically, for embodiment A, the medium may further comprise 3.02 E-05 g/L of putrescine, 2.5 g/L of BSA and 0.015 g/L of Pluronic® F68. Similarly, for embodiment B, the serum-free nutrient solution may further comprise at least about 1 E-05 g/L of putrescine, at 1 g/L of BSA and at least 0.005 g/L of Pluronic® F68. In one embodiment, the media further comprises putrescine and Pluronic® F68, such as *e.g.* 3.02 E-05 g/L of putrescine, 2.5 g/L of BSA and 0.015 g/L of Pluronic® F68.

The culture medium of the invention may further comprise at least 0.8 g/L of D-glucose and at least 0.1 g/L of sodium pyruvate. In one embodiment, culture medium of the invention further comprises about 1 g/L of D-glucose and about 0.11 g/L of sodium pyruvate.

In one embodiment of the invention, the culture medium is supplemented with serum, such as *e.g.* fetal bovine serum (FBS) or newborn calf serum (NCS). The serum content can range in concentration from 0 (a serum-free media) to 20% of the total volume of the medium. In one embodiment, the medium is supplemented with serum (such as *e.g.* FBS) during the preparation of the medium. In another embodiment, the medium is supplemented immediately prior to use (such as *e.g.* via the addition of a solution comprising serum *(e.g.* FBS)). In one embodiment, the culture medium is supplemented preferably with about 2 to 15% (v/v) of FBS, alternatively from about 2 to about 10%, alternatively from about 3 to about 12%, alternatively from about 5 to about 15%, alternatively from about 4% to about 10%. In the selected embodiments, the culture medium is supplemented with about 7.5%, about 10% or about 15% (v/v) of FBS.

**B. Serum-free nutrient solutions**

Another aspect of the invention is a serum-free nutrient solution comprising: amino acids; vitamins; salts; nucleosides; insulin; transferrin; ethanolamine; and sodium selenite. As used herein, with reference to the nutrient solution, the term "serum-free" means that the nutrient solution does not contain serum prior to addition to the culture medium. Unless otherwise indicated, the amounts disclosed for the components of the serum-free nutrient solution are based on measurements of the increase in weight of each component after the serum-free nutrient solution has been added to a culture.

In one embodiment, the serum-free nutrient solution comprises the common 20 L-amino acids. In another embodiment, the serum-free nutrient solution comprises the amino acids L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, and optionally L-Alanine, L-Aspargine, L-Aspartic Acid, L-Glutamic Acid, L-Proline and L-Taurine. In one embodiment, the solution comprises from at least about 0.0001 g/L to at least about 0.03 g/L of each of these amino acids. In another embodiment, the serum-free nutrient solution comprises at least about 0.005 g/L of L-Arginine, at least about 0.002 g/L of L-Cystine, at least about 0.0003 g/L of L-Cysteine, at least about 0.0005 g/L of Glycine, at least about 0.002 g/L of L-Histidine, at least about 0.01 g/L of L-Isoleucine, at least about 0.01 g/L of L-Leucine, at least about 0.008 g/L of L-Lysine, at least about 0.002 g/L of L-Methionine, at least about 0.002g/L of L-Phenylalanine, at least about 0.002 g/L of L-serine, at least about 0.003 g/L of L-Threonine, at least about 0.0008 g/L of L-tryptophan, at least about 0.005 g/L of L-tyrosine, at least about 0.005 g/L of L-Valine, at least about 0.0001 g/L of L-Alanine, at least about 0.005 g/L of L-Aspargine, at least about 0.002 g/L of L-Aspartic Acid, at least about 0.01 g/L of L-Glutamic Acid, at least about 0.008 g/L of L-Proline and at least about 0.008 g/L of L-Taurine. In an alternate embodiment, serum-free nutrient solution comprises about 0.013 g/L L-Arginine, HCl; about 0.005 g/L L-Cystine, 2HCl; about 0.009 g/L L-Cysteine HCl $H_2O$; about 0.001 g/L Glycine; about 0.006 g/L L-Histidine, HCl, $H_2O$; about 0.059 g/L L-Isoleucine; about 0.059 g/L L-Leucine; about 0.022 g/L L-Lysine, HCl; about 0.007 g/L L-Methionine; about 0.008 g/L L-Phenylalanine; about 0.009 g/L L-serine; about 0.013 g/L L-Threonine; about 0.002 g/L L-tryptophan; about 0.018 g/L L-tyrosine, 2Na, $2H_2O$; about 0.018 g/L L-Valine; about 0.001 g/L L-Alanine; about 0.032 g/L L-Aspargine $H_2O$; about 0.008 g/L L-Aspartic Acid; about 0.055 g/L L-Glutamic Acid; about 0.024 g/L L-Proline; and about 0.0008 L-Taurine. The amino acids are thought to help replenish consumed amino acids in culture.

The serum-free nutrient solution further comprises one or more vitamins. In one embodiment, serum-free nutrient solution comprises at least the following vitamins: D-calcium pantothenate; choline chloride; folic acid; I-inositol; niacinamide; pyridoxal; riboflavin; thiamine; d-Biotin; pyridoxine; and Vitamin $B_{12}$ (cyanocobalamin). The solution may further comprise Vitamin C and Vitamin A. In one embodiment, the solution comprises from about 5 x $10^{-6}$ g/L to 0.001 g/L of each of the one or more vitamins. In one embodiment, the serum-free nutrient solution comprises at least about 0.0001 g/L of D-calcium pantothenate; at least about 0.0008 g/L of choline chloride; at least about 0.0001 g/L of folic acid; at least about 0.0008 g/L of 1-inositol; at least about 0.0001 g/L of niacinamide; at least about 0.00005 g/L of pyridoxal; at least about 1x $10^{-5}$ g/L of riboflavin; at least about 0.00001 g/L of thiamine; at least about 1x $10^{-5}$ g/L of d-Biotin; at least about 1x $10^{-5}$ g/L of pyridoxine; and at least about 1x $10^{-5}$ g/L of Vitamin $B_{12}$ (cyanocobalamin). In one embodiment, the serum-free nutrient solution comprises about 0.000338 g/L D-Calcium Pantothenate; about 0.002094 g/L Choline Chloride; about 0.000302 g/L Folic Acid; about 0.002568 g/L I-Inositol; about 0.000302 g/L Niacinamide; about 0.000153 g/L Pyridoxal, HCl; about 3.11E-05 g/L Riboflavin; about 0.000304 g/L thiamine, HCl; about 3.75E-05 g/L d-Biotin; about 1.85E-05 g/L Pyridoxine HCl; and about 0.000102 g/L Vitamin $B_{12}$ (cyanocobalamin).

Additionally, the serum-free nutrient solution comprises one or more salts. In another embodiment, the solution comprises from about 0.0005 g/L to about 0.001 g/L of the phosphate salts. In an alternate embodiment, the solution comprises sodium phosphate such as sodium phosphate, monobasic and sodium phosphate, dibasic heptahydrate. In yet another embodiment, the solution comprises at least about 0.005 g/L of sodium phosphate, monobasic and at least about 0.001 g/L of sodium phosphate, dibasic heptahydrate. In an alternate embodiment, the solution comprises about 0.008 g/L of sodium phosphate, monobasic and about 0.002 g/L of sodium phosphate, dibasic heptahydrate.

The serum-free nutrient solution further comprises nucleosides. In one embodiment, the serum-free nutrient solution comprises the nucleic acid derivatives thymidine, adenosine, cytidine, uridine, and guanosine. In an alternate embodiment, the solution comprises at least about 0.0001 g/L to at least about 0.005 g/L of the each of the nucleosides. In one embodiment, the solution comprises at least about 0.0001 g/L of thymidine and least about 0.005 g/L of each of adenosine, cytidine, uridine, and guanosine. In another embodiment, the solution comprises about 0.0005 g/L of thymidine and about 0.015 g/L of each of adenosine, cytidine, uridine, and guanosine.

The serum-free nutrient solution further comprises insulin, transferrin, ethanolamine, and sodium selenite. In one embodiment, the solution comprises at least 0.002 g/L, alternatively about 0.01 g/L of insulin. In another embodiment, the solution comprises at least about 0.01 g/L, alternatively about 0.06 g/L of transferrin. In alternate embodiment, the solution comprise at least about 0.005 g/L, alternatively about 0.02 g/L of ethanolamine. In yet another embodiment, the solution comprises at least about 2 x $10^{-5}$ g/L, alternatively about 7 x $10^{-5}$ g/L of sodium selenite.

In addition to the trace mineral sodium selenite, in certain the embodiments, the serum-free nutrient solution further comprises one or more additional trace minerals. In one embodiment, the solution comprises about 3 x $10^{-8}$ g/L to about 2 x $10^{-5}$ g/L of each of the one or more additional trace minerals. In one embodiment, the solution further comprises Copper(II)sulfate pentahydrate ($CuSO_4.5H_2O$) and Zinc sulfate, heptahydrate, ($ZnSO_4.7H_2O$). In another embodiment, the solution further comprises at least about 3 x $10^{-8}$ g/L of Copper(II)sulfate pentahydrate ($CuSO_4.5H_2O$) and at least about 5 x $10^{-6}$ g/L of Zinc sulfate, heptahydrate, ($ZnSO_4.7H_2O$). In an alternate embodiment, the solution further comprises about 9 x $10^{-8}$ g/L of Copper(II)sulfate pentahydrate ($CuSO_4.5H_2O$) and about 3 x $10^{-5}$ g/L of Zinc sulfate, heptahydrate, ($ZnSO_4.7H_2O$).

Furthermore, in addition to the lipid ethanolamine, the serum-free nutrient solution may further comprise lipoic acid/thioctic acid, which may comprise at least about 1 x $10^{-5}$ g/L of the solution.

In one embodiment of the invention, the serum-free nutrient solution comprises:

*the amino acids* L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Aspargine, L-Aspartic Acid, L-Glutamic Acid, L-Proline and L-Taurine;
*the vitamins* D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);
*the salts* sodium phosphate, monobasic and sodium phosphate, dibasic heptahydrate;
*the nucleosides* adenosine, cytidine, uridine and guanosine;
insulin; transferrin; ethanolamine; and sodium selenite.

This serum-free solution may optionally further comprise Copper(II)sulfate pentahydrate ($CuSO_4.5H_2O$) and Zinc sulfate, heptahydrate, ($ZnSO_4.7H_2O$). Additionally, the serum-free solution may optionally further comprise lipoic acid/thioctic acid. In one embodiment, the solution comprises: (1) from about 0.0001 g/L to about 0.03 g/L of each of the amino acids; (2) from about 0.00001 g/L to about 0.001 g/L of each of the vitamins; (3) at least about $5 \times 10^{-6}$ g/L of sodium phosphate, monobasic and at least about 0.001 g/L of sodium phosphate, dibasic heptahydrate; (4) at least about 0.0001 g/L of thymidine and least about 0.005 g/L of each of adenosine, cytidine, uridine and guanosine; (5) at least 0.002 g/L of insulin; (6) at least about 0.03 g/L of transferrin; (7) at least about 0.005 g/L of ethanolamine and (8) at least about $5 \times 10^{-5}$ g/L of sodium selenite. Optionally, this solution further comprises (9) at least about $7 \times 10^{-8}$ g/L of Copper(II)sulfate pentahydrate ($CuSO_4.5H_2O$); (10) at least about $5 \times 10^{-6}$ g/L of Zinc sulfate, heptahydrate, ($ZnSO_4.7H_2O$); and (11) at least about $1 \times 10^{-6}$ g/L of lipoic acid/thioctic acid.

In another embodiment of the invention, the serum-free nutrient solution comprises amino acids, vitamins, salts, nucleosides, Copper(II)sulfate pentahydrate ($CuSO_4.5H_2O$), zinc sulfate, heptahydrate, ($ZnSO_4.7H_2O$) and lipoic/thioctic acid. In that embodiment, the solution comprises:

*the amino acids* L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Aspargine, L-Aspartic Acid, L-Glutamic Acid, L-Proline and L-Taurine;
*the vitamins* D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);
*the salts* sodium phosphate, monobasic and sodium phosphate, dibasic heptahydrate; and
*the nucleosides* thymidine, adenosine, cytidine, uridine and guanosine.

In one embodiment, this serum-free nutrient solution comprises at least about 0.005 g/L of L-Arginine, at least about 0.002 g/L of L-Cystine, at least about 0.0003 g/L of L-Cysteine, at least about 0.0005 g/L of Glycine, at least about 0.002 g/L of L-Histidine, at least about 0.01 g/L of L-Isoleucine, at least about 0.01 g/L of L-Leucine, at least about 0.008 g/L of L-Lysine, at least about 0.002 g/L of L-Methionine, at least about 0.002g/L of L-Phenylalanine, at least about 0.002 g/L of L-serine, at least about 0.003 g/L of L-Threonine, at least about 0.0008 g/L of L-tryptophan, at least about 0.005 g/L of L-tyrosine, at least about 0.005 g/L of L-Valine, at least about 0.0001 g/L of L-Alanine, at least about 0.005 g/L of L-Aspargine, at least about 0.002 g/L of L-Aspartic Acid, at least about 0.01 g/L of L-Glutamic Acid, at least about 0.008 g/L of L-Proline and at least about 0.008 g/L of L-Taurine.. In another embodiment, this serum-free nutrient solution further comprises at least about 0.0001 g/L of D-calcium pantothenate; at least about 0.0008 g/L of choline chloride; at least about 0.0001 g/L of folic acid; at least about 0.0008 g/L of 1-inositol; at least about 0.0001 g/L of niacinamide; at least about 0.00005 g/L of pyridoxal; at least about $1 \times 10^{-5}$ g/L of riboflavin; at least about 0.00001 g/L of thiamine; at least about $1 \times 10^{-5}$ g/L of d-Biotin; at least about $1 \times 10^{-5}$ g/L of pyridoxine; and at least about $1 \times 10^{-5}$ g/L of Vitamin B12 (cyanocobalamin). In an alternate embodiment, this solution further comprises at least about 0.0001 g/L of thymidine and least about 0.01 g/L of each of adenosine, cytidine, uridine, and guanosine. In another embodiment, this solution further comprises at least about $3 \times 10^{-8}$ g/L of Copper(II)sulfate pentahydrate ($CuSO_4.5H_2O$), about $5 \times 10^{-5}$ g/L of Zinc sulfate, heptahydrate, ($ZnSO_4.7H_2O$) and about $2 \times 10^{-5}$ g/L of lipoic acid/thioctic acid.

Optionally, the serum-free nutrient solutions may further comprise putrescine, bovine serum albumin, a stabilizer, and/or foaming agent. In one embodiment, bovine serum albumin is provided in the form of the commercially available AlbuMAX® I (Gibco™ Cell Culture, Invitrogen Corporation, Carlsbad, CA), which is lipid-rich bovine serum albumin. In another embodiment, the serum-free nutrient solution comprises the commercially available stabilizer/anti-foaming agent Pluronic® F68 (Invitrogen Corporation, Carlsbad, CA).

Exemplary suitable embodiments of the serum-free nutrient solution are shown in the table below:

| | Embodiment A Amount (g/L) | Embodiment B Amount (g/L) | Embodiment C Amount (g/L) |
|---|---|---|---|
| **Inorganic Salts** | | | |
| sodium phosphate, monobasic, $H_2O$, USP | 0.008175 | At least about 0.001 | 0.001 - 0.016 |

(continued)

| | Embodiment A Amount (g/L) | Embodiment B Amount (g/L) | Embodiment C Amount (g/L) |
|---|---|---|---|
| **Inorganic Salts** | | | |
| sodium phosphate, dibasic heptahydrate ($Na_2HPO_4.7H_2O$) | 0.00201 | At least about 0.0005 | 0.0005 - 0.004 |
| **Trace Minerals** | | | |
| Copper(II)sulfate pentahydrate ($CuSO_4.5H_2O$) | 9.33E-08 | At least about 3E-08 | 3E-08 - 2E-07 |
| Zinc sulfate, heptahydrate, ($ZnSO_4.7H_2O$) | 3.24E-05 | At least about 5E-06 | 5E-06 - 7E-05 |
| Sodium Selenate $Na_2SeO_3$ | 0.000067 | At least about 0.00002 | 0.00002 - 0.00014 |
| **Amino Acids** | | | |
| L-Arginine, HCl | 0.01305 | At least about 0.005 | 0.005-0.03 |
| L-Cystine, 2HCl | 0.00522 | At least about 0.002 | 0.002 - 0.00522 |
| L-Cysteine HCl $H_2O$ | 0.009224 | At least about 0.003 | 0.003-0.02 |
| Glycine | 0.001125 | At least about 0.0005 | 0.0005 - 0.0025 |
| L-Histidine, HCl, $H_2O$ | 0.006288 | At least about 0.002 | 0.002 - 0.015 |
| L-Isoleucine | 0.058913 | At least about 0.01 | 0.01-0.12 |
| L- Leucine | 0.058913 | At least about 0.01 | 0.01-0.12 |
| L-Lysine, HCl | 0.02187 | At least about 0.008 | 0.008 - 0.05 |
| L- Methionine | 0.006748 | At least about 0.002 | 0.002 - 0.015 |
| L-Phenylalanine | 0.007695 | At least about 0.002 | 0.002 - 0.02 |
| L-Serine | 0.00945 | At least about 0.002 | 0.002 - 0.02 |
| L-Threonine | 0.013409 | At least about 0.003 | 0.003-0.03 |
| L-tryptophan | 0.002457 | At least about 0.0008 | 0.0008 - 0.005 |
| L-tyrosine, 2Na, $2H_2O$ | 0.018023 | At least about 0.005 | 0.005 - 0.04 |
| L-Valine | 0.017505 | At least about 0.005 | 0.005 - 0.04 |
| L-Alanine | 0.000668 | At least about 0.0001 | 0.0001 -0.002 |
| L-Aspargine$H_2O$ | 0.031978 | At least about 0.005 | 0.005 -0.07 |
| L-Aspartic Acid | 0.00803 | At least about 0.002 | 0.002 - 0.02 |
| L-Glutamic Acid | 0.054728 | At least about 0.01 | 0.01-0.12 |
| L- Proline | 0.02403 | At least about 0.008 | 0.008 - 0.05 |
| L-Taurine | 0.000844 | At least about 0.0002 | 0.0002 - 0.002 |
| **Vitamins** | | | |
| D-Calcium Pantothenate | 0.000338 | At least about 0.0001 | 0.0001 - 0.0007 |
| Choline Chloride | 0.002094 | At least about 0.0008 | 0.0008 - 0.005 |
| Folic Acid | 0.000302 | At least about 0.0001 | 0.0001 - 0.0007 |
| I-Inositol | 0.002568 | At least about 0.0008 | 0.0008 - 0.005 |
| Niacinamide | 0.000302 | At least about 0.0001 | 0.0001 - 0.0007 |
| Pyridoxal, HCl | 0.000153 | At least about 0.00005 | 0.00005 - 0.0004 |

(continued)

| Vitamins | | | |
|---|---|---|---|
| Riboflavin | 3.11E-05 | At least about 1E-05 | 1E-05 -7E-05 |
| thiamine, HCl | 0.000304 | At least about 0.0001 | 0.0001 - 0.0007 |
| d-Biotin | 3.75E-05 | At least about 1E-05 | 1E-05 -8E-05 |
| Pvridoxine. HCl | 1.85E-05 | At least about 5E-06 | 5E-06 - 4E-05 |
| Vitamin $B_{12}$ (cyanocobalamin) | 0.000102 | At least about 0.00002 | 0.00002 - 0.00025 |
| Lipids | | | |
| Lipoic Acid/Thioctic Acid | 0.000015 | At least about 0.000005 | 0.000005 - 0.00004 |
| Ethanolamine HCl | 0.02 | At least about 0.005 | 0.005 - 0.05 |
| Proteins | | | |
| Insulin | 0.01 | At least about 0.002 | 0.002 - 0.025 |
| Transferrin | 0.055 | At least about 0.01 | 0.01 - 0.15 |
| Nucleic Acid Derivatives | | | |
| Thymidine | 0.00045 | At least about 0.0001 | 0.0001 - 0.001 |
| Adenosine | 0.015 | At least about 0.005 | 0.005 - 0.04 |
| Cytidine | 0.015 | At least about 0.005 | 0.005 - 0.04 |
| Uridine | 0.015 | At least about 0.005 | 0.005 - 0.04 |
| Guanosine | 0.015 | At least about 0.005 | 0.005 - 0.04 |

These serum-free nutrient solutions may further comprise bovine serum albumin (BSA), putrescine, and Pluronic® F68. Specifically, for embodiment A, the serum-free nutrient solution may further comprise 3.02 E-05 g/L of putrescine, 2.5 g/L of BSA and 0.015 g/L of Pluronic® F68. Similarly, for embodiment B, the serum-free nutrient solution may further comprise at least about 1 E-05 g/L of putrescine, at 1 g/L of BSA and at least 0.005 g/L of Pluronic® F68. In one preferred embodiment, the serum-free nutrient solutions further comprise putrescine and Pluronic® F68.

In embodiment, the serum-free nutrient solution comprises sodium selenate, ethanolamine, insulin, transferrin, thymidine, adenosine, cytidine, uridine, guanosine, and serum albumin (*e.g.* bovine serum albumin).

Optionally, the serum-free nutrient solution may further comprise one or more energy substrate such as *e.g.* glucose and/or pyruvate.

In one embodiment of the invention, the culture medium and/or serum-free nutrient solution do not contain exogenously added factors that promote growth of undifferentiated cells. In one preferred embodiment, the culture medium and/or serum-free nutrient solution do not comprise a fibroblast growth factor, such as *e.g.* basic FGF or FGF-4.

## II. Human Umbilical Cord Tissue-Derived cells (hUTC)

As discussed above in certain embodiments of the invention, the culture medium and serum-free nutrient solutions may be used to grow isolated human umbilical cord tissue derived-cells ("hUTC" or "UTC"). The UTC and UTC populations suitable for use with the culture medium, serum-free nutrient solution, kits and methods of the present invention are described in detail in detailed herein below as well as U.S. Patent Nos. 7,510,873; 7,524,489; and U.S. Pub. App. No. 2005/005863, which are incorporated by reference in their entireties as they relate to the description, isolation and characterization of hUTC.

## A. Isolation and Growth of Umbilical Cord-Tissue Derived Cells

According to the methods described herein, a mammalian umbilical cord is recovered upon or shortly after termination of either a full-term or a pre-term pregnancy, e.g., after expulsion of after birth. The postpartum tissue may be transported from the birth site to a laboratory in a sterile container such as a flask, beaker, culture dish, or bag. The container may have a solution or medium, including but not limited to a salt solution, such as Dulbecco's Modified Eagle's Medium (DMEM) (also known as Dulbecco's Minimal Essential Medium) or phosphate buffered saline (PBS), or any solution used for transportation of organs used for transplantation, such as University of Wisconsin solution or perfluorochemical solution. One or more antibiotic and/or antimycotic agents, such as but not limited to penicillin, streptomycin, amphotericin B, gentamicin, and nystatin, may be added to the medium or buffer. The postpartum tissue may be rinsed with an anticoagulant solution such as heparin-containing solution. It is preferable to keep the tissue at about 4-10°C prior to extraction of UTC. It is even more preferable that the tissue not be frozen prior to extraction of UTC.

Isolation of UTC preferably occurs in an aseptic environment. The umbilical cord may be separated from the placenta by means known in the art. Alternatively, the umbilical cord and placenta are used without separation. Blood and debris are preferably removed from the postpartum tissue prior to isolation of UTC. For example, the postpartum tissue may be washed with buffer solution, including but not limited to phosphate buffered saline. The wash buffer also may comprise one or more antimycotic and/or antibiotic agents, including but not limited to penicillin, streptomycin, amphotericin B, gentamicin, and nystatin.

Postpartum tissue comprising an umbilical cord or a fragment or section thereof is disaggregated by mechanical force (mincing or shear forces). In a presently preferred embodiment, the isolation procedure also utilizes an enzymatic digestion process. Many enzymes are known in the art to be useful for the isolation of individual cells from complex tissue matrices to facilitate growth in culture. Digestion enzymes range from weakly digestive (*e.g.* deoxyribonucleases and the neutral protease, dispase) to strongly digestive (*e.g.* papain and trypsin), and are available commercially. A non-exhaustive list of enzymes compatible herewith includes mucolytic enzyme activities, metalloproteases, neutral proteases, serine proteases (such as trypsin, chymotrypsin, or elastase), and deoxyribonucleases. Presently preferred are enzyme activities selected from metalloproteases, neutral proteases and mucolytic activities. For example, collagenases are known to be useful for isolating various cells from tissues. Deoxyribonucleases can digest single-stranded DNA and can minimize cell clumping during isolation. Preferred methods involve enzymatic treatment with *e.g.* collagenase and dispase, or collagenase, dispase, and hyaluronidase. In certain embodiments, a mixture of collagenase and the neutral protease dispase are used in the dissociating step. More specific embodiments employ digestion in the presence of at least one collagenase from *Clostridium histolyticum,* and either of the protease activities, dispase, and thermolysin. Still other embodiments employ digestion with both collagenase and dispase enzyme activities. Also utilized are methods that include digestion with a hyaluronidase activity in addition to collagenase and dispase activities. The skilled artisan will appreciate that many such enzyme treatments are known in the art for isolating cells from various tissue sources. For example, the enzyme blends for tissue disassociation sold under the trade name LIBERASE (Roche, Indianapolis, Ind.) are suitable for use in the instant methods. Other sources of enzymes are known, and the skilled artisan may also obtain such enzymes directly from their natural sources. The skilled artisan is also well-equipped to assess new or additional enzymes or enzyme combinations for their utility in isolating the cells of the invention. Preferred enzyme treatments are 0.5, 1, 1.5, or 2 hours long or longer. In other preferred embodiments, the tissue is incubated at 37 °C during the enzyme treatment of the dissociation step.

In some embodiments of the invention, postpartum tissue is separated into sections comprising various aspects of the tissue, such as neonatal, neonatal/maternal, and maternal aspects of the placenta, for instance. The separated sections then are dissociated by mechanical and/or enzymatic dissociation according to the methods described herein. Cells of neonatal or maternal lineage may be identified by any means known in the art, *e.g.* by karyotype analysis or *in situ* hybridization for a Y chromosome.

Isolated cells or umbilical cord tissue from which a UTC is derived may be used to initiate, or seed, cell cultures. Isolated cells are transferred to sterile tissue culture vessels either uncoated or coated with extracellular matrix or ligands such as laminin, collagen (native, denatured or cross-linked), gelatin, fibronectin, and other extracellular matrix proteins. In addition to the culture media disclosed herein, a UTC may be cultured in any culture medium capable of sustaining growth of the cell such as, but not limited to, DMEM (high or low glucose), advanced DMEM, DMEM/MCDB 201, Eagle's basal medium, Ham's F10 medium (F10), Ham's F-12 medium (F12), Iscove's modified Dulbecco's medium, Mesenchymal Stem Cell Growth Medium (MSCGM), DMEM/F12, RPMI 1640, and serum/media free medium sold under the trade name CELL-GRO-FREE (Mediatch, Inc., Herndon, Va.). The culture medium may be supplemented with one or more components including, e.g., fetal bovine serum (FBS), preferably about 2-15% (v/v); equine serum (ES); human serum (HS); beta-mercaptoethanol (BME or 2-ME), preferably about 0.001% (v/v); one or more growth factors, e.g., platelet-derived growth factor (PDGF), epidermal growth factor (EGF), fibroblast growth factor (FGF), vascular endothelial growth factor (VEGF), insulin-like growth factor-1 (IGF-1), leukocyte inhibitory factor (LIF) and erythropoietin (EPO); amino acids, including L-valine; and one or more antibiotic and/or antimycotic agents to control microbial contamination, such as penicillin G, streptomycin sulfate, amphotericin B, gentamicin, and nystatin, either alone or in combination. The culture medium may comprise Growth Medium as defined in the Examples.

The cells are seeded in culture vessels at a density to allow cell growth. In a preferred embodiment, the cells are cultured at about 0 to about 5% by volume $CO_2$ in air. In some preferred embodiments, the cells are cultured at about 2 to about 25% $O_2$ in air, preferably about 5 to about 20% $O_2$ in air. The cells preferably are cultured at a temperature of about 25 to about 40 °C. and more preferably are cultured at 37 °C. The cells are preferably cultured in an incubator. The medium in the culture vessel can be static or agitated, e.g., using a bioreactor. The UTC is preferably grown under low oxidative stress (e.g., with addition of glutathione, Vitamin C, Catalase, Vitamin E, N-Acetylcysteine). "Low oxidative stress" refers to conditions of no or minimal free radical damage to the cultured cells.

Methods for the selection of the most appropriate culture medium, medium preparation, and cell culture techniques are well known in the art and are described in a variety of sources, including Doyle et al., (eds.), 1995, Cell & Tissue Culture: Laboratory Procedures, John Wiley & Sons, Chichester; and Ho and Wang (eds.), 1991, Animal Cell Bioreactors, Butterworth-Heinemann, Boston, which are incorporated herein by reference.

After culturing the isolated cells or tissue fragments for a sufficient period, a UTC will have grown out, either because of migration from the postpartum tissue or cell division, or both. In some embodiments of the invention, the UTC is passaged, or removed to a separate culture vessel containing fresh medium of the same or a different type as that used initially, where the population of cells can be mitotically expanded. The cells of the invention may be used at any point between passage 0 and senescence. The cells preferably are passaged between about 3 and about 25 times, more preferably are passaged about 4 to about 12 times, and preferably are passaged 10 or 11 times. Cloning and/or subcloning may be performed to confirm that a clonal population of cells has been isolated.

In certain embodiments, the different cell types present in postpartum tissue are fractionated into subpopulations from which the UTC can be isolated. Fractionation or selection may be accomplished using standard techniques for cell separation including, but not limited to, enzymatic treatment to dissociate postpartum tissue into its component cells, followed by cloning and selection of specific cell types, including but not limited to selection based on morphological and/or biochemical markers; selective growth of desired cells (positive selection), selective destruction of unwanted cells (negative selection); separation based upon differential cell agglutinability in the mixed population such as, *e.g.,* with soybean agglutinin; freeze-thaw procedures; differential adherence properties of the cells in the mixed population; filtration; conventional and zonal centrifugation; centrifugal elutriation (counter-streaming centrifugation); unit gravity separation; countercurrent distribution; electrophoresis; and fluorescence activated cell sorting (FACS). For a review of clonal selection and cell separation techniques, see Freshney, 1994, Culture of Animal Cells: A Manual of Basic Techniques, 3rd Ed., Wiley-Liss, Inc., New York, which is incorporated herein by reference.

The culture medium is changed as necessary, *e.g.*, by carefully aspirating the medium from the dish, *e.g.,* with a pipette, and replenishing with fresh medium. Incubation is continued until a sufficient number or density of cells accumulates in the dish. The original explanted tissue sections may be removed and the remaining cells trypsinized using standard techniques or using a cell scraper. After trypsinization, the cells are collected, removed to fresh medium, and incubated as above. In some embodiments, the medium is changed at least once at approximately 24 hours post-trypsinization to remove any floating cells. The cells remaining in culture are considered to be UTC.

The UTC may be cryopreserved. Accordingly, UTC for autologous transfer (for either the mother or child) may be derived from appropriate postpartum tissues following the birth of a child, then cryopreserved so as to be available in the event they are later needed for transplantation.

## B. Characteristics of Umbilical Cord Tissue-Derived Cells

The UTC may be characterized, *e.g.,* by growth characteristics *(e.g.,* population doubling capability, doubling time, passages to senescence), karyotype analysis (e.g., normal karyotype; maternal or neonatal lineage), flow cytometry *(e.g.*, FACS analysis), immunohistochemistry and/or immunocytochemistry *(e.g.,* for detection of epitopes), gene expression profiling *(e.g.,* gene chip arrays; polymerase chain reaction *(e.g.,* reverse transcriptase PCR, real time PCR, and conventional PCR)), protein arrays, protein secretion (*e.g.*, by plasma clotting assay or analysis of PDC-conditioned medium, e.g., by Enzyme Linked ImmunoSorbent Assay (ELISA)), mixed lymphocyte reaction (e.g., as measure of stimulation of PBMCs), and/or other methods known in the art.

Examples of suitable UTC derived from umbilicus tissue were deposited with the American Type Culture Collection (10801 University Boulevard, Manassas, VA 20110) on June 10, 2004, and assigned ATCC Accession Numbers as follows: (1) strain designation UMB 022803 (P7) was assigned Accession No. PTA-6067; and (2) strain designation UMB 022803 (P17) was assigned Accession No. PTA-6068.

In various embodiments, the UTC possesses one or more of the following growth features: (1) they require L-valine for growth in culture; (2) they are capable of growth in atmospheres containing oxygen from about 5% to at least about 20% (3) they have the potential for at least about 40 doublings in culture before reaching senescence; and (4) they attach and expand on a coated or uncoated tissue culture vessel, wherein the coated tissue culture vessel comprises a coating of gelatin, laminin, collagen, polyornithine, vitronectin or fibronectin.

In certain embodiments, the UTC possesses a normal karyotype, which is maintained as the cells are passaged. Methods for karyotyping are available and known to those of skill in the art.

In other embodiments, the UTC may be characterized by production of certain proteins, including (1) production of at least one of tissue factor, vimentin, and alpha-smooth muscle actin; and (2) production of at least one of CD10, CD13, CD44, CD73, CD90, PDGFr-alpha, PD-L2 and HLA-A,B,C cell surface markers, as detected by flow cytometry. In other embodiments, the UTC may be characterized by lack of production of at least one of CD31, CD34, CD45, CD80, CD86, CD117, CD141, CD178, B7-H2, HLA-G, and HLA-DR, DP, DQ cell surface markers, as detected by flow cytometry. Particularly preferred are cells that produce at least two of tissue factor, vimentin, and alpha-smooth muscle actin. More preferred are those cells producing all three of the proteins tissue factor, vimentin, and alpha-smooth muscle actin.

In other embodiments, the UTC may be characterized by gene expression, which relative to a human cell that is a fibroblast, a mesenchymal stem cell, or an iliac crest bone marrow cell, is increased for a gene encoding at least one of interleukin 8; reticulon 1; chemokine (C-X-C motif) ligand 1 (melonoma growth stimulating activity, alpha); chemokine (C-X-C motif) ligand 6 (granulocyte chemotactic protein 2); chemokine (C-X-C motif) ligand 3; and tumor necrosis factor, alpha-induced protein 3.

In yet other embodiments, the UTC may be characterized by gene expression, which relative to a human cell that is a fibroblast, a mesenchymal stem cell, or an iliac crest bone marrow cell, is reduced for a gene encoding at least one of: short stature homeobox 2; heat shock 27 kDa protein 2; chemokine (C-X-C motif) ligand 12 (stromal cell-derived factor 1); elastin (supravalvular aortic stenosis, Williams-Beuren syndrome); *Homo sapiens* mRNA; cDNA DKFZp586M2022 (from clone DKFZp586M2022); mesenchyme homeo box 2 (growth arrest-specific homeo box); sine oculis homeobox homolog 1 (Drosophila); crystallin, alpha B; disheveled associated activator of morphogenesis 2; DKFZP586B2420 protein; similar to neuralin 1; tetranectin (plasminogen binding protein); src homology three (SH3) and cysteine rich domain; cholesterol 25-hydroxylase; runt-related transcription factor 3; interleukin 11 receptor, alpha; procollagen C-endopeptidase enhancer; frizzled homolog 7 (Drosophila); hypothetical gene BC008967; collagen, type VIII, alpha 1; tenascin C (hexabrachion); iroquois homeobox protein 5; hephaestin; integrin, beta 8; synaptic vesicle glycoprotein 2; neuroblastoma, suppression of tumorigenicity 1; insulin-like growth factor binding protein 2, 36 kDa; *Homo sapiens* cDNA FLJ12280 fis, clone MAMMA1001744; cytokine receptor-like factor 1; potassium intermediate/small conductance calcium-activated channel, subfamily N, member 4; integrin, beta 7; transcriptional co-activator with PDZ-binding motif (TAZ); sine oculis homeobox homolog 2 (Drosophila); KIAA1034 protein; vesicle-associated membrane protein 5 (myobrevin); EGF-containing fibulin-like extracellular matrix protein 1; early growth response 3; distal-less homeo box 5; hypothetical protein FLJ20373; aldo-keto reductase family 1, member C3 (3-alpha hydroxysteroid dehydrogenase, type II); biglycan; transcriptional co-activator with PDZ-binding motif (TAZ); fibronectin 1; proenkephalin; integrin, beta-like 1 (with EGF-like repeat domains); *Homo sapiens* mRNA full length insert cDNA clone EUROIMAGE 1968422; EphA3; KIAA0367 protein; natriuretic peptide receptor C/guanylate cyclase C (atrionatriuretic peptide receptor C); hypothetical protein FLJ14054; *Homo sapiens* mRNA; cDNA DKFZp564B222 (from clone DKFZp564B222); BCL2/adenovirus E1B 19 kDa interacting protein 3-like; AE binding protein 1; and cytochrome c oxidase subunit VIIa polypeptide 1 (muscle).

In other embodiments, the UTC may be characterized when cultured by secretion of at least one of MCP-1, IL-6, IL-8, GCP-2, HGF, KGF, FGF, HB-EGF, BDNF, TPO, MIP1b, 1309, MDC RANTES, and TIMP1. In addition, the UTC may be characterized when cultured by lack of secretion of at least one of TGF-beta2, ANG2, PDGFbb, MIP1A, and VEGF, as detected by ELISA.

In some embodiments, the UTC is derived from umbilical cord tissue substantially free of blood, are capable of self-renewal and expansion in culture, require L-valine for growth, can grow in at least about 5% oxygen, and comprise at least one of the following characteristics: potential for at least about 40 doublings in culture; attachment and expansion on a coated or uncoated tissue culture vessel that comprises a coating of gelatin, laminin, collagen, polyornithine, vitronectin, or fibronectin; production of vimentin and alpha-smooth muscle actin; production of CD10, CD13, CD44, CD73, and CD90; and, expression of a gene, which relative to a human cell that is a fibroblast, a mesenchymal stem cell, or an iliac crest bone marrow cell, is increased for a gene encoding interleukin 8 and reticulon 1. In some embodiments, such UTC does not produce CD45 and CD117.

In preferred embodiments, the cell comprises two or more of the above-listed growth, protein/surface marker production, gene expression, or substance-secretion characteristics. More preferred is a cell comprising three, four, five, or more of the characteristics. Still more preferred is a UTC comprising six, seven, eight, or more of the characteristics. Still more preferred presently is a cell comprising all of above characteristics.

Among cells that are presently preferred for use with the invention in several of its aspects are postpartum cells having the characteristics described above and more particularly those wherein the cells have normal karyotypes and maintain normal karyotypes with passaging, and further wherein the cells express each of the markers CD10, CD13, CD44, CD73, CD90, PDGFr-alpha, and HLA-A, B,C, wherein the cells produce the immunologically-detectable proteins which correspond to the listed markers. Still more preferred are those cells which in addition to the foregoing do not produce proteins corresponding to any of the markers CD31, CD34, CD45, CD117, CD141, or HLA-DR,DP,DQ, as detected by flow cytometry.

In one embodiment, the UTC are isolated from human umbilical cord tissue substantially free of blood, are capable of self-renewal and expansion in culture, have the potential to differentiate, lack the production of CD117 or CD45, and do not express hTERT or telomerase. These UTC optionally express oxidized low density lipoprotein receptor 1, reticulon, chemokine receptor ligand 3, and/or granulocyte chemotactic protein; and/or do not express CD31 or CD34; and/or express, relative to a human fibroblast, mesenchymal stem cell, or iliac crest bone marrow cell, increased levels of interleukin 8 or reticulon 1; and/or express CD10, CD13, CD44, CD73, and CD90.

In another embodiment, the UTC are isolated from human umbilical cord tissue substantially free of blood, are capable of self-renewal and expansion in culture, have the potential to differentiate, express CD13 and CD90, and do not express CD34 and CD117. Optionally, these cells do not express hTERT or telomerase. In yet another embodiment, the cells also express CD10, CD44, and CD43. In an alternate embodiment, the cells also do not express CD45 and CD31. These UTC optionally (i) express oxidized low density lipoprotein receptor 1, reticulon, chemokine receptor ligand 3, and/or granulocyte chemotactic protein; and/or (ii) express, relative to a human fibroblast, mesenchymal stem cell, or iliac crest bone marrow cell, increased levels of interleukin 8 or reticulon 1.

In another embodiment, the UTC are isolated from human umbilical cord tissue substantially free of blood, are capable of self-renewal and expansion in culture, have the potential to differentiate, express CD13, CD90, and HLA-ABC, and do not express CD34, CD117, and HLA-DR. Optionally, these cells also do not express hTERT or telomerase. In one embodiment, the cells also express CD10, CD44, and CD43. In an alternate embodiment, the cells also do not express CD45 and CD31. These UTC optionally (i) express oxidized low density lipoprotein receptor 1, reticulon, chemokine receptor ligand 3, and/or granulocyte chemotactic protein; and/or (ii) express, relative to a human fibroblast, mesenchymal stem cell, or iliac crest bone marrow cell, increased levels of interleukin 8 or reticulon 1.

In alternate embodiment, the UTC are isolated from human umbilical cord tissue substantially free of blood, are capable of self-renewal and expansion in culture, have the potential to differentiate, and have the following characteristics: (1) express CD10, CD13, CD44, CD90, and HLA-ABC; (2) do not express CD31, CD34, CD45, HLA-DR and CD117, and (3) do not express hTERT or telomerase. In alternate embodiment, the UTC are isolated from human umbilical cord tissue substantially free of blood, are capable of self-renewal and expansion in culture, have the potential to differentiate, and have the following characteristics: (1) express CD10, CD13, CD44, CD90, and HLA-ABC; (2) do not express CD31, CD34, CD45, HLA-DR and CD117; (3) do not express hTERT or telomerase; (4) express oxidized low density lipoprotein receptor 1, reticulon, chemokine receptor ligand 3, and/or granulocyte chemotactic protein; and (4) express, relative to a human fibroblast, mesenchymal stem cell, or iliac crest bone marrow cell, increased levels of interleukin 8 or reticulon 1.

In one embodiment, the hUTC are provided as a population of cells, which may be homogenous. In some embodiments, the cell population may be heterogeneous. A heterogeneous cell population of the invention may comprise at least about 5%, 10%, 20%, 30%, 40%, 50%, 60%, 70%, 80%, 90%, or 95% UTC of the invention. The heterogeneous cell populations of the invention may further comprise stem cells or other progenitor cells, such as myoblasts or other muscle progenitor cells, hemangioblasts, or blood vessel precursor cells; or it may further comprise fully differentiated skeletal muscle cells, smooth muscle cells, pericytes, or blood vessel endothelial cells. In some embodiments, the population is substantially homogeneous, *i.e.,* comprises substantially only the UTC (preferably at least about 96%, 97%, 98%, 99% or more UTC). The homogeneous cell populations of the invention are comprised of umbilicus-derived cells. Homogeneous populations of umbilicus-derived cells are preferably free of cells of maternal lineage. Homogeneity of a cell population may be achieved by any method known in the art, *e.g.,* by cell sorting (*e.g.,* flow cytometry) or by clonal expansion in accordance with known methods. Homogeneous UTC populations may comprise a clonal cell line of postpartum-derived cells.

In one embodiment, the hUTC after culturing have substantially the same characteristics (*e.g.* marker profile and/or gene expression profile) as the hUTC before culturing. In an alternate embodiment, the hUTC after culturing have the same characteristics (*e.g.* marker profile and/or gene expression profile) as the hUTC before culturing. In one embodiment, the hUTC after culturing have the same characteristics as the hUTC before culturing for at least CD13, CD34, CD90, and CD117.

### III. Other Suitable Cells

As discussed above in certain embodiments of the invention, the culture medium and serum-free nutrient solutions may be used to grow isolated human umbilical cord tissue derived-cells ("hUTC" or "UTC"). In addition, the culture medium and serum-free nutrient solutions may be used to grow other anchorage dependent cells.

In one embodiment of the invention, the culture medium and serum-free nutrient solution are utilized to grow other anchorage dependent cells such as cells derived from placenta. The examples of other anchorage dependent cells include but are not limited to bone marrow derived mesenchymal stem cells, bone marrow derived progenitors of mesenchymal stem cells, cells derived from non-marrow tissues, such as adipose tissue, muscle tissue, blood vessel including internal mammary artery derived cells, cells derived from the dental pulp of teeth. Additionally, amniotic fluid has been shown to be a very rich source of anchorage dependent stem cells. Another example of anchorage dependent cells are fibroblasts including neonatal foreskin fibroblasts.

## IV. Methods of Culturing

Another embodiment of the invention is methods of culturing cells comprising use of the conditioned medium and serum-free nutrient solution of the invention. The methods may utilize roller bottles, spinner flasks, and/or microcarriers. In preferred embodiments, the methods are used to culture anchorage-dependent cells, such as *e.g.* hUTC cells, and require microcarriers.

In a preferred embodiment, the method comprises culturing hUTC with the conditioned medium and serum-free nutrient solution of the invention without the need for serum exchange. As discussed above, UTC can be grown in a variety of culture media. The methods of the invention optimally reduce the serum usage and increase volumetric productivity to reduce the cost of manufacturing compositions comprising anchorage dependent cells, *e.g.* hUTC. Furthermore, the method allows for growth of the cells (*e.g.* hUTC) without medium exchange.

Exemplary methods of growing cells in roller bottles and microcarriers are disclosed in U.S. Pub. App. Nos. 2007/0141700 and 2008/0166328, respectively, which are incorporated herein by reference in its entirety as it relates to the description of roller bottle, microcarriers and culturing on roller bottles and microcarriers. Exemplary suitable roller bottles, spinner flasks, microcarriers, characteristics thereof and suitable culture parameters are discussed below.

### A. Roller Bottles

Roller bottle culture systems are known in the art of cell culture. As used herein, roller bottle culture systems comprise at least a cell line of interest, growth medium, roller bottles, an apparatus for rotating the bottles, and means for harvesting the cells.

Roller bottle culture systems typically further comprise means for controlling the temperature during the incubation, as well as means for aseptically handling the cultures, *e.g.* during initial seeding of the bottles with cells, or during subsequent transfers. Harvesting of the cells may be achieved through enzymatic treatment such as with trypsin, trypsin-EDTA, dispase, and collagenase, or other enzymes or combinations of enzymes with or without other components. Other commercial products such as but not limited to TrypLE™ Express (Gibco, Inc.) can be utilized. The cells be also be harvested by manual operations including, *e.g.*, batch centrifugation, or harvesting can be automated.

In one embodiment, the bottles are filled about 100-300 ml of growth medium, in other embodiments about 100-200 ml are used. In an alternate embodiment about 100-120 ml, or even 105-115 ml are placed in the bottles. In other embodiments, the bottles are filled with about 112 ml of growth medium to achieve maximal population doublings. In one embodiment, the bottles are seeded with about 2,500 to about 10,000 cells/cm$^2$. In a one embodiment, the lower end of that range is used, for example seeding is with less than about 3000 cells per square centimeter. The seeding bottles are rotated during attachment and growth. The rotational speed may be set at between about 0.5 to 1 rpm. Preferably, the rotation is between about 0.75 and 1 rpm. More preferably, the bottles are rotated at about 0.8 to 1 rpm.

In another embodiment, roller bottles are filled about 100-300 ml of growth medium, preferably about 300 ml are used. The bottles are seeded with about 2500 to about 10,000 cells per square centimeter. In one embodiment, the lower end of that range is used, for example seeding is with less than about 3000 cells per square centimeter. Still more preferred are embodiments where the seeding is at about 2500 cells/cm$^2$. The seeded bottles are rotated during attachment and growth. The rotational speed is set at between about 0.5 to 1 rpm. Preferably, the rotation is between about 0.75 and 1 rpm. More preferably, the bottles are rotated at about 0.8 to 1 rpm. Rotation near or about 0.9 -1.0 rpm is presently preferred, as shown in the figure.

The filled and seeded roller bottles are rotated and incubated for about 5 to 7 days to achieve maximal doublings. Presently, an incubation time of about 5.5 to about 6.5 days is preferred.

The roller bottles may be coated with an agent that aids in the attachment of the cells to the inner surface of the roller bottles, such as gelatin, extracellular matrix molecules (such as gelatin, laminin, vitronectin, fibronectin, collagen types I, IV, and VI), or the like. One example of such commercially available coated bottles are those coated with CellBind (available from Corning as catalog number 3907). It is envisioned that various coating agents will be found acceptable for attachment and growth of cells in accordance with the methods provided herein.

### B. Spinner Flasks

Spinner flask culture systems are also known in the art of cell culture. As used herein, spinner flask culture systems comprise at least a cell line of interest, growth medium, one or more spinner flask, a means for rotating the one or more flask, and a means for harvesting the cells.

Spinner flask culture systems typically further comprise means for controlling the temperature during the incubation, as well as means for aseptically handling the cultures, *e.g.* during initial seeding of the flasks with cells, or during subsequent transfers. Harvesting of the cells may be achieved through enzymatic treatment such as with trypsin, trypsin-EDTA, dispase, and collagenase, or other enzymes or combinations of enzymes with or without other components.

Other commercial products such as but not limited to TrypLE™ Express (Gibco, Inc.) can utilized. The cells be also be harvested by manual operations including, *e.g.*, batch centrifugation, or harvesting can be automated.

In one embodiment, the rotational speed is set at between about 35 to about 65 rpm, alternatively between about 35 and 45 rpm, alternatively from between about 40 and about 50 rpm, alternatively between about 45 rpm and about 55 rpm, alternatively from between about 55 to about 65 rpm, alternatively from about 50 to about 60 rpm. In preferred embodiments, a rotational speed of about 40 rpm or 60 rpm is maintained.

In one embodiment, 3 L spinner flasks are used which may be filled with approximately 3 L of growth medium, FBS, serum-free nutrient solution, microcarriers, and cells. In that embodiment, the rotational speed may be set between about 35 and 45 rpm. Preferably, the rotation is about 40 rpm.

In another embodiment, 125 ml flasks are used. These flasks may be filled with approximately 100 ml of a solution comprising the growth medium, FBS, serum-free nutrient solution, microcarriers, and cells. In one embodiment, the flasks contain approximately 85 ml to 115 ml of growth medium, FBS, serum-free nutrient solution, microcarriers, and cells. In that embodiment, the rotational speed may be set between about 55 and 65 rpm. Preferably, the rotation is about 50 rpm.

In yet another embodiment, 500 ml spinner flasks are used. These flasks may be filled with approximately 500 ml of a solution comprising the growth medium, FBS, serum-free nutrient solution, microcarriers, and cells. In one embodiment, the flasks contain approximately 450 ml to 500 ml of growth medium, FBS, serum-free nutrient solution, microcarriers, and cells. In that embodiment, the rotational speed may be set between about 55 and 65 rpm. Preferably, the rotation is about 50 rpm.

In an alternate embodiment, 500 ml spinner flasks are used. These flasks may be filled with approximately 500 ml of a solution comprising the growth medium, FBS, serum-free nutrient solution, microcarriers, and cells. In one embodiment, the flasks contain approximately 450 ml to 500 ml of growth medium, FBS, serum-free nutrient solution, microcarriers, and cells. In that embodiment, the rotational speed may be set between about 35 and 45 rpm. Preferably, the rotation is about 40 rpm.

The flasks may be seeded with about 2,500 to about 10,000 cells/cm$^2$. The cells may be seeded directly into the flask, onto the surface of the flask or onto microcarrier placed inside the flask. In a preferred embodiments, the seeding is carried with about 3,000 to about 7,500, alternatively with about 3,000 to about 7,000, alternatively with about 4,000 to about 7,000, alternatively with about 3,000 to about 5,000, alternatively with about 5,000 to about 7,000, alternatively with about 3,500 to about 5,000, alternatively with about 4,500 to about 7,500, alternatively about 3,500 to about 5,500 cells/cm$^2$. The flasks are rotated during attachment and growth. In one embodiment, to maximize the population doubling rate, the filled and seeded flasks are rotated and incubated for about 5 to 7 days, with an incubation time of about 5 to about 6 days preferred.

## C. Microcarriers

Microcarrier culture is a technique, which makes possible the practical high yield culture of anchorage-dependent cells, *e.g.*, anchorage-dependent postpartum cells. Microcarriers have been specifically developed for the culture of cells, such as mammalian postpartum cells, in culture volumes ranging from a few milliliters to greater than one thousand liters. The microcarrier is biologically inert and provides a strong but non-rigid substrate for stirred microcarrier cultures. The microcarriers may be transparent, allowing microscopic examination of the attached cells. Cytodex® 3 (GE Healthcare Life Sciences, Piscataway N.J.) consists of a thin layer of denatured collagen chemically coupled to a matrix of cross-linked dextran. The denatured collagen layer on Cytodex® 3 is susceptible to digestion by a variety of proteases, including trypsin and collagenase, and provides the ability to remove cells from the microcarriers while maintaining maximum cell viability, function, and integrity.

Protein free microcarriers can be used to culture the cells. For example, microcarrier beads for use in manufacturing and laboratory or research use sold under the tradename HILLEX® (SoloHill Engineering, Inc., Ann Arbor, MI) are modified polystyrene beads with cationic trimethyl ammonium attached to the surface to provide a positively charged surface to the microcarrier. The bead diameter may range from about 90 to about 200 μm in diameter.

Microcarrier-based methods of cell culture provide many advantages including ease of downstream processing in many applications. Microcarriers are typically roughly spherical in shape, and can be either porous or solid. The use of microcarriers for cell attachment facilitates the use of stirred tank and related reactors for growth of anchorage-dependent cells. The cells attach to the readily suspended microparticles. The requirement for suspendability limits the physical parameters of the microcarriers. Thus, microcarriers commonly have a mean diameter in the range of 50-2000 μm. In some applications solid-type microcarriers range from about 100 to about 250 μm whereas porous-type micro-carrier beads range from about 250 to about 2500 μm. These size ranges allow for selection of microcarriers, which are large enough to accommodate many anchorage-dependent cells, while small enough to form suspensions with properties suitable for use in stirred reactors.

Among the factors considered in using microcarrier beads and the like are: attachment efficiency, immunogenicity, biocompatibility, ability to biodegrade, time to reach confluence, the growth parameters of attached cells including maximum attainable density per unit surface area, detachment techniques where required, and the efficiency of the detachment, scalability of the culture conditions as well as homogeneity of the culture under scaled-up conditions, the ability to successfully scale-up detachment procedures, and whether the beads will be used for implantation. These considerations can be influenced by the surface properties of the microcarrier beads, as well as by the porosity, diameter, density, and handling properties of the microcarrier.

For example, the density of the microcarrier particles or beads is a consideration. Excessive density may cause the microcarrier particles or beads to settle out of the suspension, or tend to remain completely towards the bottom of the culture vessel, and thus may result in poor bulk mixing of the cells, culture medium, and gaseous phases in the reactor. On the other hand, a density that is too low may result in excessive floating of the microcarrier. A density of 1.02 to 1.15 g/cm$^3$ is typical of many microcarrier beads.

The small diameter of microcarrier particles and the volume of particles that can be added to a reactor allows the microcarriers to contribute substantial surface area in vast excess to that found in roller bottles or other methods of growing anchorage-dependent cells, *e.g.* on plates. Porous microcarriers provide even greater surface area per unit volume or weight. These porous microcarriers possess large cavities that are available for the growth of anchorage-dependent cells. These cavities increase the surface area greatly, and may protect cells from detrimental mechanical effects, such as shear stress, *e.g.* from mixing or from gas sparging.

The microcarrier surface may be textured to enhance cell attachment and proliferation. The microcarrier surface texture be achieved by techniques including, but not limited to, molding, casting, leeching, and etching. The resolution of the features of the textured surface may be on the nanoscale. The textured surface may be used to induce a specific cell alignment on the microcarrier surface. The surface of the pores within the porous microcarriers may also be textured to enhance cell attachment and proliferation. Pore surface texture be achieved by techniques such as but not limited to molding, casting, leeching, and etching.

The microcarrier surface may be plasma-coated to impart a specific charge to microcarrier surfaces. These charges may enhance cell attachment and proliferation.

In other embodiments, the microcarriers comprise, or are coated with, thermoresponsive polymers *e.g.* poly-N-isopropylacrylamide, or have electromechanical properties. The microcarriers may also possess a microcurrent, such as microcarriers with a particulate galvanic couple of zinc and copper that produces low levels of biologically relevant electricity. The microcarriers may be paramagnetic, such as paramagnetic calcium-alginate microcarriers.

Both porous and solid types of microparticulate carriers are commercially available. Examples of commercially available solid microcarriers include Cytodex® 1 and Cytodex® 3, both of which are dextran-based microcarriers from GE Healthcare Life Sciences. Suitable commercially available porous microcarriers include Cytoline™ and Cytopore™ from GE Healthcare Life Sciences, Biosilon (NUNC) and Cultispher® (Percell Biolytical).

In certain embodiments, the methods and kits of the invention utilize a dextran bead microcarrier having an approximate particle size of about 60-90 μm and a density of about 1.03 g/cm$^3$ at 25°C. In other embodiments, the methods and kits of the invention utilize a dextran bead microcarrier having an approximate particle size of about 114-198 μm and a density of about 1.04 g/cm$^3$ at 25°C. In yet another embodiment, the methods and kits of the invention utilize a dextran bead microcarrier having an approximate particle size of about 60-87 μm and a density of about 1.04 g/cm$^3$ at 25°C. In an alternate embodiment, the methods and kits of the invention utilize porous microcarriers. These porous microcarriers may have a particle diameter of about 200-280 μm, an effective surface area of about 1.1 m$^2$/g dry and an average pore diameter opening of about 30 μm. In other embodiments, the methods and kits of the invention utilize microcarriers having an amine treated surface. In preferred embodiments, the microcarriers having an amine treated surface have a particle size of about 160-200 μm, a relative density range of about 1.090-1.150, a surface area of about 515 cm$^2$/g. Such microcarriers may be provided in solutions contains $5.5 \times 10^5$ microcarriers/g.

The carrier particles may also contain a bioactive agent. The carrier particle may also contain a bioactive agent or factor that may regulate the growth or function of cells or the tissue milieu. Suitable factors include but are not limited to fibroblast growth factors, erythropoietin, vascular endothelial cell growth factors, platelet-derived growth factors, bone morphogenic proteins, transforming growth factors, tumor necrosis factors, epidermal growth factors, and insulin-like growth factors. Complete factors, mimetics, or active fragments thereof may be used.

## D. Methods

Generally, the methods of the invention comprise culturing (*e.g.* expanding) cells in a culture medium of the invention, which has been supplemented with serum (*e.g.* FBS). After the cells have been grown to a desired density (such as e.g. for a period of about 3-4 days), the serum-free nutrient solution is added. The cells may be anchorage-dependent cells, which may be seeded on a microcarrier. The culturing may be carried out in a roller bottle or spinner flask culture system. Preferably, when the anchorage-dependent cells are seeded on a microcarrier, a spinner flask culture system is used. The desired period of time is determined by such parameters as *e.g.* desired population density, desired population doubling(s) or time. In certain embodiments, the cells are cultured for 4 to 7 days with the serum-free nutrient medium added at about day 3. In other embodiments, the cells are grown for 1-2 or population doublings or until a desired initial population density is achieved prior to addition of the serum-free nutrient solution. As discussed above, the methods allow for growth of the cells (e.g. hUTC) without medium exchange

## 1. Methods of Culturing Isolated Anchorage-Dependent Cells

One embodiment of the invention is a method for culturing (e.g. expanding) anchorage-dependent cells. The method comprises culturing isolated anchorage-dependent cells seeded on tissue flask surface or preferably microcarrier in a culture medium of the invention, which has been supplemented with serum, and adding a serum-free nutrient solution after the cells have been grown for a sufficient period of time to allow for a desired initial population density. In one embodiment, the cells are grown between about 3 and about 7 days, alternatively between about 3 and about 5 days, alternatively between about 4 days and about 5 days, prior to addition of the serum-free nutrient solution. In one embodiment, the cells are grown about 3 days prior to addition the serum-free nutrient solution. In another embodiment, the cells are grown to allow for at least one or two population doublings prior to addition to the serum-free nutrient solution. In a preferred embodiment, the cells are seeded on any of the microcarriers disclosed herein and are grown in spinner flasks under the conditions described above. In one embodiment, the method comprises thawing a cell bank vial containing these cells and expansion of the cells to inoculate a production vessel. Another embodiment comprises the step of first isolating the cells and then seeding the isolated cells.

As discussed, the medium, including the medium used in these methods, may be supplemented with about 2% to about 20% of serum (e.g. FBS). In one embodiment, the medium is supplemented with serum (such as e.g. FBS) during the preparation of the medium. In another embodiment, the medium is supplemented immediately prior to use (such as via the addition of a solution comprising serum (e.g. FBS)). In another embodiment, the medium is supplemented with preferably about 2 to 15% (v/v) of FBS, alternatively from about 2 to about 10%, alternatively from about 3 to about 12%, alternatively from about 5 to about 15%, alternatively from about 4% to about 10%. In the selected embodiments, the culture medium is supplemented with about 7.5%, about 10% or about 15% (v/v) of FBS.

In another embodiment, the method also encompasses seeding the anchorage-dependent cells. While the target seeding density may vary, in certain embodiments, from about 5,000 to about 8,000, alternatively from about 5,500 to about 7,500, alternatively from about 6,000 to about 8,000, alternatively from about 6,500 to about 7,500 viable cells/cm$^2$ are seeded at a microcarrier concentration of about 12 to about 30, alternatively of about 12 to 20, alternatively of about 18 to about 25, alternatively of about 15 to about 23 g/L.

## 2. Methods of Culturing Isolated Umbilical Cord-Tissue Derived Cells

One embodiment of the invention is a method of culturing umbilical cord tissue-derived cells. While this method generally comprises the steps of the method of culturing isolated anchorage-dependent cells discussed above, there may be some variations. Accordingly one embodiment of the invention is a method of culturing isolated anchorage-dependent hUTC attached on microcarriers to high cell density in suspension culture in spinner flasks by enriching the growth medium with a serum-free nutrient solution. This method optimally eliminates the need for medium exchange (e.g. on day 3) to consistently grow cells >20,000 cells/cm$^2$. Furthermore, this method improves robustness for passaging of hUTC. This method utilizes the culture media and serum-free nutrient solutions of the invention.

The method comprises culturing isolated hUTC seeded on a microcarrier in a culture medium of the invention supplemented with serum (e.g. FBS) for a sufficient period of time to allow the cells to achieve a desired initial population density. In certain embodiments, culture medium embodiment A, B or C are used. In certain embodiments, the cells are cultured in a roller bottle and the culturing is carried out on a roller bottle system. In preferred embodiment, the cells are seeded on microcarriers, cultured in spinner flasks and the culturing is carried out in the spinner flask system. In one embodiment, cells are incubated at approximately 37 °C under a 10% CO$_2$ atmosphere.

In another embodiment, the flasks are rotated at a rate of from about 55 to about 65 rpm, alternatively from about 55 rpm to about 60 rpm, alternatively from about 58 rpm to about 61 rpm. In another embodiment, the flasks are rotated at a rate of from about 35 rpm to about 45 rpm, alternatively from about 38 rpm to about 42 rpm, alternatively from about 40 rpm to about 43 rpm, alternatively from about 36 rpm to about 45 rpm. In another embodiment, the microcarrier density in the medium is from about 11.0 to about 13.0 g/L.

In one embodiment, the hUTC are cultured at approximately 37 °C under a 10% CO$_2$ atmosphere in a 125 ml flask, which is rotated at an rpm range of about 55 rpm to about 65 rpm (preferably about 60 rpm). In yet another embodiment, the hUTC are cultured at approximately 37 °C under a 10% CO$_2$ atmosphere in a 500 ml flask, which is rotated at an rpm range of about 55 rpm to about 65 rpm (preferably about 60 rpm). In another embodiment, the hUTC are cultured at approximately 37 °C under a 10% CO$_2$ atmosphere in a 500 ml flask, which is rotated at an rpm range of about 35 rpm to about 45 rpm (preferably about 40 rpm). In another embodiment, the hUTC are cultured at approximately 37 °C under a 10% $CO_2$ atmosphere in a 3 1 flask, which is rotated at an rpm range of about 35 rpm to about 45 rpm (preferably about 40 rpm). In these embodiments, the flasks may contain about 11.0 to about 13.0 g/L of microcarrier in the medium (preferably about 12 g/L).

To maximize the population density, the filled and seeded flasks or roller bottles contain hUTC are rotated and incubated for at least about 5 to 7 days, with an incubation time of about 5 to about 6 days preferred. At approximately the half point of incubation (*i.e.* after about 2.5 to about 3.5 days (preferably 3 days)) or when the cells have achieved the desired initial cell density, the serum free-nutrient solution of the invention is added to the hUTC culture. In certain embodiments, serum free-nutrient solutions embodiment A, B or C are used.

In a preferred embodiment, the cells are grown for a sufficient period of time to allow the cells to achieve a desired initial population density prior to addition of the serum-free nutrient solution.

Thus, in one embodiment, the method comprises growing umbilical cord tissue-derived cells seeded on microcarriers in a culture medium of the invention for a sufficient period of time to allow for the cells to achieve a desired initial population density; adding a serum-free nutrient solution of the invention after the cells have achieved the desired initial population density; and growing the cells for a sufficient period of time to allow for the cells to achieve a desired final population density.

In another embodiment, culture medium embodiment A and serum-free nutrient solution embodiment A are used. In another embodiment, culture medium embodiment B and serum-free nutrient solution embodiment B are used. In one embodiment, culture medium embodiment C and serum-free nutrient solution embodiment C are used.

In one embodiment, the method comprises seeding microcarriers with hUTC prior to culturing. The microcarriers may be any one of the microcarriers mentioned above. In certain embodiments, the microcarriers have an amine treated surface. In preferred embodiments, the microcarriers having an amine treated surface have a particle size of about 160-200 $\mu$m, a relative density range of about 1.090-1.150, a surface area of about 515 $cm^2$/g. In one embodiment, the microcarrier is Hillex® II Ultra.

While the target seeding density may vary, in certain embodiments, from about 5,000 to about 8,000, alternatively from about 5,500 to about 7,500, alternatively from about 6,000 to about 8,000, alternatively from about 6,500 to about 7,500 viable cells/$cm^2$ are seeded at a microcarrier concentration of about 15 to about 30, alternatively of about 18 to about 25, alternatively of about 15 to about 23 g/L. The seeded cells are added to a flask. Alternatively, the seeding is carried out in a flask. In one embodiment, the seeding comprises thawing cryopreserved hTUC. In one embodiment, the method further comprises thawing cryopreserved hTUC and expansion of the cells prior to seeding the microcarrier. In one embodiment, the expansion of the cells is carried out on a microcarrier.

In one embodiment, the culture media used in the method to grow hUTC is supplemented with about 2% to about 20% of serum (*e.g.* FBS). In one embodiment, medium is supplemented with serum (such as *e.g.* FBS) during the preparation of the medium. In another embodiment, the medium is supplemented immediately prior to use (such as via the addition of a solution comprising serum (*e.g.* FBS)). In one embodiment, the culture medium is supplemented with preferably about 2 to 15% (v/v) of FBS, alternatively from about 2 to about 10%, alternatively from about 3 to about 12%, alternatively from about 5 to about 15%, alternatively from about 4% to about 10%, alternatively from about 8 % to about 15%, alternatively from about 10% to about 15%. In the selected embodiments, the culture medium is supplemented with about 7.5%, about 10% or about 15% (v/v) of FBS.

## 3. Other Characteristics of Methods of the Invention

The independent variables in the methods of the invention which may be used to maximize the number of population doublings achievable in spinner flask or roller bottle culture without need for medium exchange are rotational speed, seeding density of the cells into the bottles, amount of microcarrier, time of incubation, type of culture medium, type of serum-free nutrient solution and volume of medium placed in the bottle. The skilled artisan will appreciate that other values outside of the tested ranges could be routinely tested using the same methodology, and these values may prove to offer incremental gains in the number of population doublings. Maximal response of the dependent variable, here the number of population doublings achieved is measured as a function of these parameters and embodiments not specifically exemplified herein are contemplated as part of this disclosure.

The cells cultured according to the methods provided (such as *e.g.* hUTC) are characterized as having substantially the same cell surface marker profile or gene expression profile as the starting cells. In one embodiment, the cells cultured according to the methods provided are characterized as having substantially the same cell surface marker profile or gene expression profile as the starting cells. For many applications of cell-based therapies, it is important that the cellular characteristics do not change when scaling up the culture conditions to increase quantities. For example, the morphology, cell surface markers, and expression of hallmark genes that help distinguish or denote the therapeutic cell should remain substantially unchanged if not identical. The cells provided in accordance with the invention and the methods taught therein are substantially unchanged, or preferably identical in such characteristics as the same cells grown under laboratory conditions and scale.

## V. Kits Comprising the Culture Medium and Serum-Free Nutrient Solution

Another embodiment of the invention is a kit for growing cells comprising the culture media of the invention and the serum-free nutrient solution. In one embodiment, the kit further comprises the cells. In one preferred embodiment, the kit comprises human umbilical cord tissue-derived cells. The kit may also further comprise instructions of use. Optionally, the kit further comprises a microcarriers and serum such as fetal bovine serum. In an alternate embodiment, the kit may also comprise a roller bottle system.

Without further description, it is believed that one of ordinary skill in the art can, using the preceding description and the following illustrative examples, make and utilize the present invention and practice the claimed methods. The following working examples therefore, specifically point out the preferred embodiments of the present invention, and are not to be construed as limiting in any way the remainder of the disclosure.

## EXAMPLES

## EXAMPLE 1

## Growth and Harvest of hUTC on Microcarriers in Spinner Flasks with Enriched Media

In this example the growth and harvest of hUTC on microcarriers in spinner flasks with various enriched media was investigated. For the studies in this example, umbilical cord tissue-derived cells were expanded from an inoculum and then grown under a variety of different growth conditions (Conditions 1-9), each of which employed different culture media as outlined below.

The microcarriers used in the studies was Hillex® Ultra (Solohill Engineering, Ann Arbor, MI), which have a surface area of 515 $cm^2$/g.

As used in this example, the term "passage" is defined as inoculating a vessel containing fresh microcarriers with confluent microcarriers from a separate vessel. Furthermore, as used in this example, the term "population doubling" is defined as the number of times the population of cells has doubled over a given time and is calculated as follows:

$$Population\ Doubling\ in\ current\ passage$$

$$= \frac{(\ln(\#\ of\ viable\ cells\ at\ end\ of\ passage) - \ln(\#\ of\ viable\ cells\ at\ start\ of\ passage))}{\ln(2)}$$

## Procedure for Cell Count

The cell count as required by the various conditions was carried out by the following procedure. A 10 ml sample was aseptically taken from the culture and transferred into a 15 ml conical tube. The sample was then centrifuged at 1600 RPM for 5 minutes. The supernatant was carefully removed making sure not to remove any microcarriers. 5 to 10 ml of TrypLE™ Select (Gibco®) pre-warmed to 37 °C was then added to the microcarriers and agitated on a rotator for 15 to 30 minutes. The contents of the centrifuge tube were then transferred into a 50 ml conical tube through a 40-$\mu$m filter to remove the microcarriers and only allow passage of detached cells. Two washes with 1 x PBS were performed through the filter. The 50 ml conical tube was then centrifuged at 1600 RPM for 5 minutes. The supernatant was carefully removed without disturbing the cell pallet until about 1 to 2 ml of supernatant was left in the tube. This leftover volume was measured using a pipette and the cell resuspended in this volume. The resultant cell suspension was counted using CEDEX (Innovatis) cell counting equipment which uses the Trypan Blue exclusion method. Based on the CEDEX cell count the cell concentration in the culture vessel was calculated as follows:

$$Cell\ concentration\ in\ culture\ vessel,\ cells/mL$$

$$= \frac{Resuspension\ Volume,\ mL\ X\ CEDEX\ cell\ count,\ cells/mL}{Sample\ Volume,\ mL}$$

*Cell Density in culture vessel, cells/sq.cm*

$$= \frac{(Cell\ concentration\ in\ culture\ vessel,\ cells/mL\ X\ Culture\ Volume,\ mL)}{(weight\ of\ microcarriers\ in\ culture\ vessel,\ gm)\ X\ (surface\ area\ per\ gram\ of\ microcarrier,\ sq.\ cm/gm)}$$

**Cell Culturing Procedure**

Vials of frozen hUTC were thawed and washed with fresh medium. The cell suspension was transferred into a 125 ml glass spinner flask containing the medium and microcarrier. These thawed cells were used to prepare the inoculum.

**Preparation of cells for Inoculum**

The inoculum was expanded over multiple passages in control medium comprised of DMEM and 15% v/v of FBS with 12 g/L concentration of microcarriers in glass spinner flasks (Corning, NY) of different sizes (125 ml, 500 ml and 3 L). The cumulative population doublings of the cells in the inoculum was less than 35. The criteria for passage and the culture conditions during expansion of inoculum are tabulated in table below. On the target day of passage, a cell count was performed and if the target density for passage was achieved, the cells were passaged into a new vessel based on the target seeding density in the new vessel. If the target density for passage was not achieved, an 80% medium exchange was carried out and the cells counted on the next day. For medium exchange, the vessel was set aside from the spinner platform and the microcarriers were allowed to settle by gravity for 5 minutes and 80% of the medium was removed aseptically (without removing the microcarriers) and an equal amount of fresh medium was added and the vessel was placed back on the spinner platform in the incubator.

The growth conditions and parameters during serial passaging for inoculum preparation are outlined below in Table 1-1.

| Table 1-1: Growth conditions and parameters during serial passaging for inoculums preparation. | |
| --- | --- |
| Seeding Density of New Gulture Vessels | Target (Range) |
| At Thaw, 125 ml spinner flask (viable cells/cm$^2$) | 6000 (3000-7500) |
| At Passage 1, 500 ml spinner flasks (viable cells/cm$^2$) | 5000 (3000-7000) |
| At all other passages (viable cells/cm$^2$) | 6000 (4000-7000) |
| Duration of Passages | Target |
| After Thaw, 125 ml spinner flask (days) | 4 (4-5) |
| At all other passages from 500 ml spinner flask (days) | 3 (3-4) |
| 3L spinner flask (days). Always 4 days with 80% medium exchange on Day 3 | 4 |
| Cell Density at End of Passage | Target |
| Cell Density at End of Passage (viable cells/cm$^2$) | $\geq$ 20000 |
| Spinner Flask Working Volumes | Target (Range) |
| 125 ml flask working volume (ml) | 100 (85-115) |
| 500 ml flask working volume (ml) | 500 (450-500) |
| Incubator Parameters | Target |
| Temperature (°C) | 37.0 |
| $CO_2$(%) | 10 |
| Spinner Plate Parameters | Target (Range) |
| Agitation in 125 ml flask (rpm) | 60 (55-65) |
| Agitation in 500 ml flask (rpm) | 60 (55-65) |

(continued)

| Spinner Plate Parameters | Target (Range) |
|---|---|
| Agitation in 3 L flask (rpm) | 40 (35-45) |
| Microcarrier Density | Target (Range) |
| Density of microcarrier in medium | 12 (11.0-13.0) |

**Various Growth Conditions Used in this Example**

For each of Conditions 1 to 9, the day of inoculation is considered to be Day 0. Also for each of the conditions, a cell count was performed on the inoculum.

**Condition 1 (Control):** The target seeding density of this condition was ~6000 viable cells/cm$^2$ at a microcarrier concentration of ~18 g/L. Based on the cell count of the inoculums the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~9g of microcarriers in the flask. Fresh medium comprised of DMEM and 15% (v/v) of FBS was added to bring up the volume to 500 ml. The flask was placed on a spinner platform at 60 RPM in a 37 °C and 10% $CO_2$ incubator. On Day 3, an 80% medium exchange was carried out using DMEM + 15% FBS. Periodic cell counts were performed using the procedure described above. On Day 3 and 5, 3 ml and 2.5 ml of Glucose were added to the flask, respectively. 2.5 ml of 200 mM L-Glutamine was added on Day 3.

**Condition 2:** The target seeding density of this condition was ~6000 viable cells/cm$^2$ at a microcarrier concentration of ~20 g/L. Based on the cell count of the inoculums the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M5 basal medium and 15% (v/v) FBS was added to bring up the volume to 500 ml. The flask was placed on a spinner platform at 60 RPM in a 37 °C and 10% $CO_2$ incubator. On Day 3, feed F5 was added. No medium exchange was performed. In addition, on Day 3, 2.5 ml of glucose was added. Periodic cell counts were performed as explained in the cell counting section. On Day 5,5 ml of 200 mM L-Glutamine was added to the flask.

**Condition 3:** The target seeding density of this condition was ~7000 viable cells/cm$^2$ at a microcarrier concentration of ~20 g/L. Based on the cell count of the inoculum the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M1 basal medium and 15% (v/v) FBS was added to bring up the volume to 500 ml. The flask was placed on a spinner platform at 45 RPM in a 37 °C and 10% $CO_2$ incubator. On Day 3, feed F1 was added. No medium exchange was performed. Periodic cell counts were performed as explained in the cell counting section. 2.5 ml of glucose was added on Day 3. On Day 4, 5 ml of 200 mM L-Glutamine was added to the flask.

**Condition 4:** The target seeding density of this condition was ~7000 viable cells/cm$^2$ at a microcarrier concentration of ~20 g/L. Based on the cell count of the inoculum the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M2 basal medium and 15% (v/v) FBS was added to bring up the volume to 500 ml. The flask was placed on a spinner platform at 45 RPM in a 37 °C and 10% $CO_2$ incubator. On Day 3, feed F2 was added. No medium exchange was performed. Periodic cell counts were performed as explained in the cell counting section. 2.5 ml of glucose (aqueous solution of 220 g/L of dextrose monohydrate) was added on Day 3. On Day 4, 5 ml of 200 mM L-Glutamine was added to the flask.

**Condition 5:** The target seeding density of this condition was ~7000 viable cells/cm$^2$ at a microcarrier concentration of ~20 g/L. Based on the cell count of the inoculum the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M3 basal medium and 15% (v/v) FBS was added to bring up the volume to 500 ml. The flask was placed on a spinner platform at 45 RPM in a 37 °C and 10% $CO_2$ incubator. On Day 3, feed F3 was added. No medium exchange was performed. Periodic cell counts were performed as explained in the cell counting section. 2.5 ml of glucose (aqueous solution of 220 g/L of dextrose monohydrate) was added on Day 3. On Day 4, 5 ml of 200 mM L-Glutamine was added to the flask.

**Condition 6:** The target seeding density of this condition was ~7000 viable cells/cm$^2$ at a microcarrier concentration of ~20 g/L. Based on the cell count of the inoculum the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M4 basal medium and 15% (v/v) of FBS was added to bring up the volume to 500 ml. The flask was placed on a spinner platform at 45 RPM in a 37 °C and 10% CO$_2$ incubator. On Day 3, feed F4 was added. No medium exchange was performed. Periodic cell counts were performed as explained in the cell counting section. 2.5 ml of glucose (aqueous solution of 220 g/L of dextrose monohydrate) was added on Day 3. On Day 4, 5 ml of 200 mM L-Glutamine was added to the flask.

**Condition 7:** The target seeding density of this condition was ~7000 viable cells/cm$^2$ at a microcarrier concentration of ~20 g/L. Based on the cell count of the inoculum the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M5 basal medium and 15% (v/v) of FBS was added to bring up the volume to 500 ml. The flask was placed on a spinner platform at 45 RPM in a 37 °C and 10% CO$_2$ incubator. On Day 3, feed F5 was added. No medium exchange was performed. Periodic cell counts were performed as explained in the cell counting section. 2.5 ml of glucose (aqueous solution of 220 g/L of dextrose monohydrate) was added on Day 3. On Day 4, 5 ml of 200 mM L-Glutamine was added to the flask.

**Condition 8:** The target seeding density of this condition was ~7000 viable cells/cm$^2$ at a microcarrier concentration of ~20 g/L. Based on the cell count of the inoculum the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M6 basal medium and 15% (v/v) of FBS was added to bring up the volume to 500 ml. The flask was placed on a spinner platform at 45 RPM in a 37 °C and 10% CO$_2$ incubator. On Day 3, feed F6 was added. No medium exchange was performed. Periodic cell counts were performed as explained in the cell counting section. 2.5 ml of glucose (aqueous solution of 220 g/L of dextrose monohydrate) was added on Day 3. On Day 4, 5 ml of 200 mM L-Glutamine was added to the flask.

**Condition 9:** The target seeding density of this condition was ~7000 viable cells/cm$^2$ at a microcarrier concentration of ~20 g/L. Based on the cell count of the inoculum the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M7 basal medium and 15% (v/v) of FBS was added to bring up the volume to 500 ml. The flask was placed on a spinner platform at 45 RPM in a 37 °C and 10% CO$_2$ incubator. On Day 3, feed F7 was added. No medium exchange was performed. Periodic cell counts were performed as explained in the cell counting section. 2.5 ml of glucose (aqueous solution of 220 g/L of dextrose monohydrate) was added on Day 3. On Day 4, 5 ml of 200 mM L-Glutamine was added to the flask.

**Harvest**

On day 6, all the flasks were harvested. For harvest, the microcarriers were allowed to settle by gravity and the medium was removed as much as possible without removing any microcarriers. 250-300ml of TrypLE™ pre-warmed at 37 °C was added to the flask and placed on a spinner platform in the incubator at 37 °C. After 30 minutes, the agitation was stopped and a 25 ml sample was taken from the flask and filtered through a 40 μm filter. The microcarrier retained on the filter was discarded and a cell count was performed on the sample by directly running the sample on the CEDEX. Based on the TrypLE™ volume added and the cell count obtained, the total cells in the vessel were calculated and the cell density (cells/cm$^2$) was calculated.

**Composition of the Cell Culture Media**

DMEM (with 1g/L glucose, 4mM L-Glutamine and 3.7g/L Sodium Bicarbonate and without Sodium Pyruvate and Phenol Red) was the basal medium used for Condition 1 (the control).

Several other basal media were also tested to identify some components, which are critical in eliminating medium exchange, and thereby reduce serum consumption. One of the components used in the formulations is bovine serum albumin which was provided in the form of the commercially available AlbuMAX® I (Gibco™ Cell Culture, Invitrogen Corporation, Carlsbad, CA), which is lipid-rich bovine serum albumin. Table 1-2 provides the formulation of the basal media (M1-M7). Both the basal media and the feed media contained the commercially available stabilizer of cell membranes and anti-foaming agent Pluronic® F68.

**Table 1-2 Formulation of Basal Media**

| | Basal Medium | | | | | | |
|---|---|---|---|---|---|---|---|
| | M1 (g/L) | M2 (g/L) | M3 (g/L) | M4 (g/L) | M5 (g/L) | M6 (g/L) | M7 (g/L) |
| **Inorganic Salts** | | | | | | | |
| Calcium Chloride, Anhydrous | 0.2 | 0.2 | 0.2 | 0.2 | 0.2 | 0.2 | 0.2 |
| Calcium Chloride ($CaCl_2.2H_2O$) | 0 | 0 | 0 | 0 | 0 | 0 | 0 |
| potassium chloride, USP | 0.4 | 0.4 | 0.4 | 0.4 | 0.4 | 0.4 | 0.4 |
| Mg sulfate, anhydrous | 0.9767 | 0.9767 | 0.9767 | 0.9767 | 0.9767 | 0.9767 | 0.9767 |
| sodium chloride, USP | 6.4 | 6.4 | 6.4 | 6.4 | 6.4 | 6.4 | 6.4 |
| sodium phosphate, monobasic, $H_2O$, USP | 0.133175 | 0.125 | 0.133175 | 0.133175 | 0.133175 | 0.125 | 0.125 |
| sodium phosphate, dibasic heptahydrate ($Na_2HPO_4.7H_2O$) | 0.00201 | 0 | 0.00201 | 0.00201 | 0.00201 | 0 | 0 |
| **Trace Minerals** | | | | | | | |
| Ferric Nitrate (9 $H_2O$) | 0.0001 | 0.0001 | 0.0001 | 0.0001 | 0.0001 | 0.0001 | 0.0001 |
| Copper(II)sulfate pentahydrate ($CuSO_4.5H_2O$) | 9.33E-08 | 0 | 9.33E-08 | 9.33E-08 | 9.33E-08 | 0 | 0 |
| Zinc sulfate, heptahydrate, ($ZnSO_4.7H_2O$) | 3.24E-05 | 0 | 3.24E-05 | 3.24E-05 | 3.24E-05 | 0 | 0 |
| Sodium Selenate ($Na_2SeO_3$) | 0.000067 | 0.000067 | 0 | 0.000067 | 0.000067 | 0.000067 | 0 |
| **Amino Acids** | | | | | | | |
| L-Arginine, HCl | 0.09705 | 0.084 | 0.09705 | 0.09705 | 0.09705 | 0.084 | 0.084 |
| L-Cystine, 2HCl | 0.06779 | 0.06257 | 0.06779 | 0.06779 | 0.06779 | 0.06257 | 0.06257 |
| L-Cysteine HCl $H_2O$ | 0.009224 | 0 | 0.009224 | 0.009224 | 0.009224 | 0 | 0 |
| L-Glutamine | 0.584 | 0.584 | 0.584 | 0.584 | 0.584 | 0.584 | 0.584 |
| Glycine | 0.031125 | 0.03 | 0.031125 | 0.031125 | 0.031125 | 0.03 | 0.03 |
| L-Histidine, HCl, $H_2O$ | 0.048288 | 0.042 | 0.048288 | 0.048288 | 0.048288 | 0.042 | 0.042 |
| L-Isoleucine | 0.163713 | 0.1048 | 0.163713 | 0.163713 | 0.163713 | 0.1048 | 0.1048 |
| L-Leucine | 0.163713 | 0.1048 | 0.163713 | 0.163713 | 0.163713 | 0.1048 | 0.1048 |
| L-Lysine, HCl | 0.16807 | 0.1462 | 0.16807 | 0.16807 | 0.16807 | 0.1462 | 0.1462 |
| L- Methionine | 0.036748 | 0.03 | 0.036748 | 0.036748 | 0.036748 | 0.03 | 0.03 |
| L-Phenylalanine | 0.073695 | 0.066 | 0.073695 | 0.073695 | 0.073695 | 0.066 | 0.066 |
| L-serine | 0.05145 | 0.042 | 0.05145 | 0.05145 | 0.05145 | 0.042 | 0.042 |
| L-Threonine | 0.108609 | 0.0952 | 0.108609 | 0.108609 | 0.108609 | 0.0952 | 0.0952 |
| L-tryptophan | 0.018457 | 0.016 | 0.018457 | 0.018457 | 0.018457 | 0.016 | 0.016 |
| L-tyrosine, 2Na, 2 $H_2O$ | 0.121813 | 0.10379 | 0.121813 | 0.121813 | 0.121813 | 0.10379 | 0.10379 |

(continued)

| Amino Acids | | | | | | | |
|---|---|---|---|---|---|---|---|
| L-Valine | 0.111105 | 0.0936 | 0.111105 | 0.111105 | 0.111105 | 0.0936 | 0.0936 |
| L-Alanine | 0.000668 | 0 | 0.000668 | 0.000668 | 0.000668 | 0 | 0 |
| L-Aspargine $H_2O$ | 0.031978 | 0 | 0.031978 | 0.031978 | 0.031978 | 0 | 0 |
| L-Aspartic Acid | 0.00803 | 0 | 0.00803 | 0.00803 | 0.00803 | 0 | 0 |
| L-Glutamic Acid | 0.054728 | 0 | 0.054728 | 0.054728 | 0.054728 | 0 | 0 |
| L- Proline | 0.02403 | 0 | 0.02403 | 0.02403 | 0.02403 | 0 | 0 |
| L- Taurine | 0.000844 | 0 | 0.000844 | 0.000844 | 0.000844 | 0 | 0 |
| **Vitamins** | | | | | | | |
| D-Calcium Pantothenate | 0.004338 | 0.004 | 0.004338 | 0.004338 | 0.004338 | 0.004 | 0.004 |
| Choline Chloride | 0.006094 | 0.004 | 0.006094 | 0.006094 | 0.006094 | 0.004 | 0.004 |
| Folic Acid | 0.004302 | 0.004 | 0.004302 | 0.004302 | 0.004302 | 0.004 | 0.004 |
| I-Inositol | 0.009568 | 0.007 | 0.009568 | 0.009568 | 0.009568 | 0.007 | 0.007 |
| Niacinamide | 0.004302 | 0.004 | 0.004302 | 0.004302 | 0.004302 | 0.004 | 0.004 |
| Pyridoxal, HCl | 0.004153 | 0.004 | 0.004153 | 0.004153 | 0.004153 | 0.004 | 0.004 |
| Riboflavin | 0.000431 | 0.0004 | 0.000431 | 0.000431 | 0.000431 | 0.0004 | 0.0004 |
| thiamine, HCl | 0.004304 | 0.004 | 0.004304 | 0.004304 | 0.004304 | 0.004 | 0.004 |
| d-Biotin | 3.75E-05 | 0 | 3.75E-05 | 3.75E-05 | 3.75E-05 | 0 | 0 |
| Pvridoxine. HCl | 1.85E-05 | 0 | 1.85E-05 | 1.85E-05 | 1.85E-05 | 0 | 0 |
| Vitamin $B_{12}$ (cyanocobalamin) | 0.000102 | 0 | 0.000102 | 0.000102 | 0.000102 | 0 | 0 |
| **Lipids** | | | | | | | |
| Lipoic Acid/Thioctic Acid | 0.000015 | 0 | 0.000015 | 0.000015 | 0.000015 | 0 | 0 |
| Ethanolamine HCl | 0.02 | 0.02 | 0 | 0.02 | 0.02 | 0.02 | 0 |
| **Proteins** | | | | | | | |
| Insulin | 0.01 | 0.01 | 0 | 0.01 | 0.01 | 0.01 | 0 |
| Transferrin | 0.055 | 0.055 | 0 | 0.055 | 0.055 | 0.055 | 0 |
| **Energy Substrates** | | | | | | | |
| D-Glucose | 1 | 1 | 1 | 1 | 1 | 1 | 1 |
| Sodium Pyruvate | 0.11 | 0.11 | 0.11 | 0.11 | 0.11 | 0.11 | 0.11 |
| **Nucleic Arid Derivatives** | | | | | | | |
| Thymidine | 0.00045 | 0.00045 | 0.00045 | 0 | 0.00045 | 0 | 0 |
| Adenosine | 0.015 | 0.015 | 0.015 | 0 | 0.015 | 0 | 0 |
| Cytidine | 0.015 | 0.015 | 0.015 | 0 | 0.015 | 0 | 0 |
| Uridine | 0.015 | 0.015 | 0.015 | 0 | 0.015 | 0 | 0 |
| Guanosine | 0.015 | 0.015 | 0.015 | 0 | 0.015 | 0 | 0 |
| **Others** | | | | | | | |
| Putrescine.$2H_2O$ | 3.02E-05 | 0 | 3.02E-05 | 3.02E-05 | 3.02E-05 | 0 | 0 |

(continued)

| Others | | | | | | | |
|---|---|---|---|---|---|---|---|
| Pluronic® F68 | 0.015 | 0 | 0.015 | 0.015 | 0.015 | 0 | 0 |
| Bovine Serum Albumin (AlbuMAX® I) | 2.5 | 2.5 | 2.5 | 2.5 | 0 | 2.5 | 2.5 |

As outlined above, on Day 3, an 80% medium exchange was carried out in Condition 1. For the remaining conditions, a feed was added to eliminate the medium exchange. The different feed formulations are shown in Table 1-3. The amounts shown Table 1-3 presents the increase in weight (g) of the component per liter (L) of the culture volume when the feed is added.

**Table 1-3: Feed Formulations**

| | Feed Formulations | | | | | | |
|---|---|---|---|---|---|---|---|
| | F1 (g/L) | F2 (g/L) | F3 (g/L) | F4 (g/L) | F5 (g/L) | F6 (g/L) | F7 (g/L) |
| **Inorganic Salts** | | | | | | | |
| sodium phosphate, monobasic, $H_2O$, USP | 0.008175 | 0 | 0.008175 | 0.008175 | 0.008175 | 0 | 0 |
| sodium phosphate, dibasic heptahydrate ($Na_2HPO_4.7H_2O$) | 0.00201 | 0 | 0.00201 | 0.00201 | 0.00201 | 0 | 0 |
| **Trace Minerals** | | | | | | | |
| Copper(II)sulfate pentahydrate ($CuSO_4.5H_2O$) | 9.33E-08 | 0 | 9.33E-08 | 9.33E-08 | 9.33E-08 | 0 | 0 |
| Zinc sulfate, heptahydrate, ($ZnSO_4.7H_2O$) | 3.24E-05 | 0 | 3.24E-05 | 3.24E-05 | 3.24E-05 | 0 | 0 |
| Sodium Selenate ($Na_2SeO_3$) | 0.000067 | 0.000067 | 0 | 0.000067 | 0.000067 | 0.000067 | 0 |
| **Amino Acids** | | | | | | | |
| L-Arginine, HCl | 0.01305 | 0 | 0.01305 | 0.01305 | 0.01305 | 0 | 0 |
| L-Cystine, 2HCl | 0.00522 | 0 | 0.00522 | 0.00522 | 0.00522 | 0 | 0 |
| L-Cvsteine HCl $H_2O$ | 0.009224 | 0 | 0.009224 | 0.009224 | 0.009224 | 0 | 0 |
| Glycine | 0.001125 | 0 | 0.001125 | 0.001125 | 0.001125 | 0 | 0 |
| L-Histidine, HCl, $H_2O$ | 0.006288 | 0 | 0.006288 | 0.006288 | 0.006288 | 0 | 0 |
| L- Isoleucine | 0.058913 | 0 | 0.058913 | 0.058913 | 0.058913 | 0 | 0 |
| L- Leucine | 0.058913 | 0 | 0.058913 | 0.058913 | 0.058913 | 0 | 0 |
| L-Lysine, HCl | 0.02187 | 0 | 0.02187 | 0.02187 | 0.02187 | 0 | 0 |
| L- Methionine | 0.006748 | 0 | 0.006748 | 0.006748 | 0.006748 | 0 | 0 |
| L-Phenylalanine | 0.007695 | 0 | 0.007695 | 0.007695 | 0.007695 | 0 | 0 |
| L-serine | 0.00945 | 0 | 0.00945 | 0.00945 | 0.00945 | 0 | 0 |
| L- Threonine | 0.013409 | 0 | 0.013409 | 0.013409 | 0.013409 | 0 | 0 |
| L-tryptophan | 0.002457 | 0 | 0.002457 | 0.002457 | 0.002457 | 0 | 0 |
| L-tyrosine, 2Na, 2 $H_2O$ | 0.018023 | 0 | 0.018023 | 0.018023 | 0.018023 | 0 | 0 |

(continued)

| Amino Acids | | | | | | | |
|---|---|---|---|---|---|---|---|
| L-Valine | 0.017505 | 0 | 0.017505 | 0.017505 | 0.017505 | 0 | 0 |
| L-Alanine | 0.000668 | 0 | 0.000668 | 0.000668 | 0.000668 | 0 | 0 |
| L-Aspargine $H_2O$ | 0.031978 | 0 | 0.031978 | 0.031978 | 0.031978 | 0 | 0 |
| L-Aspartic Acid | 0.00803 | 0 | 0.00803 | 0.00803 | 0.00803 | 0 | 0 |
| L-Glutamic Acid | 0.054728 | 0 | 0.054728 | 0.054728 | 0.054728 | 0 | 0 |
| L-Proline | 0.02403 | 0 | 0.02403 | 0.02403 | 0.02403 | 0 | 0 |
| L- Taurine | 0.000844 | 0 | 0.000844 | 0.000844 | 0.000844 | 0 | 0 |
| **Vitamins** | | | | | | | |
| D-Calcium Pantothenate | 0.000338 | 0 | 0.000338 | 0.000338 | 0.000338 | 0 | 0 |
| Choline Chloride | 0.002094 | 0 | 0.002094 | 0.002094 | 0.002094 | 0 | 0 |
| Folic Acid | 0.000302 | 0 | 0.000302 | 0.000302 | 0.000302 | 0 | 0 |
| I-Inositol | 0.002568 | 0 | 0.002568 | 0.002568 | 0.002568 | 0 | 0 |
| Niacinamide | 0.000302 | 0 | 0.000302 | 0.000302 | 0.000302 | 0 | 0 |
| Pvridoxal, HCl | 0.000153 | 0 | 0.000153 | 0.000153 | 0.000153 | 0 | 0 |
| Riboflavin | 3.11E-05 | 0 | 3.11E-05 | 3.11E-05 | 3.11E-05 | 0 | 0 |
| thiamine, HCl | 0.000304 | 0 | 0.000304 | 0.000304 | 0.000304 | 0 | 0 |
| d-Biotin | 3.75E-05 | 0 | 3.75E-05 | 3.75E-05 | 3.75E-05 | 0 | 0 |
| Pvridoxine. HCl | 1.85E-05 | 0 | 1.85E-05 | 1.85E-05 | 1.85E-05 | 0 | 0 |
| Vitamin $B_{12}$ (cyanocobalamin) | 0.000102 | 0 | 0.000102 | 0.000102 | 0.000102 | 0 | 0 |
| **Lipids** | | | | | | | |
| Lipoic Acid/Thioctic Acid | 0.000015 | 0 | 0.000015 | 0.000015 | 0.000015 | 0 | 0 |
| Ethanolamine HCl | 0.02 | 0.02 | 0 | 0.02 | 0.02 | 0.02 | 0.02 |
| **Proteins** | | | | | | | |
| Insulin | 0.01 | 0.01 | 0 | 0.01 | 0.01 | 0.01 | 0.01 |
| Transferrin | 0.055 | 0.055 | 0 | 0.055 | 0.055 | 0.055 | 0.055 |
| **Nucleic Acid Derivatives** | | | | | | | |
| Thymidine | 0.00045 | 0.00045 | 0.00045 | 0 | 0.00045 | 0 | 0 |
| Adenosine | 0.015 | 0.015 | 0.015 | 0 | 0.015 | 0 | 0 |
| Cytidine | 0.015 | 0.015 | 0.015 | 0 | 0.015 | 0 | 0 |
| Uridine | 0.015 | 0.015 | 0.015 | 0 | 0.015 | 0 | 0 |
| Guanosine | 0.015 | 0.015 | 0.015 | 0 | 0.015 | 0 | 0 |
| **Others** | | | | | | | |
| Putrescine.$2H_2O$ | 3.02E-05 | 0 | 3.02E-05 | 3.02E-05 | 3.02E-05 | 0 | 0 |
| Pluronic® F68 | 0.015 | 0 | 0.015 | 0.015 | 0.015 | 0 | 0 |
| Bovine Serum Albumin (AlbuMAX® I) | 2.5 | 2.5 | 2.5 | 2.5 | 0 | 2.5 | 2.5 |

The media used in Conditions 1 to 9 all had FBS added to them. The components of FBS are shown below:

| Table 1-4: Profile of Fetal Bovine Sera (see Price et al., In Vitro 18:576-584 (1982)) | | | |
|---|---|---|---|
| Description | Average | Range | N |
| Endotoxin | 0.356 ng/ml | 0.008-10.0 | 39 |
| pH | 7.4* | 7.20-7.60 | 40 |
| **Inorganic Salts** | | | |
| Calcium ($Ca^{2+}$) | 13.6/100 ml | 12.6-14.3 | 43 |
| Chloride ($Cl^-$) | 103 meq/L | 98-108 | 43 |
| Inorganic Phosphorous | 9.8 mg/100 ml | 4.3-11.4 | 43 |
| Potassium ($K^+$) | 11.2 meq/L | 10.0-14.0 | 43 |
| Selenium | 0.026 $\mu$g/ml | 0.014-0.038 | 25 |
| Sodium ($Na^+$) | 137 meq/L | 125-143 | 43 |
| **Other components** | | | |
| Alkaline Phosphatase | 255 mU/ml | 111-352 | 43 |
| Blood Urea Nitrogen | 16 mg/100 ml | 14-20 | 43 |
| Creatine | 3.1 mg/100 ml | 1.6-4.3 | 43 |
| Direct Bilirubin | 0.2 mg/100 ml | 0.0-0.5 | 43 |
| Glucose | 125 mg/100 ml | 85-247 | 43 |
| Hemoglobin | 11.3 mg/100 ml | 2.4-18.1 | 17 |
| Lactate Dehydrogenase | 864 mU/ml | 260-1,215 | 43 |
| Serum Glutamate Oxalacetate Transaminase | 130 mU/ml | 20-201 | 43 |
| Total Bilirubin | 0.4 mg/100 ml | 0.3-1.1 | 43 |
| Uric Acid | 2.9 mg/100 ml | 1.3-4.1 | 43 |
| **Steroids and Hormones** | | | |
| Cholesterol | 31 mg/100 ml | 12-63 | 43 |
| Cortisol | 0.5 $\mu$g/ml | <0.1-2.3 | 43 |
| Follicle Stimulating Hormone | 9.5 ng/ml | <2-33.8 | 34 |
| Growth Hormone | 39.0 ng/ml | 18.7-51.6 | 40 |
| Leutinizing Hormone | 0.79 ng/ml | 0.12-1.8 | 38 |
| Parathyroid Hormone | 1,718 pg/ml | 85-6,180 | 41 |
| Progesterone | 8 ng/100 ml | <0.3-36 | 42 |
| Prolactin | 17.6 ng/ml | 2.00-49.55 | 40 |
| Prostaglandin E | 5.91 ng/ml | 0.5-30.48 | 37 |
| Prostaglandin F | 12.33 ng/ml | 3.77-42.00 | 38 |
| T3 | 119 ng/100 ml | 56-233 | 41 |
| T4 | 12.1 ng/100 ml | 7.8-15.6 | 42 |
| Testosterone | 40 ng/100 ml | 21-99 | 42 |
| Thyroid Stimulating Hormone | 1.22 ng/ml | <0.2-4.5 | 40 |
| **Protein** | | | |
| Total Protein | 3.8 g/100 ml | 3.2-7.0 | 43 |

(continued)

| Protein | | | |
|---|---|---|---|
| Albumin | 2.3 g/100 ml | 2.0-3.6 | 43 |
| Insulin | 10 mU/ml | 6-14 | 40 |

**Results**

The cell counts from the different conditions are shown in Table 1-5 below. The results can be analyzed in two parts. By comparing Conditions 1 and 2, it is evident that the combination of M5 medium and F5 feed on Day3 eliminates medium exchange. This is important, as this will drastically reduce the serum concentration by over 44%. By comparing conditions 3 through 9, it is evident that the following three conditions have the lowest performance in comparison to others: Condition 6, M4 basal medium + 15% FBS with F4 feed on Day 3; Condition 8, M6 basal medium + 15% FBS with F6 feed on Day 3; and Condition 9, M7 basal medium + 15% FBS with F7 feed on Day 3. A closer look at the formulations indicates that none of these three conditions have nucleic acid derivatives in the media or feed. Hence, it can also be inferred that nucleic acid derivatives are critical for hUTC growth. This example demonstrates that hUTC can be grown for 6 days without medium exchange by enriching the culture medium and supplementing additional medium components on Day 3 and nucleic acid derivatives are critical in order to maintain comparable cell growth.

| Table 1-5: Viable Cells counts, cells/cm$^2$ for hUTC cultures grown under Conditions 1-9 | | | | | | | | | |
|---|---|---|---|---|---|---|---|---|---|
| | Condition | | | | | | | | |
| Day | 1 | 2 | 3 | 4 | 5 | 6 | 7 | 8 | 9 |
| 0 | 5690 | 6061 | 7030 | 7030 | 7030 | 7030 | 7030 | 7030 | 7030 |
| 3 | 11942 | - | 28747 | 32633 | 21431 | 21810 | 19506 | 21320 | 25037 |
| 4 | - | 20035 | 31976 | 38891 | 25825 | 21256 | 20134 | 17146 | 17569 |
| 5 | 32035 | 34329 | 40470 | 49787 | 46249 | 32827 | 36203 | 26921 | 27699 |
| 6 | 49757 | 53418 | 69408 | 71271 | 64602 | 38443 | 65313 | 38982 | 39718 |
| Condition 1: DMEM + 15% FBS and 80% medium exchange on Day 3 | | | | | | | | | |
| Condition 2: M5 + 15% FBS and addition of F5 on Day 3 | | | | | | | | | |
| Condition 3: M1 + 15% FBS and addition of F1 on Day 3 | | | | | | | | | |
| Condition 4: M2 + 15% FBS and addition of F2 on Day 3 | | | | | | | | | |
| Condition 5: M3 + 15% FBS and addition of F3 on Day 3 | | | | | | | | | |
| Condition 6: M4 + 15% FBS and addition of F4 on Day 3 | | | | | | | | | |
| Condition 7: M5 + 15% FBS and addition of F5 on Day 3 | | | | | | | | | |
| Condition 8: M6 + 15% FBS and addition of F6 on Day 3 | | | | | | | | | |
| Condition 9: M7 + 15% FBS and addition of F7 on Day 3 | | | | | | | | | |

**EXAMPLE 2**

**Growth and Serial passaging of HUTCs on Microcarriers and in Spinner Flasks with Enriched Media**

Serial passaging of Human umbilical tissue cells (hUTCs) attached on microcarriers in suspension culture in spinner flasks requires medium exchange if cells do not reach a predetermined optimal cell density of >20,000 cells/cm$^2$ on day 3. This medium exchange adds additional manipulations to the process and makes it difficult to predict cell passaging schedule. Therefore, this procedure to passage cells is not commercially desirable. hUTC can be grown to high cell density by exchanging cell growth medium containing 15% Fetal Bovine Serum. This example investigated an alternate more commercially desirable method for passaging hUTC. hUTC attached on microcarrier were grown to high cell density in suspension culture in spinner flasks by enriching the growth medium with serum-free nutrients. This method eliminates medium exchange on day 3 to consistently grow cells >20,000 cells/cm$^2$. This method improves

process robustness for passaging of hUTCs.

The following were used in this study: DMEM (with 2g/L glucose, 4mM L-Glutamine and 3.7g/L Sodium Bicarbonate and without Sodium Pyruvate and Phenol Red); Fetal Bovine Serum (15% v/v); M5 Basal medium (see Example 1 above); F5 feed (see Example 1 above); and Hillex® Ultra (Solohill Engineering, Ann Arbor, MI) microcarriers were used.

**Surface Marker Analysis method:**

3% FBS in Dulbecco's Phosphate Buffer Saline (DPBS) without Ca or Mg was used as the staining buffer. Six antibodies were used to identify the surface markers and two antibodies were used as controls. The list of antibodies and their dilutions in staining buffer for the assay are shown in Table 2-1 below.

| Table 2-1 List of Antibodies and Dilutions | | | | | |
|---|---|---|---|---|---|
| Antibody | Manufacturer | Catalog No. | Manufacturer's concentration | Dilution in Staining Buffer | Comments |
| IgG1 | BD Biosciences | 555749 | 1 μg/20 μL | 1:10 | Control for CD13, CD90, HLA-ABC, CD34 |
| | | | | 1:2 | Control for CD117 |
| IgG2 | BD Biosciences | 555574 | 1 μg/20 μL | 1:8 | Control for HLA-DR |
| CD13 | BD Biosciences | 555394 | 2 μg/20 μL | 1:20 | |
| CD34 | BD Biosciences | 555822 | 0.06 μg/20 μL | none | |
| CD90 | BD Biosciences | 555596 | 1 μg/20 μL | 1:10 | |
| CD117 | BD Biosciences | 340529 | 10 μg/ml | none | |
| HLA-ABC | BD Biosciences | 555553 | 0.03 μg/20 μL | none | |
| HLA-DR | BD Biosciences | 555812 | 0.125 μg /20 μL | none | |

Methodology: A cell count was performed on the sample and $2.5 \times 10^6$ cells were resuspended in 10 ml of DMEM + 15% FBS medium. The cells were then centrifuged, the supernatant removed and resuspended in staining buffer to obtain a cell concentration of $1 \times 10^6$ cells/ml. This cell suspension was then distributed into different tubes such that each tube receives 20000 cells. Appropriate amounts of diluted antibody and staining buffer were then added as shown in Table 2-1 above. The samples were then incubated for 30 minutes at 2-8 °C. After incubation, 3 ml of DPBS was added and the samples were centrifuged. The supernatant was removed and the cell pallet was re-suspended in 500 μl of DPBS. The samples were then run on Guava® PCA™ instrument, Millipore, MD, USA. Of the six surface markers, three are positive markers (CD13, CD90, HLA-ABC) and three are negative markers (CD34, CD117, HLA-DR).

**Culture Conditions**

The methods of culturing and serial passaging of hUTCs were explained in Example 1. As mentioned, this serial passaging used DMEM with 15% FBS as the culture medium and frequently required medium exchange to meet the passaging criterion. In this example, it was attempted to eliminate the need for frequent medium exchange during serial passaging. This was achieved by using modified M5 medium (with 2g/L of glucose instead of 1g/L) and 15% FBS (v/v). With the use of this modified M5 medium with 15% FBS, the day of passage was fixed and no medium exchange was performed over six passages. The culture conditions and criteria are presented in Table 2-2 below.

| Table 2-2: Culture conditions | |
|---|---|
| Seeding Density of New Culture Vessels | Target (Range) |
| At Thaw: 125 ml spinner flask(viable cells/cm²) | 6000 (3000-7500) |
| At Passage 1: from 125 ml to 500 ml spinner flask (viable cells/cm²) | 5000 (3200-7000) |
| At all other passages: 500 ml spinner flasks (viable cells/cm²) | 7000 (6000-8000) |

(continued)

| Duration of Passages | Target |
|---|---|
| After Thaw: 125 ml spinner flask (days) | 4 |
| At all other passages: 500 ml spinner flasks (days) | 3 |
| Spinner Flask Parameters | Target (Range) |
| 125 ml flask working volume (ml) | 100 (85-115) |
| 500 ml flask working volume (ml) | 500 (450-500) |
| Incubator Parameters | Target |
| Temperature (°C) | 37.0 |
| $CO_2$(%) | 10 |
| Spinner Plate Parameters | Target (Range) |
| Agitation in 125 ml flask (rpm) | 60 (55-65) |
| Agitation in 500 flask (rpm) | 40 (35-45) |
| Microcarrier Density | Target (Range) |
| Density of microcarrier in medium | 12 (11.0-13.0) |

The cells had a robust growth over six passages as shown in Table 2-3 below. In a process with only DMEM + 15% FBS the cells frequently required a medium exchange on the day before the passage in order to achieve a population doubling of 1.5, thereby extending the duration of passage by an extra day. With the enriched medium, the cells had at least over 1.5 population doublings consistently within 3 days (4 days from vial thaw) and did not require any medium exchange. The cells retained their identity after seven passages in this medium and then growing these cells for 6 days in this medium with F5 feed on day 3 of the 6-day culture. The surface markers analysis of these cells is shown in Table 2-4 below.

| Table 2-3: Results | | | |
|---|---|---|---|
| Passage # | Day # | Viable cell density, cells/cm$^2$ | Population doublings within passage |
| 0 (After Vial Thaw) | 4 | 31890 | 2.5 |
| 1 | 3 | 34311 | 2.5 |
| 2 | 3 | 40464 | 2.5 |
| 3 | 3 | 19398 | 1.5 |
| 4 | 3 | 30816 | 2.2 |
| 5 | 3 | 22100 | 1.7 |
| 6 | 3 | 35607 | 2.3 |

| Table 2-4: Surface marker results of cells passaged in M5 medium + 15% FBS for 7 passages, followed by 6 day culture in M5 medium + 15% FBS and F5 feed on Day 3 of the 6 day culture | |
|---|---|
| Surface Marker ID | Result |
| CD13 | (+) |
| CD90 | (+) |
| HLA ABC | (+) |
| CD34 | (-) |
| CD117 | (-) |

(continued)

| Table 2-4: Surface marker results of cells passaged in M5 medium + 15% FBS for 7 passages, followed by 6 day culture in M5 medium + 15% FBS and F5 feed on Day 3 of the 6 day culture | |
|---|---|
| Surface Marker ID | Result |
| HLA DR | (-) |

## Example 3

### Growth of hUTCs on microcarriers in a reduced serum medium in spinner flasks

Human umbilical tissue cells (hUTCs) can be grown to high cell density by exchanging cell growth medium containing 15% FBS on day 3 of the run. High serum concentration in medium and medium exchange is not desirable from commercial perspective because of high serum cost, high serum usage for production, and additional operational manipulations. This example discloses a method to grow hUTCs attached on microcarriers in reduced serum growth medium and without medium exchange while achieving high cell density.

The culture media used in the example was M5 basal medium (*see above*), with 2g/L D-glucose instead of 1g/L D-Glucose, to which Fetal Bovine Serum was added. The F5 feed medium was used. Glucose was provided as an aqueous solution of 220 g/L of Dextrose Monohydrate. Glutamine was provided as a 200 mM solution. In this example, different growth conditions were studied. These conditions are outlined below. As used in these conditions, the day of inoculation is considered to be Day 0. Furthermore, Hillex® Ultra (Solohill Engineering, Ann Arbor, MI) microcarriers were used in each condition.

**Condition 1 (15% FBS):** A cell count was performed on the inoculum. The target seeding density of this condition was ~6000 viable cells/cm$^2$ at a microcarrier concentration of ~20 g/L. Based on the cell count of the inoculums the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M5 basal medium and 15% (v/v) of FBS was added to bring up the volume to 500 ml. The flask was placed on a spinner platform at 60 RPM in a 37 °C and 10% CO$_2$ incubator. On Day 3, feed F5 was added. No medium exchange was performed. Periodic cell counts were performed as explained in the cell counting section. 2.5 ml of glucose was added on Day 3 and Day 5. On Day 4, 5 ml of 200 mM L-Glutamine was added to the flask.

**Condition 2 (10% FBS):** A cell count was performed on the inoculum. The target seeding density of this condition was ~6000 viable cells/cm$^2$ at a microcarrier concentration of ~20 g/L. Based on the cell count of the inoculums the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M5 basal medium and 10% (v/v) of FBS was added to bring up the volume to 500 ml. The flask was placed on a spinner platform at 60 RPM in a 37 °C and 10% CO$_2$ incubator. On Day 3, feed F5 was added. No medium exchange was performed. Periodic cell counts were performed as explained in the cell counting section. 2.5 ml of glucose was added on Day 3 and Day 5. On Day 4, 5 ml of 200 mM L-Glutamine was added to the flask.

**Condition 3 (7.5% FBS):** A cell count was performed on the inoculum. The target seeding density of this condition was ~6000 viable cells/cm$^2$ at a microcarrier concentration of ~20 g/L. Based on the cell count of the inoculums the desired volume of inoculum was added to a new autoclaved 500 ml glass spinner flask. The microcarriers were allowed to settle by gravity. The medium from the inoculum was removed until ~ 100 ml was left in the flask. Fresh microcarriers were then added resulting in a final weight of ~10 g of microcarriers in the flask. Fresh medium comprised of M5 basal medium and 7.5% (v/v) of FBS was added to bring up the volume to 500 ml. The flask was placed on a spinner platform at 60 RPM in a 37 °C and 10% CO$_2$ incubator. On Day 3, feed F5 was added. No medium exchange was performed. Periodic cell counts were performed as explained in the cell counting section. 2.5 ml of glucose was added on Day 3 and Day 5. On Day 4, 5 ml of 200 mM L-Glutamine was added to the flask. Harvest: On day 6, both the flasks were harvested. For harvest, the microcarriers were allowed to settle by gravity and the medium was removed as much as possible without removing any microcarriers. 300ml of TrypLE™ pre warmed at 37 °C was added to the flask and placed on a spinner platform in the incubator at 37 °C. After 30 minutes, the agitation was stopped and a 25 ml sample was taken from the flask and filtered through a 40-μm filter. The microcarrier retained on the filter was discarded and a cell count was performed on the sample by directly running the sample on the CEDEX. Based on the TrypLE™ volume added and the cell count obtained, the total cells in the vessel were calculated and the cell density (cells/cm$^2$) was calculated.

**Results:** The cell counts from the three conditions with different concentration of FBS in each are shown in Table below. In addition to the results from this example the results from Example 1, Condition 1 (DMEM +15% FBS with Medium exchange on Day3) are included for comparison. Thus, the enriched media not only eliminate medium exchange but can also reduce the serum concentration in the medium, thereby further minimizing the amount of serum used in culture.

| Table 3-1 Cell counts, viable cells/cm$^2$ | | | | |
|---|---|---|---|---|
| Day | Condition 1 | Condition 2 | Condition 3 | Control |
| 0 | 5652 | 5652 | 5652 | 5690 |
| 4 | 30839 | 25182 | 26802 | - |
| 5 | 42358 | 34827 | 28889 | 32035 |
| 6 | 58252 | 59841 | 54369 | 49757 |
| Condition 1: M5 + 15% FBS & F5 on Day 3 | | | | |
| Condition 2: M5 + 10% FBS & F5 on Day 3 | | | | |
| Condition 3: M5 + 7.5% FBS & F5 on Day 3 | | | | |
| Control: 15% FBS with Medium Exchange on Day 3 (Data Shown in Example 1 above) | | | | |

## EXAMPLE 4

### Isolation of Cells

**Umbilical cell isolation.** Umbilical cords were obtained from National Disease Research Interchange (NDRI, Philadelphia, PA). The tissues were obtained following normal deliveries. The cell isolation protocols were performed aseptically in a laminar flow hood. To remove blood and debris, the cord was washed in phosphate buffered saline (PBS; Invitrogen, Carlsbad, CA) in the presence of penicillin at 100 U/ml, streptomycin at 100 mg/ml and amphotericin B at 0.25 $\mu$g/ml (Invitrogen Carlsbad, CA). The tissues were then mechanically dissociated in 150 cm$^2$ tissue culture plates in the presence of 50 ml of medium (DMEM-low glucose or DMEM-high glucose; Invitrogen) until the tissue was minced into a fine pulp. The chopped tissues were transferred to 50 ml conical tubes (approximately 5 g of tissue per tube).

The tissue was then digested in either DMEM-low glucose medium or DMEM-high glucose medium, each containing penicillin at 100 U/ml, streptomycin at 100 mg/ml, amphotericin B at 0.25 $\mu$g/ml and the digestion enzymes. In some experiments an enzyme mixture of collagenase and dispase was used ("C:D") (collagenase (Sigma, St Louis, MO), 500 U/ml; and dispase (Invitrogen), 50 U/ml, in DMEM-Low glucose medium). In other experiments a mixture of collagenase, dispase and hyaluronidase ("C:D:H") was used (C:D:H = collagenase, 500 U/ml; dispase, 50 U/ml; and hyaluronidase (Sigma), 5 U/ml, in DMEM-Low glucose). The conical tubes containing the tissue, medium and digestion enzymes were incubated at 37°C in an orbital shaker (Environ, Brooklyn, NY) at 225 rpm for 2 hours.

After digestion, the tissues were centrifuged at 150 x g for 5 minutes, the supernatant was aspirated. The pellet was resuspended in 20 ml of growth medium (DMEM:Low glucose (Invitrogen), 15% (v/v) fetal bovine serum (FBS; defined fetal bovine serum; Lot #AND18475; Hyclone, Logan, UT), 0.001% (v/v) 2-mercaptoethanol (Sigma), penicillin at 100 U/ml, streptomycin at 100 $\mu$g/ml, and amphotericin B at 0.25 $\mu$g/ml (each from Invitrogen, Carlsbad, CA)). The cell suspension was filtered through a 70 $\mu$m nylon BD FALCON Cell Strainer (BD Biosciences, San Jose, CA). An additional 5 ml rinse comprising growth medium was passed through the strainer. The cell suspension was then passed through a 40-$\mu$m nylon cell strainer (BD Biosciences, San Jose, CA) and chased with a rinse of an additional 5 ml of growth medium.

The filtrate was resuspended in growth medium (total volume 50 ml) and centrifuged at 150 x g for 5 minutes. The supernatant was aspirated and the cells were resuspended in 50 ml of fresh growth medium. This process was repeated twice more.

After the final centrifugation, supernatant was aspirated and the cell pellet was resuspended in 5 ml of fresh growth medium. The number of viable cells was determined using trypan blue staining. Cells were then cultured under standard conditions.

The cells isolated from umbilical cord tissues were seeded at 5,000 cells/cm$^2$ onto gelatin-coated T-75 flasks (Corning Inc., Corning, NY) in growth medium. After two days, spent medium and unadhered cells were aspirated from the flasks. Adherent cells were washed with PBS three times to remove debris and blood-derived cells. Cells were then replenished with growth medium and allowed to grow to confluence (about 10 days from passage 0 to passage 1). On subsequent passages (from passage 1 to 2 etc.), cells reached sub-confluence (75-85% confluence) in 4-5 days. For these subsequent passages, cells were seeded at 5,000 cells/cm$^2$. Cells were grown in a humidified incubator with 5% carbon dioxide at 37°C.

In some experiments, cells were isolated from postpartum tissues in DMEM-low glucose medium after digestion with LIBERASE (2.5 mg/ml, Blendzyme 3; Roche Applied Sciences, Indianapolis, IN) and hyaluronidase (5 U/ml, Sigma). Digestion of the tissue and isolation of the cells was as described for other protease digestions above, however, the LIBERASE/hyaluronidase mixture was used instead of the C:D or C:D:H enzyme mixture. Tissue digestion with LIBERASE resulted in the isolation of cell populations from postpartum tissues that expanded readily.

Procedures were compared for isolating cells from the umbilical cord using differing enzyme combinations. Enzymes compared for digestion included: i) collagenase; ii) dispase; iii) hyaluronidase; iv) collagenase : dispase mixture (C:D); v) collagenase:hyaluronidase mixture (C:H); vi) dispase:hyaluronidase mixture (D:H); and vii) collagenase:dispase:hyaluronidase mixture (C:D:H). Differences in cell isolation utilizing these different enzyme digestion conditions were observed (*see* Table 4-1).

Other attempts were made to isolate pools of cells from umbilical cord by different approaches. In one instance, umbilical cord was sliced and washed with growth medium to dislodge the blood clots and gelatinous material. The mixture of blood, gelatinous material and growth medium was collected and centrifuged at 150 x g. The pellet was resuspended and seeded onto gelatin coated flasks in growth medium. From these experiments a cell population was isolated that readily expanded.

Cells have also been isolated from cord blood samples obtained from NDRI. The isolation protocol used was that of International Patent Application PCT/US2002/029971 by Ho et al. Samples (50 ml and 10.5 ml, respectively) of umbilical cord blood (NDRI, Philadelphia PA) were mixed with lysis buffer (filter-sterilized 155 mM ammonium chloride, 10 millimolar potassium bicarbonate, 0.1 mM EDTA buffered to pH 7.2 (all components from Sigma, St. Louis, MO)). Cells were lysed at a ratio of 1:20 cord blood to lysis buffer. The resulting cell suspension was vortexed for 5 seconds, and incubated for 2 minutes at ambient temperature. The lysate was centrifuged (10 minutes at 200 x g). The cell pellet was resuspended in Complete Minimal Essential Medium (Gibco, Carlsbad CA) containing 10% fetal bovine serum (Hyclone, Logan UT), 4 mM glutamine (Mediatech Herndon, VA), penicillin at 100 U/ml and streptomycin at 100 μg/ml (Gibco, Carlsbad, CA). The resuspended cells were centrifuged (10 minutes at 200 x g), the supernatant was aspirated, and the cell pellet was washed in complete medium. Cells were seeded directly into either T75 flasks (Corning, NY), T75 laminin-coated flasks, or T175 fibronectin-coated flasks (both Becton Dickinson, Bedford, MA).

To determine whether cell populations could be isolated under different conditions and expanded under a variety of conditions immediately after isolation, cells were digested in growth medium with or without 0.001% (v/v) 2-mercaptoethanol (Sigma, St. Louis, MO), using the enzyme combination of C:D:H, according to the procedures provided above. All cells were grown in the presence of penicillin at 100 U/ml and streptomycin at 100 μg/ml. Under all tested conditions cells attached and expanded well between passage 0 and 1 (Table 4-2). Cells in conditions 5-8 and 13-16 were demonstrated to proliferate well up to 4 passages after seeding, at which point they were cryopreserved.

The combination of C:D:H, provided the best cell yield following isolation, and generated cells that expanded for many more generations in culture than the other conditions (Table 4-1). An expandable cell population was not attained using collagenase or hyaluronidase alone. No attempt was made to determine if this result is specific to the collagenase that was tested.

| Table 4-1: Isolation of cells from umbilical cord tissue using varying enzyme combinations | | |
|---|---|---|
| **Enzyme Digest** | **Cells Isolated** | **Cell Expansion** |
| Collagenase | X | X |
| Dispase | + (>10 h) | + |
| Hyaluronidase | X | X |
| Collagenase:Dispase | ++ (< 3 h) | ++ |
| Collagenase:Hyaluronidase | ++ (< 3 h) | + |
| Dispase:Hyaluronidase | + (>10h) | + |
| Collagenase:Dispase:Hyaluronidase | +++ (< 3 h) | +++ |
| Key: + = good, ++ = very good, +++ = excellent, X = no success | | |

Cells attached and expanded well between passage 0 and 1 under all conditions tested for enzyme digestion and growth (Table 4-2). Cells in experimental conditions 5-8 and 13-16 proliferated well up to four passages after seeding, at which point they were cryopreserved. All cells were cryopreserved for further analysis.

| Table 4-2: Isolation and culture expansion of postpartum cells under varying conditions | | | | | | |
|---|---|---|---|---|---|---|
| Condition | Medium | 15% FBS | BME | Gelatin | 20% $O_2$ | Growth Factors |
| 1 | DMEM-Lg; | Y | Y | Y | Y | N |
| 2 | DMEM-Lg; | Y | Y | Y | N (5%) | N |
| 3 | DMEM-Lg; | Y | Y | N | Y | N |
| 4 | DMEM-Lg; | Y | Y | N | N (5%) | N |
| 5 | DMEM-Lg; | N (2%) | Y | N (Laminin) | Y | EGF/FGF (20 ng/ml) |
| 6 | DMEM-Lg; | N (2%) | Y | N (Laminin) | N (5%) | EGF/FGF (20 ng/ml) |
| 7 | DMEM-Lg; | N (2%) | Y | N (Fibronectin) | Y | PDGF/VEGF |
| 8 | DMEM-Lg; | N (2%) | Y | N (Fibronectin) | N (5%) | PDGF/VEGF |
| 9 | DMEM-Lg; | Y | N | Y | Y | N |
| 10 | DMEM-Lg; | Y | N | Y | N (5%) | N |
| 11 | DMEM-Lg; | Y | N | N | Y | N |
| 12 | DMEM-Lg; | Y | N | N | N (5%) | N |
| 13 | DMEM-Lg; | N (2%) | N | N (Laminin) | Y | EGF/FGF (20 ng/ml) |
| 14 | DMEM-Lg; | N (2%) | N | N (Laminin) | N (5%) | EGF/FGF (20 ng/ml) |
| 15 | DMEM-Lg; | N (2%) | N | N (Fibronectin) | Y | PDGF/VEGF |
| 16 | DMEM-Lg; | N (2%) | N | N (Fibronectin) | N (5%) | PDGF/VEGF |

Nucleated cells attached and grew rapidly. These cells were analyzed by flow cytometry and were similar to cells obtained by enzyme digestion.

The preparations contained red blood cells and platelets. No nucleated cells attached and divided during the first 3 weeks. The medium was changed 3 weeks after seeding and no cells were observed to attach and grow.

Populations of cells could be isolated from umbilical tissue efficiently using the enzyme combination collagenase (a metalloprotease), dispase (neutral protease) and hyaluronidase (mucolytic enzyme which breaks down hyaluronic acid). LIBERASE, which is a blend of collagenase and a neutral protease, may also be used. Blendzyme 3, which is collagenase (4 Wunsch U/g) and thermolysin (1714 casein U/g), was also used together with hyaluronidase to isolate cells. These cells expanded readily over many passages when cultured in growth expansion medium on gelatin coated plastic.

Cells were also isolated from residual blood in the cords, but not cord blood. The presence of cells in blood clots washed from the tissue, which adhere and grow under the conditions used, may be due to cells being released during the dissection process.

## EXAMPLE 5

### Karyotype Analysis of Cells

Cell lines used in cell therapy are preferably homogeneous and free from any contaminating cell type. Human cells used in cell therapy should have a normal number (46) of chromosomes with normal structure. To identify umbilicus-derived cell lines that are homogeneous and free from cells of non-umbilical tissue origin, karyotypes of cell samples were analyzed.

UTC from postpartum tissue of a male neonate were cultured in growth media. Postpartum tissue from a male neonate (X,Y) was selected to allow distinction between neonatal-derived cells and maternal derived cells (X,X). Cells were seeded at 5,000 cells per square centimeter in growth medium in a T25 flask (Corning, Corning, NY) and expanded to 80% confluence. A T25 flask containing cells was filled to the neck with growth media. Samples were delivered to a clinical cytogenetics lab by courier (estimated lab to lab transport time is one hour). Chromosome analysis was performed by the Center for Human & Molecular Genetics at the New Jersey Medical School, Newark, NJ. Cells were analyzed during metaphase when the chromosomes are best visualized. Of twenty cells in metaphase counted, five were analyzed for normal homogeneous karyotype number (two). A cell sample was characterized as homogeneous if two karyotypes were observed. A cell sample was characterized as heterogeneous if more than two karyotypes were observed. Additional metaphase cells were counted and analyzed when a heterogeneous karyotype number (four) was identified.

All cell samples sent for chromosome analysis were interpreted by the cytogenetics laboratory staff as exhibiting a normal appearance. Three of the sixteen cell lines analyzed exhibited a heterogeneous phenotype (XX and XY) indicating the presence of cells derived from both neonatal and maternal origins (Table 5-1). Each of the cell samples was characterized as homogeneous. (Table 5-1).

| Table 5-1: Karyotype results of hUTC | | | | | |
|---|---|---|---|---|---|
| Tissue | Passage | Metaphase cells counted | Metaphase cells analyzed | Number of karyotypes | ISCN Karyotype |
| Umbilical | 23 | 20 | 5 | 2 | 46, XX |
| Umbilical | 6 | 20 | 5 | 2 | 46, XY |
| Umbilical | 3 | 20 | 5 | 2 | 46, XX |

Chromosome analysis identified umbilicus-derived UTC whose karyotypes appear normal as interpreted by a clinical cytogenetic laboratory. Karyotype analysis also identified cell lines free from maternal cells, as determined by homogeneous karyotype.

## EXAMPLE 6

### Flow Cytometric Evaluation of Cell Surface Markers

Characterization of cell surface proteins or "markers" by flow cytometry can be used to determine a cell line's identity. The consistency of expression can be determined from multiple donors, and in cells exposed to different processing and culturing conditions. Postpartum cell lines isolated from the umbilicus were characterized by flow cytometry, providing a profile for the identification of these cell lines.

Cells were cultured in growth medium, in plasma-treated T75, T150, and T225 tissue culture flasks (Corning, Corning, NY) until confluent. The growth surfaces of the flasks were coated with gelatin by incubating 2% (w/v) gelatin (Sigma, St. Louis, MO) for 20 minutes at room temperature.

Adherent cells in flasks were washed in phosphate buffered saline (PBS); (Gibco, Carlsbad, MO) and detached with trypsin/EDTA (Gibco). Cells were harvested, centrifuged, and resuspended in 3% (v/v) FBS in PBS at a cell concentration of $1 \times 10^7$/ml. In accordance with the manufacture's specifications, antibody to the cell surface marker of interest (see below) was added to 100 $\mu$l of cell suspension and the mixture was incubated in the dark for 30 minutes at 4 °C. After incubation, cells were washed with PBS and centrifuged to remove unbound antibody. Cells were resuspended in 500 $\mu$l PBS and analyzed by flow cytometry. Flow cytometry analysis was performed with a FACS calibur instrument (Becton Dickinson, San Jose, CA).

The following antibodies to cell surface markers were used.

| Table 6-1: Antibodies used in characterizing cell surface markers of UDCs. | | |
|---|---|---|
| Antibody | Manufacturer | Catalog Number |
| CD10 | BD Pharmingen (San Diego, CA) | 555375 |
| CD13 | BD Pharmingen | 555394 |
| CD31 | BD Pharmingen | 555446 |
| CD34 | BD Pharmingen | 555821 |
| CD44 | BD Pharmingen | 555478 |
| CD45RA | BD Pharmingen | 555489 |
| CD73 | BD Pharmingen | 550257 |
| CD90 | BD Pharmingen | 555596 |

(continued)

| Table 6-1: Antibodies used in characterizing cell surface markers of UDCs. | | |
|---|---|---|
| Antibody | Manufacturer | Catalog Number |
| CD117 | BD Pharmingen | 340529 |
| CD141 | BD Pharmingen | 559781 |
| PDGFr-alpha | BD Pharmingen | 556002 |
| HLA-A, B, C | BD Pharmingen | 555553 |
| HLA-DR, DP, DQ | BD Pharmingen | 555558 |
| IgG-FITC | Sigma (St. Louis, MO) | F-6522 |
| IgG- PE | Sigma | P-4685 |

Umbilicus-derived cells were analyzed at passages 8, 15, and 20.

To compare differences among donors, umbilical cord tissue-derived cells from different donors were compared to each other. Umbilicus-derived cells cultured on gelatin-coated flasks were also compared to umbilicus-derived cells cultured on uncoated flasks.

Four treatments used for isolation and preparation of cells were compared. Cells derived from postpartum tissue by treatment with: 1) collagenase; 2) collagenase/dispase; 3) collagenase/hyaluronidase; and 4) collagenase/hyaluronidase/dispase were compared.

Umbilical cord-derived cells at passage 8, 15, and 20 analyzed by flow cytometry all expressed CD10, CD13, CD44, CD73, CD 90, PDGFr-alpha and HLA-A, B, C, indicated by increased fluorescence relative to the IgG control. These cells were negative for CD31, CD34, CD45, CD117, CD141, and HLA-DR, DP, DQ, indicated by fluorescence values consistent with the IgG control.

Umbilical cord-derived cells isolated from separate donors analyzed by flow cytometry each showed positive for the production of CD10, CD13, CD44, CD73, CD 90, PDGFr-alpha and HLA-A, B, C, reflected in the increased values of fluorescence relative to the IgG control. These cells were negative for the production of CD31, CD34, CD45, CD117, CD141, and HLA-DR, DP, DQ with fluorescence values consistent with the IgG control.

The umbilical cord-derived cells expanded on gelatin-coated and uncoated flasks analyzed by flow cytometry were all positive for the production of CD10, CD13, CD44, CD73, CD 90, PDGFr-alpha and HLA-A, B, C, with increased values of fluorescence relative to the IgG control. These cells were negative for the production of CD31, CD34, CD45, CD117, CD141, and HLA-DR, DP, DQ, with fluorescence values consistent with the IgG control.

Analysis of umbilical cord-derived cells by flow cytometry has established an identity of these cell lines. These umbilical cord-derived cells are positive for CD10, CD13, CD44, CD73, CD90, PDGFr-alpha, and HLA-A,B,C; and negative for CD31, CD34, CD45, CD117, CD141 and HLA-DR, DP, DQ. This identity was consistent between variations in variables including the donor, passage, culture vessel surface coating, digestion enzymes, and placental layer. Some variation in individual fluorescence value histogram curve means and ranges were observed, but all positive curves under all conditions tested were normal and expressed fluorescence values greater than the IgG control, thus confirming that the cells comprise a homogeneous population, which has positive expression of the markers.

## EXAMPLE 7

### Analysis of Cells by Oligonucleotide Array

Oligonucleotide arrays were used to compare gene expression profiles of umbilicus-derived and placenta-derived cells with fibroblasts, human mesenchymal stem cells, and another cell line derived from human bone marrow. This analysis provided a characterization of the postpartum-derived cells and identified unique molecular markers for these cells.

*Postpartum tissue-derived cells.* Human umbilical cords and placenta were obtained from National Disease Research Interchange (NDRI, Philadelphia, PA) from normal full term deliveries with patient consent. The tissues were received and cells were isolated as described in Example 5 after digestion with a C:D:H mixture. The cells were cultured in growth medium on gelatin-coated plastic tissue culture flasks. The cultures were incubated at 37° C with 5% $CO_2$.

*Fibroblasts.* Human dermal fibroblasts were purchased from Cambrex Incorporated (Walkersville, MD; Lot number 9F0844) and ATCC CRL-1501 (CCD39SK). Both lines were cultured in DMEM/F12 medium (Invitrogen, Carlsbad, CA) with 10% (v/v) fetal bovine serum (Hyclone) and penicillin/streptomycin (Invitrogen)). The cells were grown on standard tissue-treated plastic.

*Human Mesenchymal Stem Cells (hMSC).* hMSCs were purchased from Cambrex Incorporated (Walkersville, MD; Lot numbers 2F1655, 2F1656 and 2F1657) and cultured according to the manufacturer's specifications in MSCGM Media (Cambrex). The cells were grown on standard tissue cultured plastic at 37 °C with 5% $CO_2$.

*Human Iliac Crest Bone Marrow Cells (ICBM).* Human iliac crest bone marrow was received from NDRI with patient consent. The marrow was processed according to the method outlined by Ho, et al. (WO03/025149). The marrow was mixed with lysis buffer (155 mM $NH_4Cl$, 10 mM $KHCO_3$, and 0.1 mM EDTA, pH 7.2) at a ratio of 1 part bone marrow to 20 parts lysis buffer. The cell suspension was vortexed, incubated for 2 minutes at ambient temperature, and centrifuged for 10 minutes at 500 x g. The supernatant was discarded and the cell pellet was resuspended in Minimal Essential Medium-alpha (Invitrogen) supplemented with 10% (v/v) fetal bovine serum and 4 mM glutamine. The cells were centrifuged again and the cell pellet was resuspended in fresh medium. The viable mononuclear cells were counted using trypan blue exclusion (Sigma, St. Louis, MO). The mononuclear cells were seeded in plastic tissue culture flasks at 5 x $10^4$ cells/cm². The cells were incubated at 37 °C with 5% $CO_2$ at either standard atmospheric $O_2$ or at 5% $O_2$. Cells were cultured for 5 days without a media change. Media and non-adherent cells were removed after 5 days of culturing. The adherent cells were maintained in culture.

Actively growing cultures of cells were removed from the flasks with a cell scraper in cold phosphate buffered saline (PBS). The cells were centrifuged for 5 minutes at 300 x g. The supernatant was removed and the cells were resuspended in fresh PBS and centrifuged again. The supernatant was removed and the cell pellet was immediately frozen and stored at - 80° C. Cellular mRNA was extracted and transcribed into cDNA. The cDNA was then transcribed into cRNA and biotin-labeled. The biotin-labeled cRNA was hybridized with Affymetrix GENECHIP HG-U133A oligonucleotide arrays (Affymetrix, Santa Clara, CA). The hybridizations and data collection were performed according to the manufacturer's specifications. Data analysis was performed using "Significance Analysis of Microarrays" (SAM) version 1.21 computer software (Tusher, V.G. et al., 2001, Proc. Natl. Acad. Sci. USA 98: 5116-5121). Licenses for the analysis software are available through the Office of Technology Licensing, Stanford University, and more information is available on the World Wide Web at Professor Tibshirani's web site in the Dep't of Statistics, Stanford University.

Fourteen different populations of cells were analyzed in this study. The cells, along with passage information, culture substrate, and culture media are listed in Table 7-1. The cells lines are listed by their identification code along with passage at the time of analysis, cell growth substrate, and growth media.

| Table 7-1: Cells analyzed by the microarray study. | | | |
|---|---|---|---|
| **Cell Population** | **Passage** | **Substrate** | **Media** |
| Umbilical (022803) | 2 | Gelatin | DMEM, 15% FBS, 2-BME |
| Umbilical (042103) | 3 | Gelatin | DMEM, 15% FBS, 2-BME |
| Umbilical (071003) | 4 | Gelatin | DMEM, 15% FBS, 2-BME |
| Placenta (042203) | 12 | Gelatin | DMEM, 15% FBS, 2-BME |
| Placenta (042903) | 4 | Gelatin | DMEM, 15% FBS, 2-BME |
| Placenta (071003) | 3 | Gelatin | DMEM, 15% FBS, 2-BME |
| ICBM (070203) (5% $O_2$) | 3 | Plastic | MEM 10% FBS |
| ICBM (062703) (std. $O_2$) | 5 | Plastic | MEM 10% FBS |
| ICBM (062703)(5% $O_2$) | 5 | Plastic | MEM 10% FBS |
| hMSC (Lot 2F1655) | 3 | Plastic | MSCGM |
| hMSC (Lot 2F1656) | 3 | Plastic | MSCGM |
| hMSC (Lot 2F1657) | 3 | Plastic | MSCGM |
| hFibroblast (9F0844) | 9 | Plastic | DMEM-F12, 10% FBS |
| hFibroblast (CCD39SK) | 4 | Plastic | DMEM-F12, 10% FBS |

The data were evaluated by principle component analysis with SAM software as described above. The analysis revealed 290 genes that were expressed in different relative amounts in the cells tested. This analysis provided relative comparisons between the populations.

Table 7-2 shows the Euclidean distances that were calculated for the comparison of the cell pairs. The Euclidean distances were based on the comparison of the cells based on the 290 genes that were differentially expressed among the cell types. The Euclidean distance is inversely proportional to similarity between the expression of the 290 genes. The Euclidean distance was calculated for the cell types using these 290 genes expressed differentially between the cell types. Similarity between the cells is inversely proportional to the Euclidean distance.

### Table 7-2. The Euclidean Distances for the Cell Pairs.

| Cell Pair | Euclidean Distance |
|---|---|
| ICBM-hMSC | 24.71 |
| Placenta-umbilical | 25.52 |
| ICBM-Fibroblast | 36.44 |
| ICBM-placenta | 37.09 |
| Fibroblast-MSC | 39.63 |
| ICBM-Umbilical | 40.15 |
| Fibro blast-Umbilical | 41.59 |
| MSC-Placenta | 42.84 |
| MSC-Umbilical | 46.86 |
| ICBM-placenta | 48.41 |

Tables 7-3, 7-4, and 7-5 show the expression of genes increased in placenta-derived cells (Table 7-3), increased in umbilical cord-derived cells (Table 7-4), and reduced in umbilical cord and placenta-derived cells (Table 7-5).

### Table 7-3: Genes which are specifically increased in expression in the placenta-derived cells as compared to the other cell lines assayed.

| Probe Set ID | Gene Name | NCBI Accession Number |
|---|---|---|
| 209732_at | C-type (calcium dependent, carbohydrate-recognition domain) lectin, superfamily member 2 (activation-induced) | AF070642 |
| 206067_s_at | Wilms tumor 1 | NM_024426 |
| 207016_s_at | aldehyde dehydrogenase 1 family, member A2 | AB015228 |
| 206367_at | Renin | NM_000537 |
| 210004_at | oxidized low density lipoprotein (lectin-like) receptor 1 | AF035776 |
| 214993_at | *Homo sapiens,* clone IMAGE:4179671, mRNA, partial cds | AF070642 |
| 202178_at | protein kinase C, zeta | NM_002744 |
| 209780_at | hypothetical protein DKFZp564F013 | AL136883 |
| 204135_at | downregulated in ovarian cancer 1 | NM_014890 |
| 213542_at | *Homo sapiens* mRNA; cDNA DKFZp547K1113 (from clone DKFZp547K1113) | AI246730 |

### Table 7-4. Genes which are specifically increased in expression in umbilical cord - derived cells as compared to the other cell lines assayed.

| Probe Set ID | Gene Name | NCBI Accession Number |
|---|---|---|
| 202859_x_at | Interleukin 8 | NM_000584 |
| 211506_s_at | Interleukin 8 | AF043337 |
| 210222_s_at | reticulon 1 | BC000314 |
| 204470_at | chemokine (C-X-C motif) ligand 1 (melanoma growth stimulating activity | NM_001511 |

(continued)

**Table 7-4. Genes which are specifically increased in expression in umbilical cord - derived cells as compared to the other cell lines assayed.**

| Probe Set ID | Gene Name | NCBI Accession Number |
|---|---|---|
| 206336_at | chemokine (C-X-C motif) ligand 6 (granulocyte chemotactic protein 2) | NM_002993 |
| 207850_at | Chemokine (C-X-C motif) ligand 3 | NM_002090 |
| 203485_at | reticulon 1 | NM_021136 |
| 202644_s_at | tumor necrosis factor, alpha-induced protein 3 | NM_006290 |

**Table 7-5: Genes which were decreased in expression in the umbilical cord and placenta cells as compared to the other cell lines assayed.**

| Probe Set ID | Gene name | NCBI Accession # |
|---|---|---|
| 210135_s_at | short stature homeobox 2 | AF022654.1 |
| 205824_at | heat shock 27kDa protein 2 | NM_001541.1 |
| 209687_at | chemokine (C-X-C motif) ligand 12 (stromal cell-derived factor 1) | U19495.1 |
| 203666_at | chemokine (C-X-C motif) ligand 12 (stromal cell-derived factor 1) | NM_000609.1 |
| 212670_at | elastin (supravalvular aortic stenosis, Williams-Beuren syndrome) | AA479278 |
| 213381_at | *Homo sapiens* mRNA; cDNA DKFZp586M2022 (from clone DKFZp586M2022) | N91149 |
| 206201_s_at | mesenchyme homeobox 2 (growth arrest-specific homeobox) | NM_005924.1 |
| 205817_at | Sine oculis homeobox homolog 1 (*Drosophila*) | NM_005982.1 |
| 209283_at | crystallin, alpha B | AF007162.1 |
| 212793_at | dishevelled associated activator of morphogenesis 2 | BF513244 |
| 213488_at | DKFZP586B2420 protein | AL050143.1 |
| 209763_at | similar to neuralin 1 | AL049176 |
| 205200_at | Tetranectin (plasminogen binding protein) | NM_003278.1 |
| 205743_at | src homology three (SH3) and cysteine rich domain | NM_003149.1 |
| 200921_s_at | B-cell translocation gene 1, anti-proliferative | NM_001731.1 |
| 206932_at | cholesterol 25-hydroxylase | NM_003956.1 |
| 204198_s_at | runt-related transcription factor 3 | AA541630 |
| 219747_at | hypothetical protein FLJ23191 | NM_024574.1 |
| 204773_at | Interleukin 11 receptor, alpha | NM_004512.1 |
| 202465_at | Procollagen C-endopeptidase enhancer | NM_002593.2 |
| 203706_s_at | Frizzled homolog 7 (*Drosophila*) | NM_003507.1 |
| 212736_at | hypothetical gene BC008967 | BE299456 |

(continued)

| Table 7-5: Genes which were decreased in expression in the umbilical cord and placenta cells as compared to the other cell lines assayed. | | |
|---|---|---|
| **Probe Set ID** | **Gene name** | **NCBI Accession #** |
| 214587_at | Collagen, type VIII, alpha 1 | BE877796 |
| 201645_at | Tenascin C (hexabrachion) | NM_002160.1 |
| 210239_at | iroquois homeobox protein 5 | U90304.1 |
| 203903_s_at | Hephaestin | NM_014799.1 |
| 205816_at | integrin, beta 8 | NM_002214.1 |
| 203069_at | synaptic vesicle glycoprotein 2 | NM_014849.1 |
| 213909_at | *Homo sapiens* cDNA FLJ12280 fis, clone MAMMA1001744 | AU147799 |
| 206315_at | cytokine receptor-like factor 1 | NM_004750.1 |
| 204401_at | potassium intermediate/small conductance calcium-activated channel, subfamily N, member 4 | NM_002250.1 |
| 216331_at | integrin, alpha 7 | AK022548.1 |
| 209663_s_at | integrin, alpha 7 | AF072132.1 |
| 213125_at | DKFZP586L151 protein | AW007573 |
| 202133_at | transcriptional co-activator with PDZ-binding motif (TAZ) | AA081084 |
| 206511_s_at | Sine oculis homeobox homolog 2 (*Drosophila*) | NM_016932.1 |
| 213435_at | KIAA1034 protein | AB028957.1 |
| 206115_at | early growth response 3 | NM_004430.1 |
| 213707_s_at | distal-less homeobox 5 | NM_005221.3 |
| 218181_s_at | hypothetical protein FLJ20373 | NM_017792.1 |
| 209160_at | aldo-keto reductase family 1, member C3 (3-alpha hydroxysteroid dehydrogenase, type II) | AB018580.1 |
| 213905_x_at | Biglycan | AA845258 |
| 201261_x_at | Biglycan | BC002416.1 |
| 202132_at | transcriptional co-activator with PDZ-binding motif (TAZ) | AA081084 |
| 214701_s_at | fibronectin 1 | AJ276395.1 |
| 213791_at | Proenkephalin | NM_006211.1 |
| 205422_s_at | Integrin, beta-like 1 (with EGF-like repeat domains) | NM_004791.1 |
| 214927_at | *Homo sapiens* mRNA full length insert cDNA clone EUROIMAGE 1968422 | AL359052.1 |
| 206070_s_at | EphA3 | AF213459.1 |
| 212805_at | KIAA0367 protein | AB002365.1 |
| 219789_at | natriuretic peptide receptor C/guanylate cyclase C (atrionatriuretic peptide receptor C) | AI628360 |
| 219054_at | hypothetical protein FLJ14054 | NM_024563.1 |

(continued)

| Table 7-5: Genes which were decreased in expression in the umbilical cord and placenta cells as compared to the other cell lines assayed. | | |
|---|---|---|
| Probe Set ID | Gene name | NCBI Accession # |
| 213429_at | *Homo sapiens* mRNA; cDNA DKFZp564B222 (from clone DKFZp564B222) | AW025579 |
| 204929_s_at | vesicle-associated membrane protein 5 (myobrevin) | NM_006634.1 _ |
| 201843_s_at | EGF-containing fibulin-like extracellular matrix protein 1 | NM_004105.2 |
| 221478_at | BCL2/adenovirus E1B 19kDa interacting protein 3-like | AL132665.1 |
| 201792_at | AE binding protein 1 | NM_001129.2 |
| 204570_at | cytochrome c oxidase subunit VIIa polypeptide 1 (muscle) | NM_001864.1 |
| 201621_at | neuroblastoma, suppression of tumorigenicity 1 | NM_005380.1 |
| 202718_at | Insulin-like growth factor binding protein 2, 36kDa | NM_000597.1 |

Tables 7-6, 7-7, and 7-8 show the expression of genes increased in human fibroblasts (Table 7-6), ICBM cells (Table 7-7), and MSCs (Table 7-8).

| Table 7-6: Genes which were increased in expression in fibroblasts as compared to the other cell lines assayed. |
|---|
| dual specificity phosphatase 2 |
| KIAA0527 protein |
| *Homo sapiens* cDNA: FLJ23224 fis, clone ADSU02206 |
| dvnein, cytoplasmic, intermediate polypeptide 1 |
| ankyrin 3, node of Ranvier (ankyrin G) |
| inhibin, beta A (activin A, activin AB alpha polypeptide) |
| ectonucleotide pyrophosphatase/phosphodiesterase 4 (putative function) |
| KIAA1053 protein |
| microtubule-associated protein 1A |
| zinc finger protein 41 |
| HSPC019 protein |
| *Homo sapiens* cDNA: FLJ23564 fis, clone LNG10773 |
| *Homo sapiens* mRNA; cDNA DKFZp564A072 (from clone DKFZp564A072) |
| LIM protein (similar to rat protein kinase C-binding enigma) |
| inhibitor of kappa light polypeptide gene enhancer in B-cells, kinase complex-associated protein |
| hypothetical protein FLJ22004 |
| Human (clone CTG-A4) mRNA sequence |
| ESTs, Moderately similar to cytokine receptor-like factor 2; cytokine receptor CRL2 precursor [*Homo sapiens*] |
| transforming growth factor, beta 2 |
| hypothetical protein MGC29643 |

(continued)

| Table 7-6: Genes which were increased in expression in fibroblasts as compared to the other cell lines assayed. |
| --- |
| antigen identified by monoclonal antibody MRC OX-2 |
| putative X-linked retinopathy protein |

| Table 7-7: Genes which were increased in expression in the ICBM-derived cells as compared to the other cell lines assayed. |
| --- |
| •cardiac ankyrin repeat protein |
| •MHC class I region ORF |
| •integrin, alpha 10 |
| •hypothetical protein FLJ22362 |
| •UDP-N-acetyl-alpha-D-galactosamine:polypeptide N-acetylgalactosaminyltransferase 3 (GalNAc-T3) |
| •interferon-induced protein 44 |
| •SRY (sex determining region Y)-box 9 (campomelic dysplasia, autosomal sex-reversal) |
| •keratin associated protein 1-1 |
| •hippocalcin-like 1 |
| •jagged 1 (Alagille syndrome) |
| •proteoglycan 1, secretory granule |

| Table 7-8: Genes which were increased in expression in the MSC cells as compared to the other cell lines assayed. |
| --- |
| •interleukin 26 |
| •maltase-glucoamylase ($\alpha$-glucosidase) |
| •nuclear receptor subfamily 4, group A, member 2 |
| •v-fos FBJ murine osteosarcoma viral oncogene homolog |
| •hypothetical protein DC42 |
| •nuclear receptor subfamily 4, group A, member 2 |
| •FBJ murine osteosarcoma viral oncogene homolog B |
| •WNT1 inducible signaling pathway protein 1 |
| •MCF.2 cell line derived transforming sequence |
| •potassium channel, subfamily K, member 15 |
| •cartilage paired-class homeoprotein 1 |
| •*Homo sapiens* cDNA FLJ12232 fis, clone MAMMA1001206 |
| •*Homo sapiens* cDNA FLJ34668 fis, clone LIVER2000775 |
| •jun B proto-oncogene |
| •B-cell CLL/lymphoma 6 (zinc finger protein 51) |
| •zinc finger protein 36, C3H type, homolog (mouse) |

This example was performed to provide a molecular characterization of the cells derived from umbilical cord and placenta. This analysis included cells derived from three different umbilical cords and three different placentas. The study also included two different lines of dermal fibroblasts, three lines of mesenchymal stem cells, and three lines of iliac crest bone marrow cells. The mRNA that was expressed by these cells was analyzed on a GENECHIP oligonucleotide array that contained oligonucleotide probes for 22,000 genes.

The analysis revealed that transcripts for 290 genes were present in different amounts in these five different cell types. These genes include ten genes that are specifically increased in the placenta-derived cells and seven genes specifically increased in the umbilical cord-derived cells. Fifty-four genes were found to have specifically lower expression levels in placenta-derived and umbilical cord tissue-derived cells.

## EXAMPLE 8

### Immunohistochemical Characterization of Cellular Phenotypes

The phenotypes of cells found within human umbilical cord tissue were analyzed by immunohistochemistry.

Human umbilical cord tissue was harvested and immersion fixed in 4% (w/v) paraformaldehyde overnight at 4°C. Immunohistochemistry was performed using antibodies directed against the following epitopes (*see* Table 8-1): vimentin (1:500; Sigma, St. Louis, MO), desmin (1:150, raised against rabbit; Sigma; or 1:300, raised against mouse; Chemicon, Temecula, CA), alpha-smooth muscle actin (SMA; 1:400; Sigma), cytokeratin 18 (CK18; 1:400; Sigma), von Willebrand Factor (vWF; 1:200; Sigma), and CD34 (human CD34 Class III; 1:100; DAKOCytomation, Carpinteria, CA). In addition, the following markers were tested: anti-human GROalpha-PE (1:100; Becton Dickinson, Franklin Lakes, NJ), anti-human GCP-2 (1:100; Santa Cruz Biotech, Santa Cruz, CA), anti-human oxidized LDL receptor 1 (ox-LDL R1; 1:100; Santa Cruz Biotech), and anti-human NOGO-A (1:100; Santa Cruz Biotech). Fixed specimens were trimmed with a scalpel and placed within OCT embedding compound (Tissue-Tek OCT; Sakura, Torrance, CA) on a dry ice bath containing ethanol. Frozen blocks were then sectioned (10 $\mu$m thick) using a standard cryostat (Leica Microsystems) and mounted onto glass slides for staining.

Immunohistochemistry was performed similar to previous studies. (*E.g.*, Messina et al., Exper. Neurol., 2003; 184: 816-829). Tissue sections were washed with phosphate-buffered saline (PBS) and exposed to a protein blocking solution containing PBS, 4% (v/v) goat serum (Chemicon, Temecula, CA), and 0.3% (v/v) Triton (Triton X-100; Sigma) for 1 hour to access intracellular antigens. In instances where the epitope of interest would be located on the cell surface (CD34, ox-LDL R1), triton was omitted in all steps of the procedure in order to prevent epitope loss. Furthermore, in instances where the primary antibody was raised against goat (GCP-2, ox-LDL R1, NOGO-A), 3% (v/v) donkey serum was used in place of goat serum throughout the procedure. Primary antibodies, diluted in blocking solution, were then applied to the sections for a period of 4 hours at room temperature. Primary antibody solutions were removed, and cultures washed with PBS prior to application of secondary antibody solutions (1 hour at room temperature) containing block along with goat anti-mouse IgG-Texas Red (1:250; Molecular Probes, Eugene, OR) and/or goat anti-rabbit IgG-Alexa 488 (1:250; Molecular Probes) or donkey anti-goat IgG-FITC (1:150; Santa Cruz Biotech). Cultures were washed, and 10 micromolar DAPI (Molecular Probes) was applied for 10 minutes to visualize cell nuclei.

Following immune-staining, fluorescence was visualized using the appropriate fluorescence filter on an Olympus inverted epifluorescent microscope (Olympus, Melville, NY). Positive staining was represented by fluorescence signal above control staining. Representative images were captured using a digital color video camera and ImagePro software (Media Cybernetics, Carlsbad, CA). For triple-stained samples, each image was taken using only one emission filter at a time. Layered montages were then prepared using Adobe Photoshop software (Adobe, San Jose, CA).

| **Table 8-1:** Summary of Primary Antibodies Used | | |
|---|---|---|
| **Antibody** | **Concentration** | **Vendor** |
| Vimentin | 1:500 | Sigma, St. Louis, MO |
| Desmin (rb) | 1:150 | Sigma |
| Desmin (m) | 1:300 | Chemicon, Temecula, CA |
| alpha-smooth muscle actin (SMA) | 1:400 | Sigma |
| Cytokeratin 18 (CK18) | 1:400 | Sigma |
| von Willebrand factor (vWF) | 1:200 | Sigma |
| CD34 III | 1:100 | DakoCytomation, Carpinteria, CA |
| GROalpha-PE | 1:100 | BD, Franklin Lakes, NJ |
| GCP-2 | 1:100 | Santa Cruz Biotech |
| Ox-LDL R1 | 1:100 | Santa Cruz Biotech |

(continued)

| **Table 8-1:** Summary of Primary Antibodies Used | | |
|---|---|---|
| **Antibody** | **Concentration** | **Vendor** |
| NOGO-A | 1:100 | Santa Cruz Biotech |

Vimentin, desmin, SMA, CK18, vWF, and CD34 markers were expressed in a subset of the cells found within umbilical cord (data not shown). In particular, vWF and CD34 expression were restricted to blood vessels contained within the cord. CD34+ cells were on the innermost layer (lumen side). Vimentin expression was found throughout the matrix and blood vessels of the cord. SMA was limited to the matrix and outer walls of the artery and vein, but not contained within the vessels themselves. CK18 and desmin were observed within the vessels only, desmin being restricted to the middle and outer layers.

None of these markers were observed within umbilical cord (data not shown).

Vimentin, desmin, alpha-smooth muscle actin, cytokeratin 18, von Willebrand Factor, and CD 34 are expressed in cells within human umbilical cord. Based on *in vitro* characterization studies showing that only vimentin and alpha-smooth muscle actin are expressed, the data suggests that the current process of umbilical cord-derived cell isolation harvests a subpopulation of cells or that the cells isolated change expression of markers to express vimentin and alpha-smooth muscle actin.

## EXAMPLE 9

## Secretion of Trophic Factors

The secretion of selected trophic factors from UTC was measured. Factors were selected that have angiogenic activity *e.g.,* hepatocyte growth factor (HGF) (Rosen et al., Ciba Found. Symp., 1997; 212:215-26); monocyte chemotactic protein 1 (MCP-1) (Salcedo et al., Blood, 2000; 96;34-40); interleukin-8 (IL-8) (Li et al., J. Immunol., 2003; 170:3369-76); keratinocyte growth factor (KGF); basic fibroblast growth factor (bFGF); vascular endothelial growth factor (VEGF) (Hughes et al., Ann. Thorac. Surg. 2004; 77:812-8); tissue inhibitor of matrix metalloproteinase 1 (TIMP1); angiopoietin 2 (ANG2); platelet derived growth factor (PDGFbb); thrombopoietin (TPO); heparin-binding epidermal growth factor (HB-EGF); stromal-derived factor 1alpha (SDF-1alpha), neurotrophic/neuroprotective activity (brain-derived neurotrophic factor (BDNF) (Cheng et al., Dev. Biol., 2003; 258;319-33); interleukin-6 (IL-6); granulocyte chemotactic protein-2 (GCP-2); transforming growth factor beta2 (TGFbeta2)); or chemokine activity (macrophage inflammatory protein 1alpha (MIP1alpha); macrophage inflammatory protein 1 beta (MIP1beta); monocyte chemoattractant-1 (MCP-1); Rantes (regulated on activation, normal T cell expressed and secreted); 1309; thymus and activation-regulated chemokine (TARC); Eotaxin; macrophage-derived chemokine (MDC); and (IL-8).

Cells derived from umbilical cord, as well as human fibroblasts derived from human neonatal foreskin, were cultured in growth medium on gelatin-coated T75 flasks. Cells were cryopreserved at passage 11 and stored in liquid nitrogen. After thawing, growth medium was added to the cells, followed by transfer to a 15 ml centrifuge tube and centrifugation of the cells at 150 x g for 5 minutes. The cell pellet was resuspended in 4 ml growth medium, and the cells were counted. Cells were seeded at 5,000 cells/cm$^2$ in T75 flasks each containing 15 ml of growth medium, and cultured for 24 hours. The medium was changed to a serum-free medium (DMEM-low glucose (Gibco), 0.1% (w/v) bovine serum albumin (Sigma), penicillin (50 U/ml) and streptomycin (50 $\mu$g/ml, Gibco)) for 8 hours. Conditioned serum-free medium was collected at the end of incubation by centrifugation at 14,000 x g for 5 minutes and stored at -20°C.

To estimate the number of cells in each flask, the cells were washed with phosphate-buffered saline (PBS) and detached using 2 ml trypsin/EDTA (Gibco). Trypsin activity was inhibited by addition of 8 ml growth medium. The cells were centrifuged at 150 x g for 5 minutes. The supernatant was removed, and the cells were resuspended in 1 ml Growth Medium. The cell number was estimated with a hemocytometer.

Cells were grown at 37°C in 5% CO$_2$ and atmospheric oxygen. The amount of MCP-1, IL-6, VEGF, SDF-1alpha, GCP-2 , IL-8, and TGF-beta2 produced by each cell sample was determined by ELISA (R&D Systems, Minneapolis, Mn.). All assays were performed according to the manufacturer's instructions. Values presented are picograms per ml per million cells (n=2, sem).

Chemokines (MIP1alpha, MIP1beta, MCP-1, Rantes, 1309, TARC, Eotaxin, MDC, IL8), BDNF, and angiogenic factors (HGF, KGF, bFGF, VEGF, TIMP1, ANG2, PDGFbb, TPO, HB-EGF were measured using SearchLight Proteome Arrays (Pierce Biotechnology Inc.). The Proteome Arrays are multiplexed sandwich ELISAs for the quantitative measurement of two to sixteen proteins per well. The arrays are produced by spotting a 2 x 2, 3 x 3, or 4 x 4 pattern of four to sixteen different capture antibodies into each well of a 96-well plate. Following a sandwich ELISA procedure, the entire plate is imaged to capture the chemiluminescent signal generated at each spot within each well of the plate. The signal generated at each spot is proportional to the amount of target protein in the original standard or sample.

MCP-1 and IL-6 were secreted by umbilicus-derived PPDCs and dermal fibroblasts (Table 9-1). SDF-1alpha and GCP-2 were secreted by fibroblasts. GCP-2 and IL-8 were secreted by umbilicus-derived PPDCs. TGF-beta2 was not detected from either cell type by ELISA.

| Table 9-1. ELISA Results: Detection of Trophic Factors | | | | | | | |
|---|---|---|---|---|---|---|---|
| | **MCP-1** | **IL-6** | **VEGF** | **SDF-1$\alpha$** | **GCP-2** | **IL-8** | **TGF-beta2** |
| **Fibroblast** | 17±1 | 61±3 | 29±2 | 19±1 | 21±1 | ND | ND |
| **Umbilical (022803)** | 1150±74 | 4234+289 | ND | ND | 160±11 | 2058±145 | ND |
| **Umbilical (071003)** | 2794+84 | 1356+43 | ND | ND | 2184+98 | 2369+23 | ND |
| Key: ND: Not Detected., =/- sem | | | | | | | |

Searchlight™ Multiplexed ELISA assay. TIMP1, TPO, KGF, HGF, FGF, HBEGF, BDNF, MIP1beta, MCP1, RANTES, 1309, TARC, MDC, and IL-8 were secreted from umbilicus-derived PPDCs (Tables 9-2 and 9-3). No Ang2, VEGF, or PDGFbb were detected.

| Table 9-2. Searchlight™ Multiplexed ELISA assay results | | | | | | | | | |
|---|---|---|---|---|---|---|---|---|---|
| | **TIMP1** | **ANG2** | **PDGFbb** | **TPO** | **KGF** | **HGF** | **FGF** | **VEGF** | **HBEGF** | **BDNF** |
| hFB | 19306.3 | ND | ND | 230.5 | 5.0 | ND | ND | 27.9 | 1.3 | ND |
| **U1** | 57718.4 | ND | ND | 1240.0 | 5.8 | 559.3 | 148.7 | ND | 9.3 | 165.7 |
| U3 | 21850.0 | ND | ND | 1134.5 | 9.0 | 195.6 | 30.8 | ND | 5.4 | 388.6 |
| Key: hFB (human fibroblasts), U1 (umbilicus-derived PPDC (022803)), U3 (umbilicus-derived PPDC (071003)), ND: Not Detected. | | | | | | | | | |

| Table 9-3. Searchlight™ Multiplexed ELISA assay results | | | | | | | | | |
|---|---|---|---|---|---|---|---|---|---|
| | **MIP1a** | **MIP1b** | **MCP1** | **RANTES** | **1309** | **TARC** | **Eotaxin** | **MDC** | **IL8** |
| **hFB** | ND | ND | 39.6 | ND | ND | 0.1 | ND | ND | 204.9 |
| **U1** | ND | 8.0 | 1694.2 | ND | 22.4 | 37.6 | ND | 18.9 | 51930.1 |
| **U3** | ND | 5.2 | 2018.7 | 41.5 | 11.6 | 21.4 | ND | 4.8 | 10515.9 |
| Key: hFB (human fibroblasts), U1 (umbilicus-derived PPDC (022803)), U3 (umbilicus-derived PPDC (071003)), ND: Not Detected | | | | | | | | | |

Umbilicus-derived cells secreted a number of trophic factors. Some of these trophic factors, such as HGF, bFGF, MCP-1 and IL-8, play important roles in angiogenesis. Other trophic factors, such as BDNF and IL-6, have important roles in neural regeneration or protection.

**EXAMPLE 10**

**Assay for Telomerase Activity**

Telomerase functions to synthesize telomere repeats that serve to protect the integrity of chromosomes and to prolong the replicative life span of cells (Liu, K, et al., PNAS, 1999; 96:5147-5152). Telomerase consists of two components, telomerase RNA template (hTER) and telomerase reverse transcriptase (hTERT). Regulation of telomerase is determined by transcription of hTERT but not hTER. Real-time polymerase chain reaction (PCR) for hTERT mRNA thus is an accepted method for determining telomerase activity of cells.

Cell Isolation

Real-time PCR experiments were performed to determine telomerase production of human umbilical cord tissue-derived cells. Human umbilical cord tissue-derived cells were prepared in accordance with the above Examples and the examples set forth in U.S. Patent No. 7,510,873. Generally, umbilical cords obtained from National Disease Research Interchange (Philadelphia, Pa.) following a normal delivery were washed to remove blood and debris and mechanically dissociated. The tissue was then incubated with digestion enzymes including collagenase, dispase, and hyaluronidase in culture medium at 37°C. Human umbilical cord tissue-derived cells were cultured according to the methods set forth in the examples of the '012 application. Mesenchymal stem cells and normal dermal skin fibroblasts (cc-2509 lot # 9F0844) were obtained from Cambrex, Walkersville, Md. A pluripotent human testicular embryonal carcinoma (teratoma) cell line nTera-2 cells (NTERA-2 cl.DI) (*See,* Plaia et al., Stem Cells, 2006; 24(3):531-546) was purchased from ATCC (Manassas, Va.) and was cultured according to the methods set forth in U.S. Patent No. 7,510,873.

Total RNA Isolation

RNA was extracted from the cells using RNeasy® kit (Qiagen, Valencia, Ca.). RNA was eluted with 50 $\mu$l DEPC-treated water and stored at -80°C. RNA was reverse transcribed using random hexamers with the TaqMan® reverse transcription reagents (Applied Biosystems, Foster City, Ca.) at 25°C for 10 minutes, 37°C for 60 minutes and 95°C for 10 minutes. Samples were stored at -20°C.

Real-time PCR

PCR was performed on cDNA samples using the Applied Biosystems Assays-On-Demand™ (also known as TaqMan® Gene Expression Assays) according to the manufacturer's specifications (Applied Biosystems). This commercial kit is widely used to assay for telomerase in human cells. Briefly, hTert (human telomerase gene) (Hs00162669) and human GAPDH (an internal control) were mixed with cDNA and TaqMan® Universal PCR master mix using a 7000 sequence detection system with ABI prism 7000 SDS software (Applied Biosystems). Thermal cycle conditions were initially 50°C for 2 minutes and 95°C for 10 minutes followed by 40 cycles of 95°C for 15 seconds and 60°C for 1 minute. PCR data was analyzed according to the manufacturer's specifications.

Human umbilical cord tissue-derived cells (ATCC Accession No. PTA-6067), fibroblasts, and mesenchymal stem cells were assayed for hTert and 18S RNA. As shown in Table 10-1, hTert, and hence telomerase, was not detected in human umbilical cord tissue-derived cells.

| Table 10-1 | | |
| --- | --- | --- |
| | hTert | 18S RNA |
| Umbilical cells (022803) | ND | + |
| Fibroblasts | ND | + |
| ND- not detected; + signal detected | | |

Human umbilical cord tissue-derived cells (isolate 022803, ATCC Accession No. PTA-6067) and nTera-2 cells were assayed and the results showed no expression of the telomerase in two lots of human umbilical cord tissue-derived cells while the teratoma cell line revealed high level of expression (Table 10-2).

| Table 10-2 | | | | | |
| --- | --- | --- | --- | --- | --- |
| Cell type | hTert | | GAPDH | | hTert norm |
| | Exp. 1 | Exp. 2 | Exp. 1 | Exp. 2 | |
| nTera2 | 25.85 | 27.31 | 16.41 | 16.31 | 0.61 |
| 022803 | - | - | 22.97 | 22.79 | - |

Therefore, it can be concluded that the human umbilical tissue-derived cells of the present invention do not express telomerase.

## EXAMPLE 11

### Growth of UTC on Microcarriers in Impeller Spinner Flask Reactor

**Materials and Methods**

*Cells.* Cells from CBAT lot# 050604B passage 8 cells were thawed and expanded in a T225 flask for one passage.

Microcarriers. Cytodex® 3 (GE Healthcare Life Sciences, cat. no, 17-0485) microcarrier beads were hydrated in PBS for at least 3 hours and autoclaved.

*Spinner Flasks.* Spinner Flask with Internal Overhead Bearing Impeller Assembly, 100 ml and 250 ml (Bellco, Inc.).

*Confluence.* Confluence is defined as approximately 90% of the microcarriers observed in a representative microscopy field to have greater than approximately 60% of their surface area covered with cells.

*Passage.* Passage is defined as inoculating a spinner flask containing fresh microcarriers with an aliquot of confluent microcarriers obtained from a separate spinner flask culture.

*Inoculation and Culture.* Cells were harvested from T225 flask by trypsin and 4.0E+06 cell aliquots were added to 330 mg of microcarrier beads in 100 ml impeller or glass rod spinner flask containing 40 ml media. Flasks were flushed with 5% $CO_2$ gas for 1 minute prior to incubation. The inoculum speed-frequency was 30 rpm for 2 minutes every 30 minutes for 8 hours. At eight hours, media volume was increased to 100 ml and the spinner speed was set to 45-rpm continuous rotation and incubated at 37°C.

*Passage.* Passage 1- (100 ml to 250 ml flask) Cells were cultured for eight days. All microcarriers from the 100 ml flask are collected and allowed to separate from media by gravity. Media was aspirated and microcarriers were re-suspended in 10 ml fresh media. After pipetting to ensure an even distribution, 5ml of media with microcarriers were removed and delivered into a 250 ml spinner flask. Approximately 660 mg of fresh hydrated and autoclaved Cytodex® 3 microcarriers and media were also added to the flask. The media volume was increased to 200 ml and the flasks were flushed with 5% $CO_2$ gas for 1 minute prior to incubation. The spinner speed was set to 45-rpm continuous rotation and incubated at 37°C. Remaining cells were harvested by trypsinization and counted by using a Guava PCA instrument (Guava Technologies, Hayward, CA).

Passage 2 - (250 ml to 250 ml flask) Cells were cultured for six days. All microcarriers from the 250 ml flask are collected and allowed to separate from media by gravity. Media was aspirated and microcarriers were re-suspended in 25 ml fresh media. After pipetting to ensure an even distribution, 5ml of media with microcarriers were removed and delivered into a 250 ml spinner flask. Approximately 660 mg of fresh hydrated and autoclaved Cytodex® 3 microcarriers and media were also added to the flask. The media volume was increased to 200 ml and the flasks were flushed with 5% $CO_2$ gas for 1 minute prior to incubation. The spinner speed was set to 45-rpm continuous rotation and incubated at 37°C. Remaining cells were harvested by trypsinization and counted by using a Guava PCA instrument.

*Media Exchange.* Spinner flasks were removed from culture and the microcarriers were allowed to settle by gravity to the bottom of the flask. Approximately half the media volume was removed by aspiration and replaced with an equal volume of fresh media. The flasks were flushed with 5% $CO_2$ gas for 1 minute and returned to culture. Media exchange was performed on day 1, and day 4.

*Viability Staining.* A 1 ml aliquot was removed from flask and microcarriers were allowed to settle by gravity. Media was removed by aspiration and replaced with 1ml Live/Dead staining solution (Molecular Probes cat. no. L3224) and incubated for 15 minutes at 37° C. After incubation a 20-microliter aliquot was applied to a glass microscope slide and observed by fluorescent microscopy. Live cells stain green, dead cells stain red. Microscopic fields were manually analyzed to evaluate the distribution and ratio of live and dead cells adhered to the microcarriers. At least three microscopic fields were evaluated and the approximate percentage of viable cells was counted.

*Cell Harvest.* Microcarriers were collected from the spinner flask, washed three times in PBS, and evenly distributed between two 50 ml conical tubes. Each tube was incubated with 25 ml trypsin for 10 minutes at 37° C. Tubes were brought to 50 ml volume with PBS and microcarriers were allowed to settle by gravity. Supernatant containing cells was collected by aspiration and transferred to 50 ml conical tubes pre-filled with 2.5 ml of FBS (yielding a 5% FBS solution to inactivate trypsin). This process was repeated four times with each fraction collected separately. All harvested cells were centrifuged, re-suspended in serum containing growth media, and cells were counted by using a Guava PCA instrument.

*Static T-Flask Culture.* An aliquot of cells harvested from the T225 flask are used to seed two T225 flasks and incubated for four days using methods stated in U.S. Patent Application No. 10/877012. The cells were harvested and analyzed by flow cytometry

*Flow Cytometry.* Cells harvested were analyzed by flow cytometry using a Becton-Dickinson FACSCalibur™ instrument (Becton Dickinson, San Jose, CA) to determine the cell surface marker profile. All antibodies purchased from BD PharMingen (San Diego, CA).

**Results**

*Cell Harvest.* Table 11-1 shows the harvest fractions, cell yields and viability per passage from UTC cell line 050604B expanded from passage nine to passage eleven on Cytodex® 3 microcarriers in spinner flask cultures.

| Table 11-1: Cell Harvest | | | |
|---|---|---|---|
| **Passage** | **Fractions** | **Total Cells** | **Avg. Viability (%)** |
| Inoculation | 4 | $2.85 \times 10^7$ | $99.7 \pm 0.19$ |
| 1 | 8 | $9.34 \times 10^7$ | $99.2 \pm 2.65$ |
| 2 | 4 | $8.80 \times 10^7$ | $94.4 \pm 1.92$ |

*Cell Kinetics.* Table 11-2 shows the growth kinetics from UTC cell line 050604B expanded from passage nine to passage eleven on Cytodex® 3 microcarriers in spinner flask cultures. The table shows that the total doublings was 7.48, and the average hours per doubling was 69.53 ($\pm$ 17.52) hours.

| Table 11-2: Cell Kinetic | | | | | | |
|---|---|---|---|---|---|---|
| **Passage** | **Seeded** | **Yield** | **Days** | **Expansion** | **Doubling** | **Hours/Doubling** |
| | | $2.00 \times 10^6$ | 0 | 1 | | |
| Inoculation | $2.00 \times 10^6$ | $2.85 \times 10^7$ | 8 | 14.3 | 3.83 | 50.09 |
| 1 | $2.85 \times 10^7$ | $9.34 \times 10^7$ | 6 | 3.28 | 1.71 | 84.12 |
| 2 | $2.30 \times 10^7$ | $8.80 \times 10^7$ | 6 | 3.83 | 1.94 | 74.39 |

*Live/Dead Staining.* Analysis of the live/dead stained microcarrier aliquot shows the majority of the microcarrier surfaces covered with green stained (viable) cells with scant foci of red stained nuclei (dead). The cells exhibit morphology similar to the morphology of the cells cultured in static conditions.

*Flow Cytometry Analysis.* Table 11-3 shows the results ("+ positive" or "-negative") for cell surface markers expressed by human Umbilical Tissue-derived Cells (hUTCs) harvested microcarrier beads in spinner flasks versus hUTCs harvested from culture in static T flasks. The table shows that the markers expressed by the cells produced by the two methods were consistent.

| Table 11-3: Comparison of cell surface proteins expression by Umb 050604B cells expanded in static T flasks or on Cytodex® 3 microcarriers in spinner flask systems and analyzed by flow cytometry. | | |
|---|---|---|
| **Cell Surface Marker** | **Static T Flasks** | **Cytodex 3® Microcarriers** |
| CD 10 | (+) | (+) |
| CD 13 | (+) | (+) |
| CD 31 | (-) | (-) |
| CD 34 | (-) | (-) |
| CD 44 | (+) | (+) |
| CD 45 | (-) | (-) |
| CD 73 | (+) | (+) |
| CD 90 | (+) | (+) |
| CD 117 | (-) | (-) |
| CD 141 | (-) | (-) |
| PDGFr-$\alpha$ | (+) | (+) |
| HLA-A,B,C | (+) | (+) |

(continued)

| Table 11-3: Comparison of cell surface proteins expression by Umb 050604B cells expanded in static T flasks or on Cytodex® 3 microcarriers in spinner flask systems and analyzed by flow cytometry. | | |
|---|---|---|
| Cell Surface Marker | Static T Flasks | Cytodex 3® Microcarriers |
| HLA-DR,DP,DQ | (-) | (-) |

*Conclusion:* Human Umbilical Tissue-derived Cells (hUTCs) were cultured on Cytodex® 3 microcarriers in impeller spinner flask bioreactors. The cells achieved 7.48 population doublings over twenty days and had an average population doubling time of 69 hours. Cell viability per passage ranged from 94.4% to 99.7%. Analysis for expression of thirteen cell surface markers on hUTCs cultured on microcarriers was consistent with the cell surface marker expression by hUTCs cultured in cell culture T flasks. This example indicates that microcarriers can be used to seed, expand, and harvest hUTCs in bioreactor systems.

## EXAMPLE 12

### Growth of Expanded hUTCs on collagen coated MGSA and PLGA Microcarriers in Spinner Flasks

The ability of hUTCs to attach to materials made of synthetic resorbable biomaterials with a collagen coating was investigated, including the ability to maintain viability in spinner flask culture and to proliferate upon re-seeding into static culture. Expanded hUTCs were seeded onto collagen-coated or uncoated poly-(D,L-lactide-co-glycolide) (PLGA) and poly(monostearoylglyceride co-succinic acid) (MGSA) microcarriers. The microcarriers with cells were cultured in spinner flasks for five days, harvested by trypsinization, and re-seeded into static cultures.

Materials and Methods

| Table 12-1 Microcarriers | | | |
|---|---|---|---|
| Microcarrier | Manufacturer | Process Method | Avg. Size ($\mu$m) |
| PLGA (50/50) IV 0.43 | Alkermes (Willington, OH) | SCF | 158 |
| MGSA I | Ethicon (Somerville, NJ) | SCF | 195 |

*Microcarrier Preparation.* Microcarrier Wetting- Approximately 1g each of MGSA and PLGA microcarriers were aseptically suspended in 25 ml 70% ethanol for 30 minutes to wet the microcarriers. The ethanol was removed by aspiration and the microcarriers were then rinsed three times with PBS and re-suspended 25 ml of in Dulbecco's phosphate buffered saline (PBS).

*Collagen coating.* Wetted microcarriers (PBS) were pelletized by centrifugation, the PBS removed by aspiration, and the microcarriers were re-suspended in a 2.9% collagen solution (Vitrogen 1000, Cohesion, Inc. Palo Alto, CA). The microcarriers were incubated in collagen for 30 minutes. The residual collagen was removed by aspiration and the collagen coated microparticles were washed three times with PBS.

| Table 12-2 Microcarrier Quantities Used. | | |
|---|---|---|
| Microcarrier | Milligrams | No. cells Seeded |
| PLGA (50/50) uncoated | 260 | $3.50 \times 10^6$ |
| PLGA (50/50) coated | 260 | $3.50 \times 10^6$ |
| MGSA I uncoated | 330 | $3.50 \times 10^6$ |
| MGSA I coated | 330 | $3.50 \times 10^6$ |

*Inoculation and Culture* The materials, cell type, growth media, spinner flask, inoculation and culture conditions, media exchange, viability staining and cell harvest methods used in Example 11 were used in this example.

**Results**

| Table 12-3 Cell Harvest | | |
|---|---|---|
| **Microcarrier** | **Total Cells** | **Viability (%)** |
| PLGA (50/50) uncoated | $1.20 \times 10^6$ | 98.6 |
| PLGA (50/50) coated | $1.15 \times 10^6$ | 97.6 |
| MGSA I uncoated | $1.82 \times 10^6$ | 99.0 |
| MGSA I coated | $2.39 \times 10^6$ | 97.8 |

*Harvest Cell Re-Seed.* Cells harvested from the coated and uncoated MGSA and PLGA microcarriers were re-seeded into T225 at approximately 5,000 cells/cm$^2$. Four days after re-seeding the cells harvested from both materials proliferated to over 50% confluence.

Expanded hUTCs were seeded onto collagen coated PLGA and MGSA microcarrier, cultured in spinner flasks for five days, harvested by trypsinization, and re-seeded into static cultures. The cells that were harvested from the synthetic microcarriers were over 90% viable. The re-seed cells expanded in static culture within four days demonstrating retention of their proliferative capacity. This example demonstrates the ability of synthetic biomaterials to be used as microcarriers for spinner flasks culture.

**Example 13**

**Expansion of hUTC on Microcarriers in Continuous Spinner Flask Culture**

The goal of this study was to continuously culture expanded hUTC adherent to commercial microcarriers in spinner flasks over multiple population doublings. The ability to expand hUTC on microcarriers over multiple population doublings will serve a model system to be scale-up for large-scale production of hUTC for cell therapy applications. Two hUTC isolates, 120304- isolated, expanded and cryopreserved under research conditions, and CNTO 2476- isolated, expanded and cryopreserved under GMP conditions, were evaluated. The commercial microcarriers Cytodex® 1 or Hillex® II were evaluated also. The cryopreserved cells were thawed and used to immediately inoculate spinner flask cultures. The cells were continuously cultured over multiple passages until the cell reached approximately population-doubling 30. hUTCs were also cultured statically in T225 flasks as a control.

hUTC isolate 120304 cryopreserved at population doubling 12.8 was able to be thawed, and expanded on Cytodex® 1 and Hillex® II microcarriers to population doubling 28.6 and 28.7, respectively. The hours per population doubling was consistent from passage to passage, indicating stable logarithmic growth and was consistent with the T flask growth kinetics. hUTC isolate CNTO 2476 cryopreserved at population doubling 22.6 was able to be thawed, and expanded on Cytodex® 1 and Hillex® II microcarriers to population doubling 33.2 and 31.0 respectively. The hours per population doubling was consistent from passage to passage, indicating stable logarithmic growth and was also consistent with the T flask growth kinetics. Statistical analysis by one-way ANOA of all hours per population doubling data points show no significant difference the hUTC growth kinetics for all conditions tested. (p=0.988). The cell surface protein expression remained consistent at final harvest for all conditions tested.

This example demonstrate the ability of hUTC to be expanded to approximately 30 population doublings on microcarriers in a stable, consistent manner that maintains the cell's surface protein phenotype.

**Materials and Methods**

*Cells.* Cryopreserved expanded hUTC isolate 12034 population doubling (PD) 12 and hUTC isolate CNTO 2476 lot 25126078 PD 22 were used.

*Growth Media.* Dulbecco's Modified Eagles Media (DMEM)-low glucose (Gibco- Grand Island, NY), 15% FBS (HyClone- Logan UT), penicillin/streptomycin (P/S) (Gibco- Grand Island, NY), Betamercaptoethanol (BME) (Sigma- St. Louis, MO)

Microcarriers. Cytodex® 1 (GE Health Sciences- Piscataway, NJ) microcarriers were hydrated in PBS for at least 3 hours and autoclaved. Cytodex® 1 microcarriers were used at a concentration of 3 g/L. Hillex® II (SoloHill Engineering, Inc., Ann Arbor, MI) microcarriers were hydrated in deionized water for at least 30 minutes autoclaved. Hillex® II microcarriers were used at a concentration of 12 g/L.

*Spinner Flask.* 100 ml and 500 ml single-use, disposable spinner flasks (Corning, Inc.- Corning, NY) were used.

Inoculation and Culture in 100 ml Spinner Flask. Cryopreserved vials of hUTC were thawed, washed and resuspended in growth media. $6.6 \times 10^6$ hUTC were added to 3000mg of Cytodex® 1 ($5.0 \times 10^3$cells per cm$^2$) in a 100 ml spinner flask containing 100 ml media and placed on a37°C tissue culture incubators and incubated for three to four days. Spinner plate was set to 60-rpm continuous rotation. $3.1 \times 10^6$ hUTC were added to 1.2 g of Hillex® II ($5.0 \times 10^3$ cells per cm$^2$) in a 100 ml spinner flask containing 100 ml media and placed on a spinner plate set to 60-rpm continuous rotation. Spinner plates placed in 5% $CO_2$.

*Passage of Culture From One 100 ml Spinner Flask to One 500 ml Spinner Flask.* 100 ml spinner flask was removed from spinner plate and the microcarriers were allowed to settle. The media supernatant is removed by aspiration. The remaining microcarrier pack with adherent cells was resuspended in 20 ml fresh growth media. The microcarriers with adherent cells were then aseptically transferred by pipette to a 500 ml spinner flask containing 480ml fresh growth media and 4.8 g Hillex® II (6g final microcarrier content) or 1.2 g Cytodex® 1 (1.5g final microcarrier content). The spinner flask was then placed on a spinner plate set to 60-rpm continuous rotation. Spinner plates placed in 5% $CO_2$, 37°C tissue culture incubators and incubated for three to four days.

*Passage of culture from one 500 ml spinner flask to five 500 ml spinner flasks.* 500 ml spinner flask was removed from spinner plate and the microcarriers were allowed to settle. The media supernatant is removed by aspiration. The remaining microcarrier pack with adherent cells was resuspended in 50 ml fresh growth media. Five separate 10 ml aliquots of the microcarriers with adherent cells were then aseptically transferred by pipette to five separate 500 ml spinner flasks each containing 490 ml fresh growth media and 4.8g Hillex® II (6g final microcarrier content) or 1.2 g Cytodex® 1 (1.5g final microcarrier content). The spinner flasks were then placed on a spinner plate set to 60-rpm continuous rotation. Spinner plates placed in 5% $CO_2$, 37°C tissue culture incubators and incubated for three to four days.

*Harvest of cells adherent to Cytodex® 1 microcarriers.* The 500 ml spinner flask was removed from spinner plate and the microcarriers with adherent cells were allowed to settle by gravity. The media supernatant was removed by aspiration. 500 ml of PBS was added to the spinner flask and the microcarriers were allowed to settle by gravity. The PBS supernatant was removed by aspiration. 500 ml of DMEM- low glucose was added to the spinner flask. The spinner flask was then incubated on spinner plate for 20 minutes at 60 rpm. The spinner flask was removed from spinner plate and the microcarriers were allowed to settle by gravity. The DMEM-low glucose supernatant was removed by aspiration. 500 ml of PBS was added to the spinner flask. The spinner flask was then incubated on spinner plate for 20 minutes at 60 rpm. The spinner flask was removed from spinner plate and the microcarriers were allowed to settle by gravity. The PBS supernatant was removed by aspiration. 250 ml TrypLE select was added to the spinner flask. The spinner flask was then incubated on spinner plate for 10 minutes at 60 rpm. The spinner flask was removed from spinner plate and the microcarriers were allowed to settle by gravity. Using a 50 ml serological pipette, the microcarriers-TrypLE™ select solution was agitated by pipetting up and down ~10 times to dissociate residual adherent cells from the microcarriers. 250 ml of PBS was then added to the spinner flask the microcarriers were allowed to settle by gravity. The cell containing supernatant is collect by repeated pipetting and transfer to multiple conical tubes pre-loaded with 5ml FBS and a 100$\mu$m filter unit inserted in the tube opening. The tubes were centrifuged for 5 minutes at 300rcf, the supernatant decanted, and the cells re-suspended in growth media.

*Harvest of cells adherent to Hillex® II microcarriers.* The 500 ml spinner flask was removed from spinner plate and the microcarriers with adherent cells were allowed to settle by gravity. The media supernatant was removed by aspiration. 500 ml of PBS was added to the spinner flask and the microcarriers were allowed to settle by gravity. 100 ml TrypLE™ select was added to the spinner flask. The spinner flask was then incubated on spinner plate for 10 minutes at 60 rpm. The spinner flask was removed from spinner plate and the microcarriers were allowed to settle by gravity. Using a 25 ml serological pipette, the microcarriers-TrypLE™ select solution was agitated by pipetting up and down ~10 times to dissociate residual adherent cells from the microcarriers. The cell containing supernatant is collect by repeated pipetting and transfer to multiple conical tubes pre-loaded with 5ml FBS and a 100-$\mu$m filter unit inserted in the tube opening. The tubes were centrifuged for 5 minutes at 300rcf, the supernatant decanted, and the cells re-suspended in growth media.

*Viability Staining.* A 1 ml aliquot of media and microcarriers were transferred to a 15 ml conical tube and the microcarriers were allowed to separate by gravity. Media was removed by aspiration and replaced with 1ml Live/Dead staining solution (Molecular Probes cat. no. L3224) and incubated from 15 minutes at 37° C. After incubation, a 20-$\mu$l aliquot was applied to a glass microscope slide and observed by fluorescent microscopy. Live cells stain green. Microscopic fields were manually analyzed to evaluate the distribution of viable cells adhered to the microcarriers. At least three microscopic fields were evaluated and the approximate percentage of viable cells was counted.

*In culture cell counts- Nuclei release assay.* A 5 ml (100 ml spinner flask) or 10 ml (500 ml spinner flask) aliquot of homogenous microcarrier suspension was obtained from spinner flask vessel and transferred to a 15 ml tube. The microcarriers were allowed to gravity separate and the supernatant was removed by aspiration. The microcarriers were washed once with 10 ml PBS, the microcarriers allowed to gravity separate, and the PBS supernatant removed by aspiration. The microcarriers were incubated for one hour at 37°C in nuclei release solution (0.1M citric acid (Sigma- St. Louis, MO) containing 0.1% w/v crystal violet (Sigma- St. Louis, MO)). After incubation, a 100 µl aliquot of the microcarrier containing nuclei release solution was added to 100 µl PBS. A 10 µl aliquot of this solution was then loaded into a hemocytometer and released nuclei counted.

*In culture cell counts- TrypLE™ assay.* A 5ml (100 ml spinner flask) or 10 ml (500 ml spinner flask) aliquot of homogenous microcarrier suspension was obtained from spinner flask vessel and transferred to a 15 ml tube. The microcarriers were allowed to gravity separate and the supernatant was removed by aspiration. The microcarriers were washed once with 10 ml PBS, the microcarriers allowed to gravity separate, and the PBS supernatant removed by aspiration. The microcarriers were incubated for ten minutes at 37°C in TrypLE™ select. After incubation, 5 ml of PBS is added and the microcarriers are allowed to gravity separate. The cell containing supernatant is collect by repeated pipetting and transfer to multiple conical tubes pre-loaded with 1 ml FBS. The tubes were centrifuged for 5 minutes at 300 rcf, the supernatant decanted, the cells re-suspended in growth media, and an aliquot is used determine cell count using a Guava PCA instrument (Guava Technologies, Haywood, CA).

*Static T-Flask Culture.* Cryopreserved vials of hUTC were thawed, washed and resuspended in growth media. The cells were cultured statically in T225 over multiple passages using methods stated in US2004877012A.

*Flow Cytometry.* Harvested hUTC were analyzed by flow cytometry using a Becton-Dickinson FACSCalibur™ instrument (Becton Dickinson, San Jose, CA) to determine the cell surface marker profile using methods stated in US2004877012A. All antibodies purchased from BD PharMingen (San Diego, CA).

## Results

| Table 13-1: Continuous culture of hUTC isolate 120304 on Cytodex® 1 microcarriers. | | | | | | |
|---|---|---|---|---|---|---|
| **Passage** | **Seeded** | **Yield** | **Doubling** | **Total Doublings** | **Time (days)** | **Hours/doub** |
| 6 (seed) | | 5.32E+06 | 2.03E+00 | 1.28E+01 | | |
| 6 | 5.32E+06 | 4.71E+07 | 3.15E+00 | 1.59E+01 | 4.00 | 30.51 |
| 7 | 8.96E+06 | 7.30E+07 | 3.03E+00 | 1.89E+01 | 3.00 | 23.79 |
| 8 | 6.60E+06 | 3.30E+07 | 2.32E+00 | 2.12E+01 | 3.00 | 31.01 |
| 9 | 6.60E+06 | 3.90E+07 | 2.56E+00 | 2.38E+01 | 3.00 | 28.09 |
| 10 | 3.90E+07 | 2.17E+08 | 2.48E+00 | 2.63E+01 | 4.00 | 38.77 |
| 11 | 2.17E+08 | 1.10E+09 | 2.34E+00 | 2.86E+01 | 4.00 | 41.00 |

| Table 13-2: Continuous culture of hUTC isolate 120304 on Hillex® II microcarriers. | | | | | | |
|---|---|---|---|---|---|---|
| **Passage** | **Seeded** | **Yield** | **Doubling** | **Total Doublings** | **time (days)** | **Hours/doubling** |
| 6 (seed) | | 5.32E+06 | 2.03E+00 | 1.28E+01 | | |
| 6 | 5.32E+06 | 4.71E+07 | 3.15E+00 | 1.59E+01 | 4.00 | 30.51 |
| 7 | 8.96E+06 | 7.30E+07 | 3.03E+00 | 1.89E+01 | 3.00 | 23.79 |
| 8 | 3.00E+06 | 1.90E+07 | 2.66E+00 | 2.16E+01 | 3.00 | 27.04 |
| 9 | 3.80E+06 | 2.30E+07 | 2.60E+00 | 2.42E+01 | 3.00 | 27.72 |
| 10 | 2.30E+07 | 2.64E+08 | 3.52E+00 | 2.77E+01 | 4.00 | 27.27 |
| 11 | 2.11E+08 | 4.16E+08 | 9.79E-01 | 2.87E+01 | 3.00 | 73.52 |

| Table 13-3. Continuous culture of hUTC isolate CNTO 2476 on Cytodex® 1 microcarriers | | | | | | |
|---|---|---|---|---|---|---|
| **Passage** | **Seeded** | **Yield** | **doubling** | **Total Doublings** | **time (days)** | **Hours/doubling** |
| 6 (seed) | | 6.30E+05 | | 2.26E+01 | | |

(continued)

| Table 13-3. Continuous culture of hUTC isolate CNTO 2476 on Cytodex® 1 microcarriers | | | | | | |
|---|---|---|---|---|---|---|
| Passage | Seeded | Yield | doubling | Total Doublings | time (days) | Hours/doubling |
| 6 | 6.30E+05 | 3.44E+06 | 2.45E+00 | 2.50E+01 | 3.00 | 29.40 |
| 7 | 3.44E+06 | 5.20E+07 | 3.92E+00 | 2.90E+01 | 4.00 | 24.50 |
| 8 | 4.00E+07 | 1.60E+08 | 2.00E+00 | 3.10E+01 | 3.00 | 36.00 |
| 8 | 1.60E+08 | 7.67E+08 | 2.26E+00 | 3.32E+01 | 4.00 | 42.46 |

| Table 13-4. Continuous culture of hUTC isolate CNTO 2476 on Hillex® II microcarriers | | | | | | |
|---|---|---|---|---|---|---|
| Passage | Seeded | Yield | doubling | Total Doublings | time (days) | hours/doubling |
| 6 (seed) | | 1.68E+06 | | 2.26E+01 | | |
| 6 | 1.68E+06 | 1.29E+07 | 2.94E+00 | 2.55E+01 | 4.00 | 32.64 |
| 7 | 1.29E+07 | 5.30E+07 | 2.04E+00 | 2.76E+01 | 3.00 | 35.32 |
| 8 | 5.30E+07 | 5.60E+08 | 3.40E+00 | 3.10E+01 | 5.00 | 35.28 |

| Table 13-5: Continuous culture of hUTC isolate 120304 in T225 flasks. | | | | | | |
|---|---|---|---|---|---|---|
| Passage | Seeded | Yield | doubling | Total Doublings | time (days) | hours/doubling |
| 6 (seed) | | 1.12E+07 | 2.03E+00 | 1.28E+01 | | |
| 6 | 1.12E+07 | 3.05E+07 | 1.45E+00 | 1.42E+01 | 2.00 | 33.21 |
| 7 | 2.20E+06 | 2.03E+07 | 3.21E+00 | 1.74E+01 | 4.00 | 29.94 |
| 8 | 3.75E+05 | 1.50E+06 | 2.00E+00 | 1.94E+01 | 3.00 | 36.00 |
| 9 | 3.75E+05 | 1.85E+06 | 2.30E+00 | 2.17E+01 | 4.00 | 41.69 |
| 10 | 3.75E+05 | 2.39E+06 | 2.67E+00 | 2.44E+01 | 3.00 | 26.95 |
| 11 | 7.50E+05 | 3.14E+06 | 2.07E+00 | 2.64E+01 | 4.00 | 46.47 |
| 12 | 3.14E+06 | 2.02E+07 | 2.69E+00 | 2.91E+01 | 3.00 | 26.81 |
| 13 | 2.02E+07 | 1.14E+08 | 2.50E+00 | 3.16E+01 | 4.00 | 38.45 |

| Table 13-6 Comparison of cell surface proteins expression by hUTC expanded on microcarrier and T-flasks and analyzed by flow cytometry. | | | | | |
|---|---|---|---|---|---|
| Cell Surface Marker | 120304 T225 flask | 120304 Cytodex® 1 | 120304 Hillex® II | CNTO 2476 Cytodex® 1 | CNTO 2476 Hillex® II |
| CD 10 | (+) | (+) | (+) | (+) | (+) |
| CD 13 | (+) | (+) | (+) | (+) | (+) |
| CD 31 | (-) | (-) | (-) | (-) | (-) |
| CD 34 | (-) | (-) | (-) | (-) | (-) |
| CD 44 | (+) | (+) | (+) | (+) | (+) |
| CD 45 | (-) | (-) | (-) | (-) | (-) |
| CD 73 | (+) | (+) | (+) | (+) | (+) |

(continued)

| Table 13-6 Comparison of cell surface proteins expression by hUTC expanded on microcarrier and T-flasks and analyzed by flow cytometry. | | | | | |
|---|---|---|---|---|---|
| Cell Surface Marker | 120304 T225 flask | 120304 Cytodex® 1 | 120304 Hillex® II | CNTO 2476 Cytodex® 1 | CNTO 2476 Hillex® II |
| CD 90 | (+) | (+) | (+) | (+) | (+) |
| CD 117 | (-) | (-) | (-) | (-) | (-) |
| CD 141 | (-) | (-) | (-) | (-) | (-) |
| PDGFr-a | (+) | (+) | (+) | (+) | (+) |
| HLA-ABC | (+) | (+) | (+) | (+) | (+) |
| HLA-DRDPDQ | (-) | (-) | (-) | (-) | (-) |

hUTC isolate 120304 cryopreserved at population doubling 12.8 was able to be thawed, and expanded on Cytodex® 1 and Hillex® II microcarriers to population doubling 28.6 and 28.7, respectively. The hours per population doubling was consistent from passage to passage, indicating stable logarithmic growth and was consistent with the T flask growth kinetics. hUTC isolate CNTO 2476 cryopreserved at population doubling 22.6 was able to be thawed, and expanded on Cytodex® 1 and Hillex® II microcarriers to population doubling 33.2 and 31.0 respectively. The hours per population doubling was consistent from passage to passage, indicating stable logarithmic growth and was also consistent with the T flask growth kinetics. Statistical analysis by one-way ANOA of all hours per population doubling data points show no significant difference the hUTC growth kinetics for all conditions tested. (p=0.988). In addition, the cell surface protein expression remained consistent at final harvest for all conditions tested. This data demonstrated the ability of hUTC to be expanded to approximately 30 population doublings on microcarriers in a stable, consistent manner that maintains the cell's surface protein phenotype.

While the invention has been described and illustrated herein by references to various specific materials, procedures and examples, it is understood that the invention is not restricted to the particular combinations of material and procedures selected for that purpose. Numerous variations of such details can be implied as will be appreciated by those skilled in the art. It is intended that the specification and examples be considered as exemplary, only, with the true scope and spirit of the invention being indicated by the following claims. All references, patents, and patent applications referred to in this application are herein incorporated by reference in their entirety.

The invention provides the following numbered embodiments:

1. A culture medium for growing anchorage-dependent cells comprising:

the amino acids L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Aspargine; L-Aspartic Acid; L-Glutamic Acid; L-Proline; and L-Taurine;

the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);

the salts calcium chloride, potassium chloride, magnesium sulfate, sodium chloride, and one or more sodium phosphate salts;

the nucleosides thymidine, adenosine, cytidine, uridine and guanosine;

insulin; transferrin; lipoic acid/thioctic acid; ethanolamine; sodium selenite; and one or more energy sources.

2. The culture medium of embodiment 1, wherein the culture medium comprises:

at least about 0.05 g/L of L-Arginine; at least about 0.02 g/L of L-Cystine; at least about 0.2 g/L of L-Glutamine; at least about 0.01 g/L Glycine; at least about 0.02 g/L of L-Histidine; at least about 0.09 g/L of L-Isoleucine; at least about 0.09 g/L of L-Leucine; at least about 0.09 g/L of L-Lysine; at least about 0.02 g/L of L-Methionine; at least about 0.05 g/L of L-Phenylalanine; at least about 0.03 g/L of L-serine; at least about 0.08 g/L of L-Threonine; at least about 0.009 g/L of L-tryptophan; at least about 0.08 g/L of L-tyrosine; at least about 0.08 g/L of L-Valine; at least about 0.005 g/L L-Cysteine; at least about 0.0004 g/L of L-Alanine; at least about 0.01 g/L of L-Aspargine; at least about 0.006 g/L of L-Aspartic Acid; at least 0.03 g/L of L-Glutamic Acid; at least about 0.005 g/L L-Proline; and at least about 0.0003 g/L of L-Taurine;

from about $5 \times 10^{-6}$ g/L to about 0.015 g/L of each of the vitamins;

at least about 0.05 g/L of Calcium Chloride, Anhydrous, at least about 0.1 g/L of potassium chloride; at least about 0.2 g/L of magnesium sulfate, at least about 0.08 g/L of sodium phosphate, monobasic, $H_2O$ and at least about 0.0005 g/L of sodium phosphate, dibasic heptahydrate ($Na_2HPO_4.7H_2O$);

at least about 0.0001 g/L of thymidine and least about 0.005 g/L of each of adenosine, cytidine, uridine, and guanosine; and

at least 0.003 g/L of insulin, at least 0.05 g/L of transferrin, at least about $5 \times 10^{-6}$ g/L of lipoic acid/thioctic acid, at least 0.05 g/L of ethanolamine and at least about 0.00004 g/L of sodium selenite.

3. A serum-free nutrient solution for growing anchorage dependent cells comprising:

the amino acids L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Aspargine, L-Aspartic Acid, L-Glutamic Acid, L-Proline and L-Taurine;

the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);

the salts sodium phosphate, monobasic and sodium phosphate, dibasic heptahydrate;

the nucleosides adenosine, cytidine, uridine and guanosine; and

insulin; transferrin; lipoic acid/thioctic acid; ethanolamine; and sodium selenite.

4. A kit for growing anchorage dependent cells comprising the culture medium of embodiment 1.

5. The kit of embodiment 4, further comprising a serum-free nutrient solution, wherein the serum-free nutrient solution comprises:

the amino acids L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Aspargine, L-Aspartic Acid, L-Glutamic Acid, L-Proline and L-Taurine;

the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);

the salts sodium phosphate, monobasic and sodium phosphate, dibasic heptahydrate;

the nucleosides adenosine, cytidine, uridine and guanosine; and

insulin; transferrin; lipoic acid/thioctic acid; ethanolamine; and sodium selenite.

6. A method of culturing isolated umbilical cord tissue-derived cells comprising:

a. growing umbilical cord tissue-derived cells seeded on microcarriers in a culture medium comprising amino acids, vitamins, salts, nucleosides, lipoic/thioctic acid, ethanolamine, insulin, transferrin, sodium selenium, wherein the culture medium is supplemented with serum, for a sufficient period of time to allow for the cells to achieve a desired initial population density;

b. adding a serum-free nutrient solution after the cells have achieved the desired initial population density, wherein the serum-free nutrient solution comprises amino acids, vitamins, salts, insulin, transferrin, ethanolamine, lipoic acid/thioctic acid, sodium selenium; and

c. growing the cells for a sufficient period of time to allow for the cells to achieve a desired final population density;

wherein the umbilical cord tissue-derived cells are isolated from human umbilical cord tissue substantially free of blood, are capable of self-renewal and expansion in culture, have the potential to differentiate, express CD13, CD90, HLA-ABC, and do not express CD34, CD117 and HLA-DR.

7. The method of embodiment 6 wherein the method further comprises seeding the cells on the microcarriers.

8. The method of embodiment 6, wherein the method further comprises isolating the cells after step c.

9. The method of embodiment 6, wherein the method does not require medium exchange.

10. The method of embodiment 6, wherein the method is carried out in a spinner flask culture system.

11. The method of embodiment 6, wherein the characteristics of the cells before and after culturing are substantially the same.

12. The method of embodiment 11, wherein the characteristics of the cells before and after culturing are the same.

13. The method of embodiment 6, wherein the desired initial population density is achieved after 3 to 4 days.

14. The method of embodiment 6, wherein the microcarriers have an amine treated surface.

15. The method of embodiment 6, wherein the culture medium comprises:

the amino acids L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Aspargine; L-Aspartic Acid; L-Glutamic Acid; L-Proline; and L-Taurine;

the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);

the salts calcium chloride, potassium chloride, magnesium sulfate, sodium chloride, and one or more sodium phosphate salts;

the nucleosides thymidine, adenosine, cytidine, uridine and guanosine; and

insulin; transferrin; lipoic acid/thioctic acid; ethanolamine; sodium selenite; and one or more energy sources.

16. The method of embodiment 15, wherein the one or more energy sources are D-glucose and sodium pyruvate.

17. The method of embodiment 15, wherein the culture medium comprises:

at least about 0.05 g/L of L-Arginine; at least about 0.02 g/L of L-Cystine; at least about 0.2 g/L of L-Glutamine; at least about 0.01 g/L Glycine; at least about 0.02 g/L of L-Histidine; at least about 0.09 g/L of L-Isoleucine; at least about 0.09 g/L of L-Leucine; at least about 0.09 g/L of L-Lysine; at least about 0.02 g/L of L-Methionine; at least about 0.05 g/L of L-Phenylalanine; at least about 0.03 g/L of L-serine; at least about 0.08 g/L of L-Threonine; at least about 0.009 g/L of L-tryptophan; at least about 0.08 g/L of L-tyrosine; at least about 0.08 g/L of L-Valine; at least about 0.005 g/L L-Cysteine; at least about 0.0004 g/L of L-Alanine; at least about 0.01 g/L of L-Aspargine; at least about 0.006 g/L of L-Aspartic Acid; at least 0.03 g/L of L-Glutamic Acid; at least about 0.005 g/L L-Proline; and at least about 0.0003 g/L of L-Taurine;

from about $5 \times 10^{-6}$ g/L to about 0.015 g/L of each of the vitamins;

at least about 0.05 g/L of Calcium Chloride, Anhydrous, at least about 0.1 g/L of potassium chloride; at least about 0.2 g/L of magnesium sulfate, at least about 0.08 g/L of sodium phosphate, monobasic, $H_2O$ and at least about 0.0005 g/L of sodium phosphate, dibasic heptahydrate ($Na_2HPO_4.7H_2O$);

at least about 0.0001 g/L of thymidine and least about 0.005 g/L of each of adenosine, cytidine, uridine, and guanosine; and

at least 0.003 g/L of insulin, at least 0.05 g/L of transferrin, at least about $5 \times 10^{-6}$ g/L of lipoic acid/thioctic acid, at least 0.05 g/L of ethanolamine and at least about 0.00004 g/L of sodium selenite.

18. The method of embodiment 15, wherein the culture medium is supplemented with 2 to 20% of FBS.

19. The method of embodiment 15, wherein the culture medium is supplemented with about 7.5%, about 10%, or about 15% of FBS.

20. The method of embodiment 15, wherein the culture medium further comprises putrescine, a stabilizer, and/or a foaming agent.

21. The method of embodiment 6, wherein in the serum-free nutrient solution comprises:

the amino acids L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Aspargine, L-Aspartic Acid, L-Glutamic Acid, L-Proline and L-Taurine;

the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);

the salts sodium phosphate, monobasic and sodium phosphate, dibasic heptahydrate;

the trace minerals Copper(II)sulfate pentahydrate ($CuSO_4.5H_2O$), Zinc sulfate, heptahydrate, ($ZnSO_4.7H_2O$);

the nucleosides adenosine, cytidine, uridine and guanosine; and

insulin; transferrin; ethanolamine; lipoic acid/thioctic acid; and sodium selenite.

22. The method of embodiment 21, wherein the serum-free solution further comprises putrescine, a stabilizer, and/or a foaming agent.

**Claims**

1. A culture medium for growing anchorage-dependent cells comprising:

the amino acids L-Arginine; L-Cystine; L-Cysteine; L-Glutamine; Glycine; L-Histidine; L-Isoleucine; L-Leucine; L-Lysine; L-Methionine; L-Phenylalanine; L-serine; L-Threonine; L-tryptophan; L-tyrosine; L-Valine; L-Alanine; L-Asparagine; L-Aspartic Acid; L-Glutamic Acid; L-Proline; and L-Taurine;
the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);
the salts calcium chloride, potassium chloride, magnesium sulfate, sodium chloride, and one or more sodium phosphate salts;
the nucleosides thymidine, adenosine, cytidine, uridine and guanosine;
insulin; transferrin; lipoic acid/thioctic acid; ethanolamine; sodium selenite; and one or more energy sources, wherein the anchorage-dependent cells are umbilical cord tissue-derived cells, mesenchymal stem cells, bone marrow derived stem cells, cells derived from placental tissue, adherent cells derived from non-marrow tissues, cells derived from the dental pulp of teeth, cells derived from amniotic fluid and fibroblasts including neonatal foreskin fibroblasts.

2. The culture medium of claim 1, wherein the anchorage-dependent cells are umbilical cord tissue-derived cells, and wherein the umbilical cord tissue-derived cells are isolated from human umbilical cord tissue substantially free of blood, are capable of self-renewal and expansion in culture, have the potential to differentiate, lack the production of CD117 or CD45, and do not express hTERT or telomerase.

3. The culture medium of claims 1 or 2, wherein the medium comprises one or more of:

from 0.0006 g/L to 0.1 g/L of each of the amino acids;
from $5 \times 10^{-6}$ g/L to 0.015 g/L of each of the vitamins; and
from at least 0.0001 g/L to at least 0.02 g/L of the each of the nucleosides.

4. The culture medium of claims 1-3, wherein the medium further comprises one or more additional trace minerals.

5. The culture medium of claim 4, wherein the medium comprises from $5 \times 10^{-8}$ g/L to $3 \times 10^{-4}$ g/L of each of the trace minerals.

6. The culture medium of claims 4 or 5, wherein the trace minerals are ferric nitrate, copper sulfate, and zinc sulfate.

7. The culture medium of any one of claims 1-6, wherein the medium further comprises putrescine, a stabilizer, and/or a foaming agent.

8. The culture medium of any one of claims 1-7, wherein the energy substrate is glucose or sodium pyruvate.

9. The culture medium of claim 8, wherein the medium comprises at least 0.8 g/L of D-glucose and at least 0.1 g/L of sodium pyruvate.

10. The culture medium of claim 1, wherein the culture medium comprises:

at least 0.05 g/L of L-Arginine; at least 0.02 g/L of L-Cystine; at least 0.2 g/L of L-Glutamine; at least 0.01 g/L Glycine; at least 0.02 g/L of L-Histidine; at least 0.09 g/L of L-Isoleucine; at least 0.09 g/L of L-Leucine; at least 0.09 g/L of L-Lysine; at least 0.02 g/L of L-Methionine; at least 0.05 g/L of L-Phenylalanine; at least 0.03 g/L of L-serine; at least 0.08 g/L of L-Threonine; at least 0.009 g/L of L-tryptophan; at least 0.08 g/L of L-tyrosine; at least 0.08 g/L of L-Valine; at least 0.005 g/L L-Cysteine; at least 0.0004 g/L of L-Alanine; at least 0.01 g/L of L-Asparagine; at least 0.006 g/L of L-Aspartic Acid; at least 0.03 g/L of L-Glutamic Acid; at least 0.005 g/L L-Proline; and at least 0.0003 g/L of L-Taurine;
from $5 \times 10^{-6}$ g/L to 0.015 g/L of each of the vitamins;
at least 0.05 g/L of Calcium Chloride, Anhydrous, at least 0.1 g/L of potassium chloride; at least 0.2 g/L of magnesium sulfate, at least 0.08 g/L of sodium phosphate, monobasic, $H_2O$ and at least 0.0005 g/L of sodium phosphate, dibasic heptahydrate ($Na_2HPO_4.7H_2O$);
at least 0.0001 g/L of thymidine and least 0.005 g/L of each of adenosine, cytidine, uridine, and guanosine; and
at least 0.003 g/L of insulin, at least 0.05 g/L of transferrin, at least $5 \times 10^{-6}$ g/L of lipoic acid/thioctic acid, at least 0.05 g/L of ethanolamine and at least 0.00004 g/L of sodium selenite.

11. The culture medium of any one claims 1 to 10, wherein the culture medium does not contain exogenously added factors that promote growth of undifferentiated cells or wherein the does not comprise a fibroblast growth factor.

12. A kit for growing for growing progenitor cells comprising a culture medium of any one of claims 1-11 and a serum free-nutrient solution.

13. The kit of claim 12, wherein the serum-free nutrient solution comprises:

the amino acids L-Arginine, L-Cystine, L-Cysteine, Glycine, L-Histidine, L-Isoleucine, L-Leucine, L-Lysine, L-Methionine, L-Phenylalanine, L-serine, L-Threonine, L-tryptophan, L-tyrosine, L-Valine, L-Alanine, L-Asparagine, L-Aspartic Acid, L-Glutamic Acid, L-Proline and L-Taurine;
the vitamins D-calcium pantothenate; choline chloride; folic acid; I-inositol; niacinamide; pyridoxal; riboflavin; thiamine; d-Biotin; pyridoxine; and Vitamin $B_{12}$ (cyanocobalamin);
the salts sodium phosphate, monobasic and sodium phosphate, dibasic heptahydrate;
the nucleosides adenosine, cytidine, uridine and guanosine; and
insulin; transferrin; lipoic acid/thioctic acid; ethanolamine; and sodium selenite.

14. The kit of claim 13, wherein the serum-free nutrient solution comprises one or more of:

from at least 0.0001 g/L to at least 0.03 g/L of each of the amino acids;
from 5 x $10^{-6}$ g/L to 0.001 g/L of each of the vitamins;
from 0.0005 g/L to 0.001 g/L of the phosphate salts; and
at least 0.0001 g/L to at least 0.005 g/L of the each of the nucleosides.

15. The kit of claim 14, wherein the serum-free nutrient solution further comprises putrescine, a stabilizer, and/or a foaming agent.

Europäisches
Patentamt

European
Patent Office

Office européen
des brevets

# EUROPEAN SEARCH REPORT

Application Number

EP 18 15 9408

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Anonymous: "Minimum essential medium eagle - alpha modification with nucleosides", <br><br> 1 April 2008 (2008-04-01), XP055099835, Retrieved from the Internet: URL:http://www.stemcell.com/~/media/Technical Resources/9/9/A/1/0/29846_PIS_1_1_0.pdf?la=en [retrieved on 2014-02-03] * the whole document * | 1-15 | INV. C12N5/00 C12N5/073 |
| Y | Anonymous: "Product Profile: Minimum essential medium alpha", <br><br> 31 December 2009 (2009-12-31), XP055099833, Retrieved from the Internet: URL:http://www.dnr-is.com/src/1242 Minimum Essential Medium.pdf [retrieved on 2014-02-03] * the whole document * | 1-15 | |
| Y | US 2004/151709 A1 (GORROCHATEGUI BARRUETA ALBERTO [ES] ET AL) 5 August 2004 (2004-08-05) * paragraph [0087] - paragraph [0091]; claim 1 * | 1-15 | TECHNICAL FIELDS SEARCHED (IPC) <br><br> C12N |
| Y | WO 91/10726 A1 (US HEALTH [US]) 25 July 1991 (1991-07-25) * page 10, line 8 - line 17 * | 1-15 | |
| Y | US 2012/014921 A1 (KRAMER BRIAN C [US] ET AL) 19 January 2012 (2012-01-19) * paragraphs [0108], [0510] * | 1-15 | |

-/--

The present search report has been drawn up for all claims

| Place of search | Date of completion of the search | Examiner |
|---|---|---|
| Munich | 12 June 2018 | Offermann, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 15 9408

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2006/071794 A2 (ETHICON INC [US]; HARRIS IAN ROSS [US]; MESSINA DARIN J [US]; KIHM ANT) 6 July 2006 (2006-07-06) * paragraph [0757] * | 1-15 | |
| Y | ALLAN FENSELAU ET AL: "Nucleoside requirements for the in vitro growth of bovine aortic endothelial cells", JOURNAL OF CELLULAR PHYSIOLOGY, vol. 108, no. 3, 1 September 1981 (1981-09-01), pages 375-384, XP055099836, ISSN: 0021-9541, DOI: 10.1002/jcp.1041080311 * the whole document * | 1-15 | |
| Y | SAEZ-LARA, MJ ET AL.: "Biochemical and Molecular Actions of Nutrients: Exogenous Nucleosides Stimulate Proliferation of Fetal Rat Hepatocytes 1", THE JOURNAL OF NUTRITION, 1 June 2004 (2004-06-01), pages 1309-1313, XP055099837, * the whole document * | 1-15 | TECHNICAL FIELDS SEARCHED (IPC) |
| Y | BYONG-GON PARK ET AL: "Development of high density mammalian cell culture system for the production of tissue-type plasminogen activator", BIOTECHNOLOGY AND BIOPROCESS ENGINEERING, vol. 5, no. 2, 1 April 2000 (2000-04-01), pages 123-129, XP055099881, ISSN: 1226-8372, DOI: 10.1007/BF02931883 * the whole document * | 1-15 | |

-/--

The present search report has been drawn up for all claims

| Place of search | Date of completion of the search | Examiner |
|---|---|---|
| Munich | 12 June 2018 | Offermann, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 15 9408

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | OZTURK S S: "ENGINEERING CHALLENGES IN HIGH DENSITY CELL CULTURE SYSTEMS", CYTOTECHNOLOGY, KLUWER ACADEMIC PUBLISHERS, DORDRECHT, NL, vol. 22, no. 1-03, 1 January 1996 (1996-01-01), pages 3-16, XP008062814, ISSN: 0920-9069, DOI: 10.1007/BF00353919 * the whole document * ----- | 1-15 | |

TECHNICAL FIELDS
SEARCHED     (IPC)

The present search report has been drawn up for all claims

| Place of search | Date of completion of the search | Examiner |
|---|---|---|
| Munich | 12 June 2018 | Offermann, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 9408

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2018

| Patent document cited in search report | | | Publication date | Patent family member(s) | | | Publication date |
|---|---|---|---|---|---|---|---|
| US 2004151709 | A1 | | 05-08-2004 | AT | 512218 | T | 15-06-2011 |
| | | | | AU | 2002226426 | A1 | 24-07-2003 |
| | | | | EP | 1464697 | A1 | 06-10-2004 |
| | | | | ES | 2370012 | T3 | 12-12-2011 |
| | | | | US | 2004151709 | A1 | 05-08-2004 |
| | | | | WO | 03057865 | A1 | 17-07-2003 |
| WO 9110726 | A1 | | 25-07-1991 | AT | 145235 | T | 15-11-1996 |
| | | | | AU | 653927 | B2 | 20-10-1994 |
| | | | | CA | 2074363 | A1 | 23-07-1991 |
| | | | | DE | 69123140 | D1 | 19-12-1996 |
| | | | | DE | 69123140 | T2 | 03-04-1997 |
| | | | | DK | 0512066 | T3 | 02-12-1996 |
| | | | | EP | 0512066 | A1 | 11-11-1992 |
| | | | | JP | H0789954 | B2 | 04-10-1995 |
| | | | | JP | H05502379 | A | 28-04-1993 |
| | | | | WO | 9110726 | A1 | 25-07-1991 |
| US 2012014921 | A1 | | 19-01-2012 | NONE | | | |
| WO 2006071794 | A2 | | 06-07-2006 | AU | 2005322060 | A1 | 06-07-2006 |
| | | | | CA | 2589041 | A1 | 06-07-2006 |
| | | | | EP | 1831356 | A2 | 12-09-2007 |
| | | | | ES | 2621847 | T3 | 05-07-2017 |
| | | | | JP | 5340599 | B2 | 13-11-2013 |
| | | | | JP | 2008525039 | A | 17-07-2008 |
| | | | | JP | 2013172725 | A | 05-09-2013 |
| | | | | PL | 1831356 | T3 | 31-07-2017 |
| | | | | US | 2006223177 | A1 | 05-10-2006 |
| | | | | US | 2012315251 | A1 | 13-12-2012 |
| | | | | WO | 2006071794 | A2 | 06-07-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7510873 B
- US 7524489 B
- US 2005005863 A
- US 20070141700 A
- US 20080166328 A
- US 2002029971 W, Ho
- WO 03025149 A, Ho
- US 877012 A
- US 2004877012 A

### Non-patent literature cited in the description

- Cell & Tissue Culture: Laboratory Procedures. John Wiley & Sons, 1995
- Animal Cell Bioreactors. Butterworth-Heinemann, 1991
- **FRESHNEY.** Culture of Animal Cells: A Manual of Basic Techniques. Wiley-Liss, Inc, 1994
- **PRICE et al.** *In Vitro,* 1982, vol. 18, 576-584
- **TUSHER, V.G. et al.** *Proc. Natl. Acad. Sci. USA,* 2001, vol. 98, 5116-5121
- **MESSINA et al.** *Exper. Neurol.,* 2003, vol. 184, 816-829
- **ROSEN et al.** *Ciba Found. Symp.,* 1997, vol. 212, 215-26
- **SALCEDO et al.** *Blood,* 2000, vol. 96, 34-40
- **LI et al.** *J. Immunol.,* 2003, vol. 170, 3369-76
- **HUGHES et al.** *Ann. Thorac. Surg.,* 2004, vol. 77, 812-8
- **CHENG et al.** *Dev. Biol.,* 2003, vol. 258, 319-33
- **LIU, K et al.** *PNAS,* 1999, vol. 96, 5147-5152
- **PLAIA et al.** *Stem Cells,* 2006, vol. 24 (3), 531-546